# EUROPEAN PATENT SPECIFICATION

(11) **EP 3 155 088 B1**
(45) Date of publication and mention of the grant of the patent: **05.08.2020**
(21) Application number: 15735861.5
(22) Date of filing: 11.06.2015
(51) Int. Cl.: C12N 1/20, C12Q 1/68, A61K 39/02, C12R 1/01

(54) **NOVEL TENACIBACULUM SP ISOLATE**
NEUARTIGES ISOLAT VON TENACIBACULUM SP
NOUVEL ISOLAT DE TENACIBACULUM SP.

(30) Priority: 12.06.2014 US 201462011076 P
(43) Date of publication of application: 19.04.2017
(73) Proprietor: Cermaq Group AS, 0191 Oslo (NO)
(72) Inventor: BREVIK, Øyvind, N-5165 Laksevåg (NO); OTTEM, Karl Fredrik, N-8286 Nordfold (NO)
(74) Representative: Onsagers AS
(86) International application number: PCT/EP2015/063050
(87) International publication number: WO 2015/189328

(56) References cited:
- WO-A1-2013/171236
- OLSEN A B ET AL: "Tenacibaculum sp. associated with winter ulcers in sea-reared Atlantic salmon Salmo salar", DISEASES OF AQUATIC ORGANISMS, vol. 94, no. 3, May 2011 (2011-05), pages 189-199, XP8177541, ISSN: 0177-5103 cited in the application -& DATABASE EMBL [Online] 23 November 2009 (2009-11-23), "Tenacibaculum sp. 5136_F112_C_98 16S ribosomal RNA gene, partial sequence.", XP002745205, retrieved from EBI accession no. EMBL:GU124763 Database accession no. GU124763 -& DATABASE EMBL [Online] 23 November 2009 (2009-11-23), "Tenacibaculum sp. 5132_F78_6R_98 16S ribosomal RNA gene, partial sequence.", XP002745206, retrieved from EBI accession no. EMBL:GU124767 Database accession no. GU124767 -& DATABASE EMBL [Online] 23 November 2009 (2009-11-23), "Tenacibaculum sp. VIB F140/96 16S ribosomal RNA gene, partial sequence.", XP002745207, retrieved from EBI accession no. EMBL:GU124770 Databas
- "The Health Situation in Norwegian Aquaculture 2012", , 12 August 2013 (2013-08-12), XP055162095, Retrieved from the Internet: URL:http://www.vetinst.no/eng/content/down load/10605/142950/file/2012_Fish_Health_Re port.pdf [retrieved on 2015-01-14]

### SEQUENCE LISTING

<110> Cermaq ASA
<120> Novel Tenacibaculum isolate and the use thereof
<130> P23701PC00
<160> 55
<170> PatentIn version 3.5
<210> 1
   <211> 1376
   <212> DNA
   <213> Tenacibaculum finnmarkense
<220>
   <221> misc_feature
   <222> (250)..(250)
   <223> n is a, c, g, or t
<400> 1
   cagatgacga ccggcgaacg ggtgcgtaac gcgtatagaa tctgccttgt acaggaggat      60
   agcctttaga aatgaagatt aacactccat aatgtagtag actggcatca gtttactatt     120
   aaacatttat gggtacaaga tgactatgcg tcctattagc tagatggtga ggtaacggct     180
   taccatggcg acgatggtag ggggtctgag aggattatcc cccacactgg tactgagaca      240
   cggaccagan tcctacggga ggcagcagtg aggaatattg gtcaatggag gcaactctga     300
   accagccatg ccgcgtgcag gatgactgcc ctatgggttg taaactgctt ttatacagga     360
   agaaaccgat gtacgtgtac atccttgacg gtactgtaag aataaggacc ggctaactcc     420
   gtgccagcag ccgcggtaat acggagggtc cgagcgttat ccggaatcat tgggtttaaa     480
   gggtccgcag gcggtcaatt aagtcagagg tgaaagccca tcgctcaacg atggaactgc     540
   ctttgatact ggttgacttg agttatacgg aagtagatag aataagtagt gtagcggtga     600
   aatgcataga tattacttag aataccgatt gcgaaggcag tctactacgt atatactgac     660
   gctgagggac gaaagcgtgg ggagcgaaca ggattagata ccctggtagt ccacgccgta     720
   aacgatggat actagttgtt ggacttcggt tcagtgacta agcgaaagtg ataagtatcc     780
   cacctgggga gtacggtcgc aagactgaaa ctcaaaggaa ttgacggggg cccgcacaag     840
   cggtggagca tgtggtttaa ttcgatgata cgcgaggaac cttaccaggg cttaaatgta     900
   gattgacagg gctagagata gctttttctt cggacaattt acaaggtgct gcatggttgt     960
   cgtcagctcg tgccgtgagg tgtcaggtta agtcctataa cgagcgcaac ccctattttt    1020
   agttgctaac aggttaagct gaggactcta gagagactgc cggtgcaaac cgtgaggaag    1080
   gtggggatga cgtcaaatca tcacggccct tacgtcctgg gctacacacg tgctacaatg    1140
   gtatggacaa tgagcagcca ctgggcgacc aggagcgaat ctataaacca tatcacagtt    1200
   cggatcggag tctgcaactc gactccgtga agctggaatc gctagtaatc ggatatcagc    1260
   catgatccgg tgaatacgtt cccgggcctt gtacacaccg cccgtcaagc catggaagct    1320
   gggggtgcct gaagttcgtt accgcaagga gcgacctagg gtaaaactgg taacta         1376
<210> 2
   <211> 807
   <212> DNA
   <213> Tenacibaculum finnmarkense
<400> 2
   ttagaaatta aaaaagctga tggttcaatt ttagtattag aggttcaaca acatataggt        60
   gaagatacag ttcgtactat ttcgatggat gcaactgatg gtttaagcag aggtacagaa       120
   gttatcgcta cgggtaatcc aattcaaatg ccaataggta aagatattta cggacgttta       180
   tttaacgtta ctggtgaagc tattgatggt ttaggtaatt tacctaaaac aggagatgat       240
   ggtttatcaa tccaccgtca agcacctaaa tttgaagact tatcagtgtc aactgaagtt       300
   ttatttactg gtattaaagt aattgattta attgagccgt atgcaaaagg aggtaaaatt       360
   ggattatttg gtggagcagg agtaggtaaa accgtattaa ttcaggagtt aattaacaac       420
   attgcaaaag gacacggagg tttatctgta ttcgcaggtg ttggtgaaag aactcgtgaa       480
   ggaaacgatt tattaagaga gatgttagaa tctggaatta tcaaatatgg tgatgatttt       540
   atgcattcta tggaagaagg aggatgggat ttatctaaag ttgataaacc aggaatgaga       600
   gactctaaag ctactttcgt attcggacaa atgaacgaac cacctggagc acgtgcacgt       660
   gttgcattat ctggtttatc tattgctgaa tatttccgtg atggagcagg agaagcacaa       720
   ggaaaagatg ttcttttctt cgttgataac atcttccgtt ttacacaagc aggttctgag       780
   gtatcggcat tattaggtcg tatgcca                                           807
<210> 3
   <211> 807
   <212> DNA
   <213> Tenacibaculum finnmarkense
<400> 3
   ttagaaatta aaaaagctga tggttcaatt ttagtattag aggttcaaca acatataggt        60
   gaagatacag ttcgtactat ttcgatggat gcaactgatg gtttaagcag aggtacagaa       120
   gttatcgcta cgggtaatcc aattcaaatg ccaataggta aagatattta cggacgttta       180
   tttaacgtta ctggtgaagc tattgatggt ttaggtaatt tacctaaaac aggagatgat       240
   ggtttatcaa tccaccgtca agcacctaaa tttgaagact tatcagtgtc aactgaagtt       300
   ttatttactg gtattaaagt aattgattta attgagccgt atgcaaaagg aggtaaaatt       360
   ggattatttg gtggagcagg agtaggtaaa accgtattaa ttcaggagtt aattaacaac       420
   attgcaaaag gacacggagg tttatctgta ttcgcaggtg ttggtgaaag aactcgtgaa 480
   ggaaacgatt tattaagaga gatgttagaa tctggaatta tcaaatatgg tgatgatttt 540
   atgcattcta tggaagaagg aggatgggat ttatctaaag ttgataaacc aggaatgaga 600
   gactctaaag ctactttcgt attcggacaa atgaacgaac cacctggagc acgtgcacgt 660
   gttgcattat ctggtttatc tattgctgaa tatttccgtg atggagcagg agaagcacaa 720
   ggaaaagatg ttcttttctt cgttgataac atcttccgtt ttacacaagc aggttctgag 780
   gtatcggcat tattaggtcg tatgcca 807
<210> 4
   <211> 936
   <212> DNA
   <213> Tenacibaculum finnmarkense
<400> 4
   aaagcaacca ttattaaagt tttagaagat ggtggaagct gtgttttaat gtcgcattta 60
   gggcgtccta aagctcaaga agaggaattt tcattacgtc atattgtagc agaagtaact 120
   aaaacgttag gtgttcaagt aaaatttgtg aacgattgta tcggtgataa agttgaagaa 180
   gctatcgcta gtttacaaaa tggcgaaatt ttattattag aaaatttacg tttttatcct 240
   gaagagaaag caggagatgt tgcttttgct gaaaaattag caaatttggg agatatttat 300
   gtaaatgatg cttttggaac agcacacaga gcgcacgctt ctacggctgt aattgcgcaa 360
   ttttttactg ataacaaatg ttttggtaat ttattatcaa cagaaataga aagtattaat 420
   aaagttttaa atgatgccga aaaacctgta acagctattt taggaggcgc aaaagtatcc 480
   tctaaaatag gcgttattga aaatattatt gaaaaagtag accacattat tgttggtgga 540
   ggaatgactt ttacttttat aaaagcttta ggcggaaaca tcggaaattc attagttgaa 600
   gatgataaat taagtttagc attggctatt ttgaaattag caaaagaaaa aaatacagaa 660
   atccatttac ctgttgatgc tgtaattgct gatgcttttt ctaatgatgc aaatacgcaa 720
   attgtggata ctacagaaat tcctgaagga tggatggggc ttgattgtgg accaaaaact 780
   tcagaaaatt ttgcagaagt aatagctaaa tcgaaaacta ttttatggaa cggaccttta 840
   ggtgtttttg aattagaaaa atttgcaaca ggaactattg aattaggaaa cgctatttca 900
   aaagcaactg aaaatggtgc tttctcttta gttgga 936
<210> 5
   <211> 987
   <212> DNA
   <213> Tenacibaculum finnmarkense
<400> 5
   tgctatcaca aaagtattag ctgatgcagg attctctgaa gctagagatt ttgatcaaat 60
   cgataatgca ccagaagaaa aagaaagagg tattacaatt aactcttctc acgttgaata 120
   tgcaacagaa aaccgtcatt acgctcacgt agactgtcca ggtcacgcgg attacgtaaa 180
   gaacatggta actggtgctg ctcaaatgga tggagctatt ttagtagtag ctgcaactga 240
   tggtcctatg ccacaaacac gtgagcacat tttattaggt cgtcaagtag gtattcctcg 300
   tatggttgtt ttcttgaata aagttgacat ggttgatgat gaggagttac ttgagttagt 360
   agaaatggaa gtaagagaat tattatcttt ctatgagtac gatggagata atggtcctgt 420
   agtttcaggt tcagctttag gagctttaaa tggtgaagaa aaatgggtta atactgtttt 480
   agaattaatg gctgctgttg atgcttggat tgaagagcct ttaagagaaa ctgaaaagcc 540
   tttcttaatg cctattgaag atgtattctc tattactggt cgtggtactg ttgcaactgg 600
   tcgtattgaa acaggaatca tcaattctgg tgaatctgta gatattattg gtatgggtac 660
   tgagaagtta acttctacag ttactggtat tgaaatgttc cgtcaaatct tagatagagg 720
   tgaggctgga gataatgcag gtatcttatt aagaggtatt gctaaagaag atattaaaag 780
   aggaatggta atctgtaagc caggttctgt aactccacac gctaagttta aggctgaggt 840
   gtatgtattg aaaaaagaag aaggtggacg tcacactcca ttccataata actatcgccc 900
   tcagttttac gtacgtacta ctgatgtaac aggaaatatt ttcttgcctg aaggtgtaga 960
   aatggttatg cctggtgata acttaac 987
<210> 6
   <211> 906
   <212> DNA
   <213> Tenacibaculum finnmarkense
<400> 6
   gggtattgta gatttagtga agaatcgtgc tattgtatgg catgatgcaa cacaaggagc 60
   tacatttgat atcgttgata ttccagaaga tttaaaagaa gaagctgctg aattaagagg 120
   gaaactaatt gaagaagttg ctagttacga tgagaatctt ttagagaaat tcatggaaga 180
   tgaagactca attactgaag aggaagtgca tgctgcactt cgtgctgctg taatggatat 240
   ggctatcatt cctatgattt gtggatcagc atttaaaaat aaaggtgttc agttcttatt 300
   agatgctgtt tgtcgttatt taccttctcc aatggataag gaagctgtta tcgggacaaa 360
   tcctgataca aacgaagaag aaagaagaaa gccagatgta aaggagcctt ttgctgcttt 420
   agcatttaaa attgctactg atcctttcgt aggtcgttta gcatttttta gagcttattc 480
   tgggcgttta gacgctggtt cttatgtttt aaataatcgt tcaggtaaaa aagaacgtat 540
   ttctcgtatc tatcaaatgc atgctaacaa gcaaaacgct attccttaca ttgaagcagg 600
   ggatatcggt gcagctgtag gatttaaatc gattaaaaca ggagatactt tatctgatga 660
   aaagcatcct attgttttag aatctatgga cttccctgat cccgtaattg gtatcgcggt 720
   tgagcctaaa actaaagcag atgttgataa attaggaatg tctttagcta agttagcaga 780
   agaagatcct acatttacag ttagaactga tgaagcttca ggacagacta ttatttctgg 840
   aatgggtgag ttacacttag atattattgt tgatcgttta aaacgtgaat tcaaggtgga 900
   agttaa 906
<210> 7
   <211> 20
   <212> DNA
   <213> Tenacibaculum finnmarkense
<400> 7
   gaatctgcct tgtacaggag 20
<210> 8
   <211> 21
   <212> DNA
   <213> Tenacibaculum finnmarkense
<400> 8
   aggtcgctcc tyrcggtaac g 21
<210> 9
   <211> 18
   <212> DNA
   <213> Tenacibaculum finnmarkense
<400> 9
   acctccttca cggatagc 18
<210> 10
   <211> 20
   <212> DNA
   <213> Tenacibaculum finnmarkense
<400> 10
   ttacgatcgt tcgaagcccc 20
<210> 11
   <211> 20
   <212> DNA
   <213> Tenacibaculum finnmarkense
<400> 11
   atytctccaa aacgctgacc 20
<210> 12
   <211> 22
   <212> DNA
   <213> Tenacibaculum finnmarkense
<400> 12
   aaaacgaatc aaggwacgaa ya 22
<210> 13
   <211> 21
   <212> DNA
   <213> Tenacibaculum finnmarkense
<400> 13
   accctttcca aggcataaag g 21
<210> 14
   <211> 22
   <212> DNA
   <213> Tenacibaculum finnmarkense
<400> 14
   gagccatygg ttttgaaaga ga 22
<210> 15
   <211> 21
   <212> DNA
   <213> Tenacibaculum finnmarkense
<400> 15
   ctcttgctgt ctcctcatct g 21
<210> 16
   <211> 22
   <212> DNA
   <213> Tenacibaculum finnmarkense
<400> 16
   atccaccaag atatagcatc ca 22
<210> 17
   <211> 20
   <212> DNA
   <213> Tenacibaculum finnmarkense
<400> 17
   gctccwccac cwgtagaaac 20
<210> 18
   <211> 23
   <212> DNA
   <213> Tenacibaculum finnmarkense
<400> 18
   tycgtgtaga ttttaatgtg cct 23
<210> 19
   <211> 21
   <212> DNA
   <213> Tenacibaculum finnmarkense
<400> 19
   tggyccagtw atcgatgttg a 21
<210> 20
   <211> 20
   <212> DNA
   <213> Tenacibaculum finnmarkense
<400> 20
   aatacgytct tgcattgctc 20
<210> 21
   <211> 22
   <212> DNA
   <213> Tenacibaculum finnmarkense
<400> 21
   atggtaactc acccattcca ga 22
<210> 22
   <211> 20
   <212> DNA
   <213> Tenacibaculum finnmarkense
<400> 22
   tggcatgatg caacacaagg 20
<210> 23
   <211> 17
   <212> DNA
   <213> Tenacibaculum sp
<400> 23
   agtgtgacgt ccacctt 17
<210> 24
   <211> 18
   <212> DNA
   <213> Tenacibaculum sp
<400> 24
   ctgtaagcca ggttctgt 18
<210> 25
   <211> 21
   <212> DNA
   <213> Tenacibaculum sp
<400> 25
   tttcaataca tacacctcag c 21
<210> 26
   <211> 21
   <212> DNA
   <213> Tenacibaculum sp
<400> 26
   ggagcaaaca ttgaccaaat t 21
<210> 27
   <211> 20
   <212> DNA
   <213> Tenacibaculum sp.
<400> 27
   ggtatgtccg taacgtggaa 20
<210> 28
   <211> 23
   <212> DNA
   <213> Tenacibaculum sp.
<400> 28
   tcctgcttga tcagttaaag cgt 23
<210> 29
   <211> 19
   <212> DNA
   <213> M. viscosa
<400> 29
   gatgataacg caacagcag 19
<210> 30
   <211> 22
   <212> DNA
   <213> M. viscosa
<400> 30
   cggaaactta caccagataa tg 22
<210> 31
   <211> 29
   <212> DNA
   <213> M. viscosa
<400> 31
   tcttggagca ggtctagaat atacaccag 29
<210> 32
   <211> 21
   <212> DNA
   <213> Salmon elongation factor 1 Alpha A
<400> 32
   cccctccagg acgtttacaa a 21
<210> 33
   <211> 19
   <212> DNA
   <213> Salmon elongation factor 1 Alpha A
<400> 33
   cacacggccc acaggtaca 19
<210> 34
   <211> 17
   <212> DNA
   <213> Salmon elongation factor 1 alpha
<400> 34
   atcggtggta ttggaac 17
<210> 35
   <211> 20
   <212> DNA
   <213> Green Non-Sulfur bacteria
<400> 35
   agagtttgat cmtggctcag 20
<210> 36
   <211> 20
   <212> DNA
   <213> Green Non-Sulfur bacteria
<220>
   <221> misc_feature
   <222> (15)..(15)
   <223> n is a, c, g, or t
<400> 36
   aaggaggtga tccanccrca 20
<210> 37
   <211> 850
   <212> DNA
   <213> T.dicentrarchi
<400> 37
   ttagaaatta aaaaaactga tggttcaatt ttagtattag aggttcaaca acatataggt       60
   gaagatacag ttcgtactat ttcgatggat gcaactgatg gtttaagtag aggtacagaa      120
   gtaatcgcta cgggtaaccc aattcaaatg ccaataggta aagatattta cggacgttta      180
   tttaacgtta ctggtgaagc tattgatggt ttaggtgatt tacctaaaac aggaaatgat      240
   ggtttatcta tccaccgtca agcacctaaa tttgaagact tatcagtgtc aactgaagtt      300
   ttatttactg gtattaaagt aattgattta attgagccgt atgcaaaagg aggtaaaatt      360
   ggattatttg gtggagcagg agtaggtaaa accgtattaa ttcaagagtt aattaacaac      420
   attgcaaaag gacacggagg tttatctgta ttcgcaggtg ttggtgaaag aactcgtgaa      480
   ggaaacgatt tattaagaga gatgttagaa tctggaatta tcaaatatgg tgatgatttt      540
   atgcactcta tggaagaagg aggatgggat ttatctaaag tagataaacc aggaatgaga      600
   gactctaaag ctactttcgt attcggacaa atgaacgaac cacctggagc acgtgcacgt      660
   gttgctttat ctggtttatc tattgcagaa tatttccgtg atggagcagg agaagcacaa      720
   ggaaaagacg ttcttttctt tgttgataat atcttccgtt ttacacaagc aggttctgag      780
   gtatcagctt tattaggtcg tatgccatca gcagtaggat accaaccaac attagctacc      840
   gaaatgggag                                                             850
<210> 38
   <211> 885
   <212> DNA
   <213> T ovolyticum
<400> 38
   ttgagttcaa tatagaaaat gctgaattac caagaattta cgattcatta gaaattaaaa     60
   aagctgatgg ttctattctt gttttagaag ttcaacaaca tataggtgaa gatacagttc    120
   gtactatttc tatggatgca actgatggtt taagtagagg tacagaggtt atcgctactg    180
   gtagtccaat acaaatgcca ataggaaatg atatttatgg gcgtttattt aacgttacag    240
   gtgaagctat tgatggttta ggtgatttac caaaagcagg tgatgcaggt ttatctatac    300
   accgtcaagc acctaaattt gaagatttat ctgtatctac agaagtttta tttactggta    360
   ttaaagtaat tgatttaatt gagccttatg caaaaggtgg taaaattgga ttatttggtg    420
   gagccggagt aggtaaaact gtattaattc aagagttaat taacaacatt gcaaaaggac    480
   acggaggatt atctgtattt gcaggtgtag gagaaagaac tcgtgaagga aatgatttat    540
   taagagagat gttagagtca ggaattatca aatatggtga tgactttatg cactctatgg    600
   aagaaggagg atgggattta tctaaagtag ataagcctgg aatgagaggt tcaaaagcta    660
   ctttcgtatt tggacaaatg aacgaaccac ctggagcacg tgcacgtgtt gcattatctg    720
   gtttaacaat tgctgaatat ttccgtgatg gggcaggaga agctcaagga aaagatgtac    780
   ttttcttcgt cgataatata ttccgtttta cacaagcagg ttctgaggta tcggcattat    840
   taggtcgtat gccatcagca gtaggatatc aaccaacatt agcta                    885
<210> 39
   <211> 846
   <212> DNA
   <213> T. soleae
<400> 39
   aactaccaaa aatttacgat tcattagaaa ttaaaaaagc tgatggttca gttttagtat     60
   tagaagttca acaacatgtt ggtgaagata cagtgcgtac aatatctatg gatgcaaccg    120
   atggattaag tagaggtaca gaagtgatcg ctactggtaa tccaattcaa atgccaatag    180
   ggaatgatat ttatggtcgt ttatttaacg taacaggtga ggctatcgat ggtttaggag    240
   atttacctaa aacaggagat gcaggtttat ctattcaccg tcaagcacct aaattcgaag    300
   atttatctgt atcaacagaa gttttattta caggaattaa agtaatcgat ttaattgagc    360
   cgtatgcaaa aggaggtaaa attggattat ttggtggagc aggagttggt aaaaccgtat    420
   taattcaaga gttaattaac aacatcgcaa aaggacacgg aggtttatct gtatttgctg 480
   gtgtaggaga aagaactcgt gaaggaaacg atttattaag agagatgtta gagtctggaa 540
   ttatcaaata tggtgatgat tttatgcact ctatggaaga aggaggatgg gatttatcta 600
   aagtagataa gcctggaatg agagactcta aagctacttt cgtattcgga caaatgaacg 660
   aaccacctgg agcaagagca cgtgttgcat tatctggttt aacaattgct gaatacttcc 720
   gtgatggagc aggagaagca caaggaaaag acgttctttt cttcgttgat aatatcttcc 780
   gttttacaca agcaggttct gaggtgtcag cattattagg tcgtatgcca tcggcagtag 840
   gtacca 846
<210> 40
   <211> 3297
   <212> DNA
   <213> T. dicentrarchi
<400> 40
   catccaagaa tgtattgaaa gaggtttaac acatagtgtg cctttaaaag caaggttaaa 60
   actttattgt acagatcctg agcatgaaga ttttgaaact attgttcaag atgtgtattt 120
   aggaacaatt ccttatatga caaattctgg aaccttcatt attaatggag ctgaaagagt 180
   tgttgtatct caattacacc gttcaccagg ggtgtttttt ggacaatctt tccatgcaaa 240
   tggaactaag ttgtattctg caagagtaat tccttttaaa ggatcttgga tagaatttgc 300
   aactgatatc aatcaggtaa tgtacgctta tattgataga aagaaaaaac taccagtaac 360
   tacgttattc agagccattg gttttgaaag agataaagat atattagaga tttttgattt 420
   agcagaagaa attaaggttt ctaaagcagg attaaaaaaa gtattagggc gtaaactagc 480
   agctagggtt ctaaaaactt ggcatgaaga tttcgtagat gaagataccg gtgaagtggt 540
   gtctattgaa cgtaacgaaa ttgttttcga ccgtgataca atcatagaga aagaacatat 600
   tgatgaaata attgatgcag gtgctaaaac tatcttatta cataaggtag ataatgacat 660
   ggccgattat gccatcattc ataatacact gcagaaagat cctacgaatt ctgaaaaaga 720
   agcagtagaa cacgtctaca gacaattacg taatgctgag ccgccagatg aggagacagc 780
   aagaggaatt attgaaaaat tattcttctc tgagcaacgt tatagtttag gtgaagttgg 840
   tcgttttaga atgaacacca agttacagtt aaatgagccg attgatcaga aggtattaac 900
   taaaaatgat attattacca ttatcaaata cttaattgag ttaattaact caaaagcaga 960
   agtagatgat attgatcact tatctaaccg tcgtgtaaga actgttggtg aacaattagc 1020
   aggtcagttt ggtgtaggtt tagctcgtat ggctcgtact attcgtgaaa gaatgaatgt 1080
   gcgtgataat gaggtgttta ctcctattga tttaattaat gcgaagacct tatcttcagt 1140
   tattaattcg ttttttggaa ctaaccagtt atctcaattt atggatcaaa ccaatccatt 1200
   agctgagatt acacacaaac gtcgtttatc agcactagga cctggaggat tatctagaga 1260
   aagagcagga ttcgaggttc gtgatgttca ctatacacac tacggacgtt tatgtccaat 1320
   tgaaactcct gagggaccga atattggatt aatttcatct ttagcagtat ttgcaaaggt 1380
   taacagttta ggttttatcg aaactcctta taaaaaggtt atcgaaggaa acgtagatgt 1440
   acaccaagag catatttatt taagtgctga agaagaagaa ggaatgaagt ttgcacaatc 1500
   taatttagag gtaagtgcag ctggagattt tgttggagat aaattaattt cacgtgaagg 1560
   gggagatttc cctgtagtaa cacctactga aattgattat atggatgttg cccctaacca 1620
   gatagcatct atatcagcat cattaattcc tttcttagag catgatgatg ccaatagagc 1680
   cttaatggga tcgaacatga tgcgtcaagc agttccatta ttacgtccag agtctccaat 1740
   tgtaggtaca ggattagagc gtagagttgc aaaagattct cgtattttaa ttaatgctga 1800
   aggacctggt gttattgagt atgttgatgc gaataaaata agaattaagt atgatagatc 1860
   agatgaagag cgtttagtaa gctttgattc agatgatatt tcttataact taattaagtt 1920
   tagaaaaaca aatcaaggta cgaacattaa cctaaaacca attgttaaag ttggagatag 1980
   agttgaagaa ggtcaagtac tttgtgaagg atatgcaaca caaaaaggag aattagcttt 2040
   aggtagaaat atgaaagtag cctttatgcc ttggaaaggg tataactttg aggatgcgat 2100
   tgtaatttct gaaaaagttg ttcgtgaaga tatctttact tctattcata ttgacgaata 2160
   ttctttagat gttcgtgata cgaaattagg agctgaagag ttaacaaacg atattcctaa 2220
   cgtttcagaa gaggctacta aagacttaga tgaaaatgga atgattcgta ttggagcaga 2280
   ggttaatcca ggagatatct taattggtaa aattactcca aaaggtgaat ctgacccaac 2340
   tcctgaagaa aaattattaa gagctatctt tggtgataaa gcaggtgatg taaaagatgc 2400
   atcactaaaa gcttctccat cattaagagg ggtagtaatt aataaaaaat tattccaacg 2460
   tgctgtaaaa gataaaacaa aacgagcaag agataaagaa gctattgctg ttttagaagc 2520
   atcttatgta cacaagtttg aagatttaaa agatttatta attgaaaaat tatttaatct 2580
   aattacagga aaaacttctc aaggggtatt taatgattta ggagaggaag tattaccaaa 2640
   aggtaaaaaa ttcactcaaa aaatgttaaa ttctgtaggt gattttacgc acttacacgg 2700
   aacttggact accgataaag acttaaatag attagttgtt gaattagttc acaattataa 2760
   aattaaagtt aacgatttac aaggttcttt acgtcgtgag aaatttacaa tttctgtagg 2820
   tgatgaatta ccagcaggaa tcttaaaact tgcaaaaatt tatgttgcta aaaaacgtaa 2880
   attaaaagta ggagataaga tggcaggacg tcacggaaat aaaggtattg ttgctcgtat 2940
   tgttagagct gaagatatgc ctttcttagc agacggaaca cctgttgata ttgtattaaa 3000
   tccattaggg gtaccatctc gtatgaatat tggtcagatt tatgaaactg ttcttggatg 3060
   ggcaggtcaa aaattagatc agaagtatgc aacaccaatt tttgatggag caaacatcga 3120
   ccaaataaac gagttaactg ataaagcagg aattccacgt tacggacata catatttata     3180
   tgatggaggt acaggaaaac gtttcgatca accagcaacg gtaggtatca tttatatgat     3240
   taagttaggt cacatgattg aagataaaat gcatgcgcgt tctattggtc cttattc        3297
<210> 41
   <211> 3313
   <212> DNA
   <213> T. ovolyticum
<400> 41
   atagcatcca agaatgtatt gaaagaggat taacacatag tgtaccttta aaggcaagat      60
   taaagcttta ctgtacagat cctgaacatg aagattttga aacaattgtt caagacgtat     120
   atttaggtac tattccttat atgactaatt ctggaacttt cattattaat ggagcagaaa     180
   gagttgtagt ttctcagtta caccgttcac caggtgtttt ctttggacaa tcattccacg     240
   caaacggaac caaattatac tcagcaagag taattccttt taaaggatct tggatagaat     300
   ttgcaactga tatcaatcaa gtaatgtatg cttatattga tagaaagaaa aaattaccag     360
   taacaacatt attcagagcc attggttttg aaagagataa agatatttta gaaatttttg     420
   atttagcaga agaagtaaaa gtttctaaag ctggattaaa gaaaatatta gggcgtaaat     480
   tagctgctag agttttaaaa acttggcatg aagattttgt agatgaagat acaggagaag     540
   ttgtatcgat agaacgaaac gaaattgttt tcgatcgtga tacaatcata gaaaaagaac     600
   atattgatga aataattgat gcaggtgcaa aaactatctt attacataag gtagataatg     660
   acatggcaga ttatgcaatc atccacaata cattacaaaa agatcctacg aattctgaaa     720
   aagaagcagt agaacatgtt tatagacaat tacgtaatgc tgagccgcca gatgaggaga     780
   cagcaagagg aataattgaa aaattatttt tctctgagca acgttatagt ttaggagaag     840
   ttggtcgttt tagaatgaac accaaattac agttaaacga accgattgat cagaaggtat     900
   taactaagaa cgatattatt acaattatca aatacttaat tgagttaatt aactcaaaag     960
   cagaagtaga tgatattgat cacttatcta atcgtcgtgt aagaactgtt ggtgaacaac    1020
   tagcaggtca attcggtgtt ggtttagctc gtatggctcg tactattcgt gaaagaatga    1080
   atgtacgtga taacgaagta tttactccaa ttgatttaat taatgcaaaa acattatctt    1140
   ctgtaattaa ttctttcttt ggtactaacc agttatctca atttatggat caaacaaatc    1200
   cattagcaga gattacacac aaacgtcgtt tatcagcttt aggacctgga ggtttatcta    1260
   gagaaagagc aggatttgag gttcgtgatg ttcactatac acactatggt cgtttatgtc    1320
   caattgaaac tcctgaagga ccgaacattg gattaatttc atctttagct gtatttgcta    1380
   aggtgaataa tttaggtttt atcgaaacac cttataagaa ggttattgaa ggaaacatag    1440
   atttagatca agagcatatt tatttaagtg ctgaggaaga agaaggaatg aagtttgcac    1500
   aatctaattt agaagttagt gcagatggtg atttcgttgg tgataaatta atttcacgtg 1560
   agggtggtga ctttccagtt gttactccaa cagaaattga ttacatggat gttgctccta 1620
   accagattgc atctatatca gcatcattaa ttcctttctt agaacatgat gatgccaacc 1680
   gtgcattaat gggatcgaac atgatgcgtc aagcagttcc attattacgt ccagaatctc 1740
   caattgtagg tacaggatta gagcgtagag tagctaaaga ttctcgtatt ttaataaatg 1800
   cagaaggagc aggtgttatt gaatatgttg atgcaaataa aataacaatt aagtacgata 1860
   gatcagaaga agagcgctta gtaagtttcg attcagatga aatttcttat aacttaatta 1920
   agtttagaaa aacgaatcaa ggtacaaaca ttaaccttaa gccaattgta aaagttggtg 1980
   atagagttca agaaggtcaa gtactttgtg aaggatatgc aacacaaaaa ggagaattag 2040
   ctttaggtag aaatatgaaa gtagccttta tgccttggaa agggtataac tttgaggatg 2100
   caattgtaat ttctgaaaaa gtagttcgtg aagatatctt tacttctatt catattgatg 2160
   aatattcatt agatgttcgt gatacgaaat taggagctga agaattaacg aatgatattc 2220
   ctaatgtttc tgaagaggct actaaagact tagatgaaaa cgggatgatt cgtattggag 2280
   cagaggtaaa tcctggtgat atcttaattg gtaagattac gcctaaagga gaatctgatc 2340
   caactccaga agaaaaatta ttacgtgcta tttttggtga taaagcagga gatgtaaaag 2400
   atgcatcatt aaaagcttct ccatcattaa gaggtgtagt aattaataag aaattatttc 2460
   aacgtgctgt aaaagataag acaaaacgtg ctcgtgataa agaagcaata gcaactttag 2520
   aagcatctta tgtacataag tttgaaggtt taaaagatat tttaattgaa aagttattca 2580
   atttaattac tggaaaaact tctcaaggag tatttaatga tttaggagaa gaggttttac 2640
   caaaaggtaa aaagtttact caaaaaatgt taaactctgt aggtgatttt acacacttac 2700
   acggaacttg gactacagat aaaactttaa atagattagt tgttgaatta gttcacaatt 2760
   ataaaattaa agttaacgat ttacaaggag ccttacgtcg tgaaaaattc actatttctg 2820
   taggagatga attaccagca ggaatcttaa aactagctaa aatttatgtt gccaaaaaac 2880
   gtaaattaaa agtaggagat aagatggcag gacgtcacgg aaacaaaggt attgttgctc 2940
   gtatagttag agcagaagac atgcctttct tagaagacgg aactccagta gatattgtat 3000
   taaatccatt aggtgtacca tcgcgtatga acattggtca aatttatgaa actgttcttg 3060
   gatgggcagg acaaaaatta gatcaaaagt atgcaacacc aatttttgat ggagcaaata 3120
   ttgatcaaat taacgcattg acagacgaag ctggaattcc acgttacgga catacatatt 3180
   tatatgatgg aggtacagga aaacgtttcg atcagccagc aacagtaggt ataatttata 3240
   tgattaagtt aggtcacatg attgaagata aaatgcacgc gcgttctatt ggaccttact 3300
   cattaattac aca 3313
<210> 42
   <211> 3295
   <212> DNA
   <213> T soleae
<400> 42
   atccaagaat gtattgaaag aggattaaca catagtgtac ctttaaaagc aagattaaag      60
   ctttattgta cagatcctga acacgaagat ttcgaaacta ttgttcaaga cgtatattta     120
   ggtactatac catatatgac caactcagga actttcatta ttaatggagc agaaagagtt     180
   gtagtttctc agttacaccg ttcaccaggt gttttctttg gacaatcttt ccacgcaaat     240
   ggaaccaagt tatactctgc aagagtaatt ccttttaaag gatcttggat agaatttgca     300
   actgatatca atcaagtaat gtatgcttat attgatagaa agaaaaaatt accagtaact     360
   acgttattca gagccatcgg ttttgaaaga gataaagata tattagaaat ttttgattta     420
   gcagaagaag taaaagtttc taaagctgga ttaaaaaaag tattagggcg taaactagcg     480
   gctagagttt taaaaacttg gcatgaagat ttcgttgatg aagatactgg ggaagtagta     540
   tctattgagc gtaacgaaat agtttttgat cgtgatacaa tcatagaaaa agaacatatt     600
   gatgaaataa ttgatgcagg agctaaaact atcttattac ataaggtaga taatgacatg     660
   gcagattatg caatcattca caatacatta caaaaagatc ctaccaattc tgaaaaagaa     720
   gcagtagaac atgtttatag acaattacgt aatgctgaac cgccagatga ggaaactgca     780
   agaggaatta tagaaaaatt attcttctca gagcagcgtt acagcttagg tgaagtaggg     840
   cgttttagaa tgaatactaa acttggtctt aatgaagata tagaccaaaa agtattaaca     900
   aaaactgata ttattacgat tatcaaatac ttaatcgagt taattaattc gaaagcagaa     960
   gtagatgata tcgatcactt atctaaccgt cgtgttagaa cagtaggtga gcaattagca    1020
   ggacaatttg gtgttggttt agctcgtatg gcgcgtacaa ttcgtgaaag aatgaatgta    1080
   cgtgataacg aagtatttac tccgattgat ttaattaatg cgaagacctt atcttcagtt    1140
   attaactcat tctttggtac caaccaatta tctcaattta tggatcaaac caatccatta    1200
   gctgagatta cacacaaacg tcgtttatcc gctttaggac ctggaggttt atctagagaa    1260
   agagcaggat ttgaggtgcg tgatgttcac tatactcact atggaagatt atgtcctatt    1320
   gaaactcctg agggaccgaa tattggatta atttcttctt tatcagtatt tgccaaagta    1380
   aataacttag gatttatcga aactccttat gtaaaagtta cagaaggaaa cgtagatgct    1440
   aaaaaagaac acatttattt aagtgcagag gaagaagaag gaatgaaatt tgcacaatct    1500
   aatttagagg ttgacgcaga tggaagcttt gttggcgaaa aattaatttc acgtgaaggt    1560
   ggggatttcc cagtagtaat accaactgaa attgattata tggatgttgc tcctaaccag    1620
   attgcatcga tatcagcatc gttaattcct ttcttagaac atgatgatgc aaaccgtgca    1680
   ttaatgggat ctaacatgat gcgtcaagca gttccattat tacgtccaga gtctccaatt 1740
   gtgggtacag gtttagaacg tagagtagca aaagactctc gtattttaat caatgctgaa 1800
   ggagctggtg ttgttgagta tgttgatgcg aataaaatta aaattaagta tgatagaaca 1860
   gaagaagaac gtttagtaag ttttgattca gatgatattt catacaactt aattaagttt 1920
   agaaaaacca atcaaggtac aaacatcaac cttaaaccaa ttgtaaaagt tggcgataga 1980
   gttgaagaag gtcaagtact ttgtgaaggt tatgcaacac aaaaaggaga attagcttta 2040
   ggtagaaata tgaaagtagc ctttatgcct tggaaagggt ataactttga ggatgcaatt 2100
   gtaatttctg aaaaagttgt tcgtgaagat atctttactt ctatacatat cgatgaatat 2160
   tcattagatg ttcgtgatac aaaattagga gctgaagaat taacaaacga tattccaaat 2220
   gtttctgaag aggctactaa agacttagat gaaaacggaa tgattcgtat tggagcagaa 2280
   gtaaatcctg gtgatatcct aattggtaaa attacaccaa aaggagaatc tgatccaact 2340
   ccagaagaaa agttattacg tgccattttt ggtgataaag caggtgatgt taaagacgca 2400
   tcattaaaag cttctccatc attaagaggt gtggtaatta ataaaaaatt attcaaacgt 2460
   gctttaaaag ataaaacaaa gcgtgctcgc gataaagaag caatcgctgc gttagaagca 2520
   tcttatgtat ctaaattcga aggattaaaa gatatattaa ttgaaaaatt attcaattta 2580
   attacaggaa aaacatctca aggagtattt aatgatttag gtgaagaagt attaccaaaa 2640
   ggtaaaaaat tcactcaaaa aatgttaaac tctgtaggtg attttacaca cttacacgga 2700
   acttggacta ccgataaaga cttaaataga ttagttgttg agttagttca caactataaa 2760
   attaaagtaa acgatttaca aggatcgtta cgtcgtgaaa agtttacaat ttctgtaggt 2820
   gatgaattac cagcaggaat cttaaaactt gctaaaattt atgttgctaa aaaacgtaaa 2880
   ttaaaagtag gtgataagat ggcaggacgt cacggaaata aaggtattgt tgcacgtatt 2940
   gtacgtcaag aagatatgcc tttcttagaa gacggaacac ctgtagatat cgtattaaat 3000
   ccattagggg taccatctcg tatgaatatt ggtcagattt atgaaactgt tcttggatgg 3060
   gcaggtcaaa aattagatca gaaatatgca acaccaattt ttgatggagc aaacataaac 3120
   gagattaatg ccttaacaga tgaagcagga attccacgtt atggacatac ctatttatat 3180
   gatggaggta caggaaaacg ttttgatcag ccagcaaccg taggtataat ttacatgatt 3240
   aagttaggtc acatgattga agataaaatg catgcgcgtt ctatcggacc ttatt 3295
<210> 43
   <211> 950
   <212> DNA
   <213> T dicentrarchi
<400> 43
   cagcaaaagc aacaattatt aaagttttag aagatggtgg aagctgtgtt ttaatgtcgc        60
   atttaggacg tcctaaagcc caagaagatg ccttttcact acgtcatatt gttgctgaag       120
   taactaaaaa tttaggcgtt caagtaaaat ttgtaaatga ttgtattggt gatgaagttg       180
   ctgatgccgt agcaaattta aaaagtggcg aaattttatt attagaaaac ttacgttttt       240
   atccagaaga aaaaacagga gatattgctt ttgctgaaaa gttatcaaag tggggagata       300
   tttatgtaaa tgacgctttt ggtacagcac acagagcgca tgcttcaaca gctgtaattg       360
   ctcagttttt tactgataat aaatgttttg gtaatttatt agctactgaa atagaaagta       420
   ttgataaggt tttaaatgat tctgaaaagc ctgtaacagc tattttagga ggtgcaaaag       480
   tatcatctaa aattggagtt attgaaaata ttattgaaaa agtagaccat attattgttg       540
   gtggaggaat gacttttact tttataaaag cattaggtgg aaacatcgga aattcattag       600
   tggaagatga taaattaagt ttagcattgg ctattttgaa attagcaaaa gaaaaaaata       660
   cagaaattca cttacctgta gatgctgtaa ttgctgatgc tttttctaat gatgcaaata       720
   cgcaaacagt agatactaca aaaattcctg aaggatggat gggacttgat tgtggagtaa       780
   aaacttcaga aaattttgca gcagtaatag ctaaatcgaa aactatttta tggaatggtc       840
   ctttaggtgt ttttgaatta gaaaattttt caaaaggtac tattgaatta ggaaatgcta       900
   tttcaaaagc aactgaaaac ggtgcatttt ctttagttgg aggaggagat                  950
<210> 44
   <211> 971
   <212> DNA
   <213> T ovolyticum
<400> 44
   agaattcaag cagcaaaatc aacaattata aaagttttag aagatggagg tagttgtgtg 60
   ttaatgtcac atttagggag acctaaagga aaagaagatc agttttcatt acgacatatt 120
   attgcaaaag tatccgatgt aattggagta caagttaagt ttgttgatga ttgtattgga 180
   aatgaagcta cagaagcagt tgctaattta aaaagcggag aaattttatt gctagaaaat 240
   ttacgttttc attcagaaga gaaggctgga gatattgctt ttgcagaaca attatctaaa 300
   ttaggtgatg tatatgtaaa tgatgctttt ggtactgcac acagagcgca tgcatctaca 360
   gcagttatag cacagttttt tacagaaaac aaatgctttg gaaacttatt agctaccgaa 420
   atagaaagta ttaataaagt tttaaacgat tctgaaaaac cagtaactgc aattttagga 480
   ggcgcaaagg tatcgtctaa aattggagtg atagaaaata ttatagaaaa agtagatcat 540
   attatcgttg gaggaggaat gacctttact tttataaagg ctttaggcgg aaccatcgga 600
   aactcgttag tagaagacga taaactagaa ttagcattgt caattttaaa actagcaaaa 660
   gagaaaaata cagaaattca tttacctgta gatgctatta ttgctgatgc tttttcgaat 720
   gatgcaaata cgcaaacggt agatactact aaaattcctg atggatggat ggggcttgac 780
   tgcggaccag aaacttctac gaaatttgca gaagtaatag caaaatctaa aactatttta 840
   tggaatgggc ctttaggagt ttttgaacta gaaaactttg caacaggtac tattgaactt 900
   ggaaatgcaa tttctaaagc aaccgaaaat ggagcattct ctttagttgg aggaggagac 960
   tctgtagcag c 971
<210> 45
   <211> 979
   <212> DNA
   <213> T soleae
<400> 45
   atattacaag aattcaagca gcaaaatcaa caattattaa gattttagaa gatggaggta      60
   gttgtgtgtt aatgtcgcat ttaggtcgcc caagcggagt tgaagaaaaa tactcgttaa     120
   aacatattgt tagtaaagtt accgaagtaa ttggggtaca ggtaaagttt gttgaagatt     180
   gtattggtga taaagcagaa aaagctgttg ctaacttaaa aaatggtgaa gttttattat     240
   tagaaaactt acgtttttat agcgaagaga aaaaaggaga tgtagctttt gctgaaaaat     300
   tatcaaaatt aggagacgtt tatgtaaacg atgcttttgg aactgcgcat agagcacatg     360
   catcaacagc agttatagca cagttttttg ctgctaataa atattttgga aacctattag     420
   caaccgaaat taaaagtata gacaaggttt taaataattc agaaaaacct gttaccgcaa     480
   ttttaggagg agcaaaagta tcatctaaaa taggagtaat tgaaaatatt atcgaaaaag     540
   tagatcatat tatagtgggt ggaggtatga cgtttacctt tattaaagcc ttaggaggat     600
   cgataggaaa ctcattagtg gaagatgata aattagagtt agcattatct attttaaaat     660
   tagcgaaaga gaaaaatacc gaaatacatt taccagtaga tgcagttatt gcggatgctt     720
   tttcaaacga tgctaataca cagactgttg atactactaa aattcctgat ggatggatgg     780
   ggcttgattg cgggccaaaa acctcagaaa aatttgcaac agtcattgca aaatcgaaaa     840
   ctattttatg gaacggtcct ttaggagttt ttgaattaga aaactttgcc aacggaacaa     900
   ttcaattagg aaatgctatt gcaactgcaa ccgaaaatgg agctttttcg cttgttggag     960
   gaggagattc ggtagcagc                                                  979
<210> 46
   <211> 1011
   <212> DNA
   <213> T. dicentrarchi
<400> 46
   ctgtaatagt taagttatca ccaggcataa ccatctctac accttctggc aagaaaatat      60
   ttcctgtcac atcagtagta cgtacataaa actgagggcg atagttatta tggaatggag     120
   tgtgacgtcc accttcttct tttttcaata catacacctc agccttaaac ttagcgtgtg     180
   gagttataga acctggctta cagattacca tacctctttt aatatcttct ttagcaatac     240
   ctctcaacaa gatacctgca ttatctcctg cctcacctct atctaagatt tgacgaaaca 300
   tttcaatacc agtaactgta gaagctaact tttcagtacc cataccgata atatctacag 360
   cgtcaccaga attgattatc cctgtttcaa tacgaccagt tgcaacagta ccacgaccag 420
   taatagaaaa aacatcttca ataggcatta agaaatcttt ttcagtctct cttaaaggct 480
   cttcaaccca agaatcaact gcttccatca actctaatac agtgttaacc catttttcct 540
   caccatttaa agctcctaaa gcagaaccag caacaacagg accattatct ccatcatact 600
   catagaaaga taataattct cttacctcca tctctactaa ctctaacaac tcctcatcat 660
   caaccatatc aaccttgttc aagaaaacaa cgatacgtgg aatacctacc tgacgaccta 720
   ataagatatg ctcacgagtt tgtggcatag gaccatcagt tgcagctact actaaaatag 780
   ctccatccat ctgagcagca cctgtaacca tgttcttaac ataatcggcg tgacctggac 840
   aatctacgtg tgcatagtga cggttctgag tcgcatattc aacgtgagaa gagttaattg 900
   taatacctct ttctttttct tctggagcat tatcgatttg atcaaaatct ctagcttcag 960
   agaatcctgc atcagctaat acttttgtga tagcagcagt taatgtagtt t 1011
<210> 47
   <211> 1188
   <212> DNA
   <213> T ovolyticum
<400> 47
   atggcaaaag aaacttacga tcgttcgaag ccccacttaa atgttggaac tattggtcac 60
   gtagatcacg gtaaaactac attaactgct gctatcacta aagtattagc tgatgcagga 120
   ttctctgaag ctagagattt tgatcaaatc gataatgcac cagaagaaaa agaaagaggt 180
   ataacaatta actcttctca cgtagaatat gcaacagaaa accgtcacta cgcacacgtt 240
   gactgtccag gtcacgccga ttacgtaaag aacatggtta ctggtgctgc acaaatggat 300
   ggagctattt tagtagtagc tgcaacagat ggaccaatgc cacaaactcg tgagcacatt 360
   ttattaggtc gtcaagtagg tattccacgt atggttgttt tcttaaacaa ggtggatatg 420
   gttgatgatg aagagttact tgagttagta gaaatggaag taagagaatt attatctttc 480
   tatgaatatg atggagataa tggtcctgtt gttgctggtt cagctttagg tgctttaaat 540
   ggtgaagaaa aatgggttaa taccgtttta gaattaatgg ctaaggttga tgagtggatc 600
   gaagagcctt taagagaaac tgaaaaggat ttcttaatgc ctattgaaga tgtattctct 660
   attacaggtc gtggtacagt tgctacaggt cgtattgaaa ctggaatcat caattcaggt 720
   gatgcagtag atattatcgg tatgggtact gagaaattag cttctacagt aactggtatt 780
   gaaatgttcc gtcaaatctt agatagaggt gaggctggag ataacgcagg tatcttatta 840
   agaggtattg caaaagaaga tattaaaaga ggaatggtaa tctgtaagcc aggttctgta 900
   actccacacg ctaagtttaa ggctgaggtg tatgtattaa agaaagaaga aggtggacgt 960
   cacactccat tccataacaa ctatcgtcca cagttttacg tacgtacaac tgatgtaact 1020
   ggaaacatta tgcttcctga tggagttgaa atggttatgc ctggagataa cttaactatt 1080
   acagttgatt taattcagcc aattgcatta aacttaggtt tacagttcgc tatccgtgaa 1140
   ggaggtagaa cagtaggtgc aggtcaggta actgaaattt tagactaa 1188
<210> 48
   <211> 1004
   <212> DNA
   <213> T soleae
<400> 48
   ctactttaac tgctgcaatt acaaaagtat tagcagatgc aggattttct gaacaaagag         60
   cttttgatca aatcgataat gctccagaag aaaaagaaag aggtataaca attaactctt        120
   ctcacgtaga gtatgcaaca gctaaccgtc actacgcgca cgtagactgt ccaggtcacg        180
   ccgattacgt aaagaacatg gtaactggtg ctgcacaaat ggatggagct atcttagttg        240
   tagctgcaac tgatgggcca atgccacaaa ctcgtgagca catcttatta ggtcgtcagg        300
   taggtattcc acgtattgtt gttttcttaa acaaggtgga tatggtagat gatgaggagt        360
   tactagagtt agtagagatg gaagttagag atttattatc tttctatgaa tatgatggag        420
   ataatggacc tgtagtagct ggatctgctt taggagcttt aaatggagaa gagaaatggg        480
   taaatacagt tttagaatta atggaagctg ttgatacttg gattgaagag ccaccaagag        540
   aaactgaaaa agatttctta atgccaattg aagatgtatt ctcaattact ggtcgtggta        600
   ctgttgctac tggtcgtatt gaaacaggaa tcatcaactc tggtgatcca gttgaaatta        660
   tcggtatggg tactgagaag ttaacttcta cagtaactgg tattgaaatg ttccgtcaaa        720
   tcttagatag aggtgaggca ggagataacg caggtatctt gttaagaggt attgcaaaag        780
   aagacattaa aagaggaatg gtaatcgtta agccaggatc tgtaacacca cacgctaagt        840
   ttaaagctga ggtttatgtt ttaaagaaag aagaaggtgg acgtcacact ccattccata        900
   acaactatcg tccgcagttt tatgtgcgta caacagatgt aacaggtact attaacttac        960
   ctgatggagt agaaatggta atgcctggag ataacttaac tatt                        1004
<210> 49
   <211> 787
   <212> DNA
   <213> T dicentrarchi
<400> 49
   aggatctact tttgatatta ttgatattcc agaagattta aaagaagaag ctgctgaatt         60
   aagagggaaa cttattgaag aagttgctgc ttatgatgag aatcttttag aaaaatatat        120
   ggaagatgaa gattcaatta cagaagatga agtgcatgct gctcttcgtg ctgctgtaat 180
   ggatatgtct attattccta tgatttgtgg gtcggcattt aaaaataaag gtgttcagtt 240
   cttattagat gctgtttgtc gttatttacc ttctccttta gataaagaag caattattgg 300
   aacagatcct aacagtggtg aagagatttc tcgtaaacct gatgttaagg aaccatttgc 360
   agctttagca tttaaaattg ctacggatcc tttcgtagga cgtttagctt tctttagatc 420
   ttactcaggt agattagatg caggttctta cgtgttaaat aatcgttcag gtaaaaaaga 480
   gcgtatttct cgtatctatc aaatgcacgc taataagcaa aacgctattc cttatattga 540
   agcaggggat attggtgcag ctgtaggatt taaatcgatt aaaacaggag atactttatc 600
   tgatgaaaaa catcctattg ttttagaatc gatggatttt cctgatccag taattggtat 660
   tgcggttgag cctaaaacta aagcagatgt tgataaatta ggaatgtctt tagctaagtt 720
   agcagaggaa gaccctacat ttacagttag aactgatgaa gcttcaggac agactattat 780
   ttctgga 787
<210> 50
   <211> 791
   <212> DNA
   <213> T ovolyticum
<400> 50
   caacacaagg agcaacattc gatatcatcg atattccaga agatttaaaa gaagaagctg 60
   ccgagttaag agggaaactt attgaagagg ttgctgctta tgacgaaaat cttttagaaa 120
   aatatatgga agatgaagat tcaattactg aagaagaggt gcatgctgca cttcgtgctg 180
   cggtaatgga tatgtctatt attcctatga tttgtggatc tgcatttaaa aataaaggtg 240
   ttcagttctt attagatgct gtatgtcgtt atttaccatc tcctttagat aaagaagcta 300
   ttattggaac taatcctgat aacggtgaag aaatttctcg taaaccagat gttaaggagc 360
   ctttcgcagc attagctttt aaaattgcta ctgatccttt cgtaggtcgt ttagcattct 420
   ttagatctta ttcaggtaga ttagatgctg gatcttacgt tttaaataac cgttcaggta 480
   aaaaagaacg tatttctaga atttatcaaa tgcatgcaaa taagcaaaat gctatcgatt 540
   atatcgaagc tggagatatt ggtgcagcag taggttttaa atctattaaa actggagata 600
   ctttatctga tgaaaaacat cctattgttt tagaatctat ggatttccca gacccagtaa 660
   ttggtattgc ggttgagcct aaaactaaag cagatgttga taaattagga atgtctttag 720
   ctaagttagc agaagaagac cctacattta cagttagaac agacgaagct tcaggacaga 780
   ctattatttc t 791
<210> 51
   <211> 783
   <212> DNA
   <213> T. soleae
<400> 51
   acaaggtgct acttttgata tcgttgatat tccggaagat ttaaaagaag aagctgctga      60
   attaagagga aaacttattg aagaagttgc tgcttatgat gaaaatcttt tagaaaaatt     120
   tatggaagat gaagattcta ttactgaaga tgaagtgcat gctgctcttc gtgctgctgt     180
   aatggatatg tctatcattc ctatgatttg tggatcagcc tttaaaaata aaggagttca     240
   gttcttatta gatgctgtgt gtcgttactt accttcaccg gtagataagg atgcgattgt     300
   aggaacagat cctaatactg gtgatgagat ttctcgtaag ccagatgcaa aagagccttt     360
   ctctgcatta gcgtttaaaa ttgctacaga tcctttcgta ggtcgtttag catttttccg     420
   tgcatattca gggcgtttag atgcaggttc ttatgtgtta aataatcgtt caggtaaaaa     480
   agaacgtatt tcacgtatct accaaatgca ttctaacaag caaaatgcta tcgattatat     540
   tgaagcagga gatattggag ctgcagtagg ttttaaatct attaaaacag gagatacttt     600
   atctgatgaa aagaatccaa ttattttgga atctatggac tttcctgatc cagtaattgg     660
   tatcgcggta gagcctaaaa cgaaagcaga tgtcgataag ttagggttgt ctttagctaa     720
   attagcagaa gaagatccaa catttacagt aagaactgat gaagcttcag gacagactat     780
   tat                                                                   783
<210> 52
   <211> 1249
   <212> DNA
   <213> T.disentrarchi
<400> 52
   tgtagtgctt cggcatcgga acactattaa acatttatgg gtacaagatg actatgcgtc      60
   ctattagcta gatggtaagg taacggctta ccatggcaac gataggtagg gggtctgaga     120
   ggattatccc ccacactggt actgagacac ggaccagact cctacgggag gcagcagtga     180
   ggaatattgg tcaatggagg caactctgaa ccagccatgc cgcgtgcagg atgactgccc     240
   tatgggttgt aaactgcttt tatacaggaa gaaaccgatc tacgtgtaga tccttgacgg     300
   tactgtaaga ataaggaccg gctaactccg tgccagcagc cgcggtaata cggagggtcc     360
   gagcgttatc cggaatcatt gggtttaaag ggtccgcagg cggtcaatta agtcagaggt     420
   gaaatcccat cgctcaacga tggaactgcc tttgatactg attgacttga gttatatgga     480
   agtagataga ataagtagtg tagcggtgaa atgcatagat attacttaga ataccgattg     540
   cgaaggcagt ctactacgta tatactgacg ctgagggacg aaagcgtggg gagcgaacag     600
   gattagatac cctggtagtc cacgccgtaa acgatggata ctagttgttg gacttcggtt     660
   cagtgactaa gcgaaagtga taagtatccc acctggggag tacggtcgca agactgaaac     720
   tcaaaggaat tgacgggggc ccgcacaagc ggtggagcat gtggtttaat tcgatgatac     780
   gcgaggaacc ttaccagggc ttaaatgtgg tctgacagct ttagagatag agttttcttc     840
   ggacagatca caaggtgctg catggttgtc gtcagctcgt gccgtgaggt gtcaggttaa     900
   gtcctataac gagcgcaacc cctattttta gttgctaaca ggttaagctg aggactctag     960
   agagactgcc ggtgcaaacc gtgaggaagg tggggatgac gtcaaatcat cacggccctt    1020
   acgtcctggg ctacacacgt gctacaatgg tatggacaat gagcagccac tatgcgaata    1080
   ggagcgaatc tataaaccat atcacagttc ggatcggagt ctgcaactcg actccgtgaa    1140
   gctggaatcg ctagtaatcg gatatcagcc atgatccggt gaatacgttc ccgggccttg    1200
   tacacaccgc ccgtcaagcc atggaagctg ggggtgcctg aagttcgtt                 1249
<210> 53
   <211> 1408
   <212> DNA
   <213> T. ovolyticum
<400> 53
   caagtcgagg ggtaacaggt agcttgctat gctgacgacc ggcgaacggg tgcgtaacgc      60
   gtatagaatc tgccttgtac aggaggatag cctttagaaa tgaagattaa tactccataa     120
   tgttaattag aggcatctct tattaattaa acatttatgg gtacaagatg actatgcgtc     180
   ctattagcta gatggtaagg taacggctta ccatggcaac gataggtagg gggtctgaga     240
   ggattatccc ccacactggt actgagacac ggaccagact cctacgggag gcagcagtga     300
   ggaatattgg tcaatggagg caactctgaa ccagctatgc cgcgtgcagg aagactgccc     360
   tatgggttgt aaactgcttt tatacaggaa gaaacagtag tacgtgtact accttgacgg     420
   tactgtaaga ataaggaccg gctaactccg tgccagcagc cgcggtaata cggagggtcc     480
   aagcgttatc cggaatcatt gggtttaaag ggtccgcagg cggtcaatta agtcagaggt     540
   gaaagcccat cgctcaacga tggaactgcc tttgatactg gttgacttga gttatacgga     600
   agtagataga ataagtagtg tagcggtgaa atgcatagat attacttaga ataccgattg     660
   cgaaggcagt ctactacgta tatactgacg ctcatggacg aaagcgtggg gagcgaacag     720
   gattagatac cctggtagtc cacgccgtaa acgatggata ctagttgttg ggtttcgact     780
   cagtgactaa gcgaaagtga taagtatccc acctggggag tacggtcgca agactgaaac     840
   tcaaaggaat tgacgggggc ccgcacaagc ggtggagcat gtggtttaat tcgatgatac     900
   gcgaggaacc ttaccagggc ttaaatgtgg tctgacagct ttagagatag agttttcttc     960
   ggacagatca caaggtgctg catggttgtc gtcagctcgt gccgtgaggt gtcaggttaa    1020
   gtcctataac gagcgcaacc cctattgtta gttgctaaca ggtcatgctg aggactctag    1080
   cgagactgcc ggtgcaaacc gtgaggaagg tggggatgac gtcaaatcat cacggccctt    1140
   acgtcctggg ctacacacgt gctacaatgg tatggacaat gagcagccac tatgcgaata    1200
   ggagcgaatc tataaaccat atcacagttc ggatcggagt ctgcaactcg actccgtgaa    1260
   gctggaatcg ctagtaatcg gatatcagcc atgatccggt gaatacgttc ccgggccttg    1320
   tacacaccgc ccgtcaagcc atggaagctg ggagtgcctg aagttcgtta ccgtgaggag    1380
   cgacctaggg taaaactggt aactaggg                                       1408
<210> 54
   <211> 1221
   <212> DNA
   <213> T. soleae
<400> 54
   taatgctaat atgtggcatc acaaattagt taaagattta tcggtacaag atgactatgc      60
   gtcctattag ctagatggta aggtaacggc ttaccatggc aacgataggt agggggtctg     120
   agaggattat cccccacact ggtactgaga cacggaccag actcctacgg gaggcagcag     180
   tgaggaatat tggtcaatgg aggcaactct gaaccagcca tgccgcgtgc aggatgactg     240
   ccctatgggt tgtaaactgc ttttatacag gaagaaaccg atgtacgtgt acatccttga     300
   cggtactgta agaataagga ccggctaact ccgtgccagc agccgcggta atacggaggg     360
   tccaagcgtt atccggaatc attgggttta aagggtccgc aggcggtcaa ttaagtcaga     420
   ggtgaaatcc catagcttaa ctatggaact gcctttgata ctggttgact tgagttatac     480
   ggaagtagat agaataagta gtgtagcggt gaaatgcata gatattactt agaataccga     540
   ttgcgaaggc agtctactac gtatatactg acgctcatgg acgaaagcgt ggggagcgaa     600
   caggattaga taccctggta gtccacgccg taaacgatgg atactagttg ttgggttaat     660
   tctcagtgac taagcgaaag tgataagtat cccacctggg gagtacggtc gcaagactga     720
   aactcaaagg aattgacggg ggcccgcaca agcggtggag catgtggttt aattcgatga     780
   tacgcgagga accttaccag ggcttaaatg tagagtgaca ggggtagaga tatctttttc     840
   ttcggacact ttacaaggtg ctgcatggtt gtcgtcagct cgtgccgtga ggtgtcaggt     900
   taagtcctat aacgagcgca acccctattt ttagttgcta acaggttaag ctgaggactc     960
   tagagagact gccggtgcaa accgtgagga aggtggggat gacgtcaaat catcacggcc    1020
   cttacgtcct gggctacaca cgtgctacaa tggtatggac aatgagcagc cactatgcga    1080
   ataggagcga atctataaac catatcacag ttcggatcgg agtctgcaac tcgactccgt    1140
   gaagctggaa tcgctagtaa tcggatatca gccatgatcc ggtgaatacg ttcccgggcc    1200
   ttgtacacac cgcccgtcaa g                                               1221
<210> 55
   <211> 6768
   <212> DNA
   <213> T. finnmarkense
<400> 55
   ttagaaatta aaaaagctga tggttcaatt ttagtattag aggttcaaca acatataggt      60
   gaagatacag ttcgtactat ttcgatggat gcaactgatg gtttaagcag aggtacagaa     120
   gttatcgcta cgggtaatcc aattcaaatg ccaataggta aagatattta cggacgttta     180
   tttaacgtta ctggtgaagc tattgatggt ttaggtaatt tacctaaaac aggagatgat     240
   ggtttatcaa tccaccgtca agcacctaaa tttgaagact tatcagtgtc aactgaagtt     300
   ttatttactg gtattaaagt aattgattta attgagccgt atgcaaaagg aggtaaaatt     360
   ggattatttg gtggagcagg agtaggtaaa accgtattaa ttcaggagtt aattaacaac     420
   attgcaaaag gacacggagg tttatctgta ttcgcaggtg ttggtgaaag aactcgtgaa     480
   ggaaacgatt tattaagaga gatgttagaa tctggaatta tcaaatatgg tgatgatttt     540
   atgcattcta tggaagaagg aggatgggat ttatctaaag ttgataaacc aggaatgaga     600
   gactctaaag ctactttcgt attcggacaa atgaacgaac cacctggagc acgtgcacgt     660
   gttgcattat ctggtttatc tattgctgaa tatttccgtg atggagcagg agaagcacaa     720
   ggaaaagatg ttcttttctt cgttgataac atcttccgtt ttacacaagc aggttctgag     780
   gtatcggcat tattaggtcg tatgccagta ttagctgatg caggattctc tgaagctaga     840
   gattttgatc aaatcgataa tgcaccagaa gaaaaagaaa gaggtattac aattaactct     900
   tctcacgttg aatatgcaac agaaaaccgt cattacgctc acgtagactg tccaggtcac     960
   gcggattacg taaagaacat ggtaactggt gctgctcaaa tggatggagc tattttagta    1020
   gtagctgcaa ctgatggtcc tatgccacaa acacgtgagc acattttatt aggtcgtcaa    1080
   gtaggtattc ctcgtatggt tgttttcttg aataaagttg acatggttga tgatgaggag    1140
   ttacttgagt tagtagaaat ggaagtaaga gaattattat ctttctatga gtacgatgga    1200
   gataatggtc ctgtagtttc aggttcagct ttaggagctt taaatggtga agaaaaatgg    1260
   gttaatactg ttttagaatt aatggctgct gttgatgctt ggattgaaga gcctttaaga    1320
   gaaactgaaa agcctttctt aatgcctatt gaagatgtat tctctattac tggtcgtggt    1380
   actgttgcaa ctggtcgtat tgaaacagga atcatcaatt ctggtgaatc tgtagatatt    1440
   attggtatgg gtactgagaa gttaacttct acagttactg gtattgaaat gttccgtcaa    1500
   atcttagata gaggtgaggc tggagataat gcaggtatct tattaagagg tattgctaaa    1560
   gaagatatta aaagaggaat ggtaatctgt aagccaggtt ctgtaactcc acacgctaag    1620
   tttaaggctg aggtgtatgt attgaaaaaa gaagaaggtg gacgtcacac tccattccat    1680
   aataactatc gccctcagtt ttacgtacgt actactgatg taacaggaaa tattttcttg    1740
   cctgaaggtg tagaaatggt tatgcctggt gataacttaa aagcaaccat tattaaagtt    1800
   ttagaagatg gtggaagctg tgttttaatg tcgcatttag ggcgtcctaa agctcaagaa    1860
   gaggaatttt cattacgtca tattgtagca gaagtaacta aaacgttagg tgttcaagta 1920
   aaatttgtga acgattgtat cggtgataaa gttgaagaag ctatcgctag tttacaaaat 1980
   ggcgaaattt tattattaga aaatttacgt ttttatcctg aagagaaagc aggagatgtt 2040
   gcttttgctg aaaaattagc aaatttggga gatatttatg taaatgatgc ttttggaaca 2100
   gcacacagag cgcacgcttc tacggctgta attgcgcaat tttttactga taacaaatgt 2160
   tttggtaatt tattatcaac agaaatagaa agtattaata aagttttaaa tgatgccgaa 2220
   aaacctgtaa cagctatttt aggaggcgca aaagtatcct ctaaaatagg cgttattgaa 2280
   aatattattg aaaaagtaga ccacattatt gttggtggag gaatgacttt tacttttata 2340
   aaagctttag gcggaaacat cggaaattca ttagttgaag atgataaatt aagtttagca 2400
   ttggctattt tgaaattagc aaaagaaaaa aatacagaaa tccatttacc tgttgatgct 2460
   gtaattgctg atgctttttc taatgatgca aatacgcaaa ttgtggatac tacagaaatt 2520
   cctgaaggat ggatggggct tgattgtgga ccaaaaactt cagaaaattt tgcagaagta 2580
   atagctaaat cgaaaactat tttatggaac ggacctttag gtgtttttga attagaaaaa 2640
   tttgcaacag gaactattga attaggaaac gctatttcaa aagcaactga aaatggtgct 2700
   ttctctttag ttggaggttt aacacatagt gttcctttaa aagcaagatt aaagctttac 2760
   tgtacagatc ctgaacacga agattttgaa actattgttc aagatgtgta tttaggaacg 2820
   attccttata tgacaaattc tggaaccttc attattaatg gagctgaacg agttgttgta 2880
   tctcaattac accgttcacc aggggtgttt tttggacaat ctttccatgc aaacggaact 2940
   aagttgtatt ctgcaagagt aattcctttt aaaggatctt ggatagaatt tgcaactgat 3000
   atcaatcaag taatgtatgc ttatattgat agaaagaaaa aactaccagt aactacgtta 3060
   ttcagagcca ttggttttga aagagataaa gatatattag aaatttttga tttagcagag 3120
   gaaattaagg tttctaaagc aggattaaaa aaagtattag ggcgtaaatt agctgctaga 3180
   gttttaaaaa cttggcatga agatttcgtt gatgaagata ccggtgaagt ggtgtctatt 3240
   gaacgtaacg aaattgtttt cgaccgtgat accatcatag agaaagaaca tattgatgaa 3300
   ataattgacg caggtgctaa aactatctta ttacataagg tagataatga catggccgat 3360
   tatgccatca ttcacaatac actgcagaaa gatcctacga attctgaaaa agaagcagta 3420
   gaacacgtct acagacaatt acgtaatgct gagccgccag atgaggagac agcaagagga 3480
   attattgaaa aattattctt ctctgagcaa cgttatagtt taggtgaagt tggtcgtttt 3540
   agaatgaaca ccaagttaca gttaaatgag ccgattgatc agaaggtatt aactaagaat 3600
   gacattatta ccattatcaa atacttaatt gagttaatta actcaaaagc agaagtagat 3660
   gatattgacc atttatctaa ccgtcgtgta agaactgttg gtgaacaatt agcaggtcaa 3720
   tttggtgtag gtttagctcg tatggctcgt actattcgtg aaagaatgaa tgtgcgtgat 3780
   aacgaggtat ttactcctat tgatttaatt aatgcgaaga ccttatcttc agtaattaat 3840
   tcgttttttg gaactaacca gttatctcaa tttatggatc aaaccaatcc attagctgag 3900
   attacgcaca aacgtcgttt atcagcacta ggacctggag gtttatctag agaaagagca 3960
   ggatttgagg ttcgtgatgt tcactataca cactacggtc gtttatgtcc aattgaaact 4020
   cctgagggac cgaatattgg attaatttca tctttagcag tatttgcaaa ggttaacagt 4080
   ttaggtttta tcgaaacacc ttataaaaag gttatcgaag gaaatgtaga tgtacaccaa 4140
   gagcatatat atttaagtgc tgaagaagaa gaaggaatga agtttgcaca atctaattta 4200
   gaggtaagtg cagctggaga ttttgttgga gataaattaa tttcacgtga agggggagat 4260
   ttccctgtgg taacacctac tgaaattgat tatatggatg ttgcacctaa ccagattgca 4320
   tctatatcag catcattaat tcctttctta gagcatgatg atgccaatag agccttaatg 4380
   ggatcgaaca tgatgcgtca agcagttcca ttattgcgtc cagaatcacc aattgtaggt 4440
   acaggattag agcgtagagt tgcaaaagat tctcgtattt taatcaatgc tgaaggacct 4500
   ggagttattg agtatgttga tgcgaataaa attagaatta agtatgacag atcagatgaa 4560
   gagcgtttag ttagctttga atcagatgat atttcatata acttaattaa gtttagaaaa 4620
   acgaatcaag gtacgaacat taacttaaag ccaattgtaa aagttggaga tagagttcaa 4680
   gaaggtcaag tactttgtga aggttatgca acacaaaaag gagaattagc tttaggaaga 4740
   aatatgaaag tagcctttat gccttggaaa gggtataact ttgaggatgc gattgtaatt 4800
   tcagaaaaag tggttcgtga agatattttc acttctattc atattgacga atattcatta 4860
   gatgttcgtg atactaaatt aggagctgaa gaattaacaa acgatattcc taatgtttca 4920
   gaagaggcta ctaaagactt agatgaaaac ggaatgatcc gtattggagc agaggtaaac 4980
   cctggtgata tcttaattgg taaaattaca ccaaaaggag aatctgatcc aactcctgaa 5040
   gaaaaattat tacgcgctat ttttggtgat aaagcaggtg atgtaaaaga tgcatcatta 5100
   aaagcttctc cgtcattaag aggtgtagta attaataaaa aattatttca acgtgctgta 5160
   aaagataaga caaaacgtgc tcgtgataaa gaagcaattg ctactttaga agcatcttat 5220
   gttcataagt tcgaagattt aaaagattta ttaattgaaa agttattcaa cttaattaca 5280
   gggaaaactt ctcaaggagt atttaatgat ttaggagagg aagtattacc aaaaggtaaa 5340
   aaattcactc aaaaaatgtt aaactctgta ggtgatttta cgcacttaca cggaacttgg 5400
   actaccgata aagacttaaa tagattagtt gttgaattag ttcacaatta taaaattaaa 5460
   gtaaacgatt tacaaggttc tttacgtcgt gagaaattta caatttctgt aggagatgaa 5520
   ttaccagcag gaatcttaaa acttgccaaa atttatgttg ctaaaaaacg taaattaaaa 5580
   gtaggagata agatggcagg acgtcatgga aataaaggta ttattgctcg tattgttaga 5640
   gcagaagata tgcctttctt agaagacgga acacctgttg atatcgtatt aaatccatta     5700
   ggggtaccat cgcgtatgaa tattggtcag atttatgaaa ctgttcttgg atgggcaggt     5760
   caaaaattag atcaaaaata tgcgacacca atttttgatg gagcaaacat tgaccaaatt     5820
   aacgctttaa ctgatcaagc aggaattcca cgttacggac atacctattt atatgatgga     5880
   ggaacaggaa aacgtttcga ccaaccagca acggtaggga tcatttatat gattaagtta     5940
   ggtcacatga ttgaagataa aatgcatgcg cgttctatcg gaggagctac atttgatatc     6000
   gttgatattc cagaagattt aaaagaagaa gctgctgaat taagagggaa actaattgaa     6060
   gaagttgcta gttacgatga gaatctttta gagaaattca tggaagatga agactcaatt     6120
   actgaagagg aagtgcatgc tgcacttcgt gctgctgtaa tggatatggc tatcattcct     6180
   atgatttgtg gatcagcatt taaaaataaa ggtgttcagt tcttattaga tgctgtttgt     6240
   cgttatttac cttctccaat ggataaggaa gctgttatcg ggacaaatcc tgatacaaac     6300
   gaagaagaaa gaagaaagcc agatgtaaag gagccttttg ctgctttagc atttaaaatt     6360
   gctactgatc ctttcgtagg tcgtttagca ttttttagag cttattctgg gcgtttagac     6420
   gctggttctt atgttttaaa taatcgttca ggtaaaaaag aacgtatttc tcgtatctat     6480
   caaatgcatg ctaacaagca aaacgctatt ccttacattg aagcagggga tatcggtgca     6540
   gctgtaggat ttaaatcgat taaaacagga gatactttat ctgatgaaaa gcatcctatt     6600
   gttttagaat ctatggactt ccctgatccc gtaattggta tcgcggttga gcctaaaact     6660
   aaagcagatg ttgataaatt aggaatgtct ttagctaagt tagcagaaga agatcctaca     6720
   tttacagtta gaactgatga agcttcagga cagactatta tttctgga                  6768

## Description

The present invention relates to a novel fish pathogen, in particular a novel *Tenacibaculum* specie involved in the development of tenacibaculosis in farmed fish. More particularly, the present invention relates to a vaccine useful in providing protection against tenacibaculosis in farmed fish.

The present invention furthermore relates to novel nucleic acid sequences and oligonucleotide sequences useful inter alia in for determination of the presence of *Tenacibaculum* in e.g. a sample taken from a fish having symptoms of winter ulcers or suspected to be infected with *Tenacibaculum.*

### The technical field

The Norwegian aquaculture industry has for the last three decades been affected by several ulcerative diseases. One of the most economically costly is the disease commonly known as "Winter ulcers", a disease which affects salmonids in seawater and has been recognized since the early 1980's (cf. Lunder, T., et al., Winter ulcer in the Atlantic salmon Salmo salar. Pathological and bacteriological investigations and transmission experiments. Diseases of Aquatic Organisms, 1995. 23(1): p. 39-49). Clinical symptoms typically consist of ulcers on the scale-covered parts of the body, frequently located along the side of the fish, and typically appear during low sea water temperatures (Lunder et al.(1995), *supra,* Salte, R., et al. (1994), Winter ulcers of the skin in Atlantic salmon, Salmo salar L.: pathogenesis and possible aetiology. Journal of Fish Diseases, 17(6): p. 661-665. Winter ulcers are causing a significant financial loss due to e.g. downgrading of slaughter, as well as being an ethical problem of farmed fish suffering from large ulcers after outbreak of the disease.

Winter ulcer is also a problem in the fish farming industry in Canada, the Faroe Islands, Iceland, Ireland, Main in the USA and Scotland (Bruno et al., (1998), Gudmunsdóttir, B.K. et al (2006), J Fish Dis, 29, p. 481 - 487, Whitman et al., (2000), Aquacul. Assoc. Canada Spes. Publ., No. 4, 115 - 117).

The main causative agent for winter ulcers is considered to be the Gram-negative bacteria *Moritella viscosa* (launder et al., (1995), *supra,* Salte et al. (1994), *supra,* Bruno, D.W., et al., Vibrio viscosus in farmed Atlantic salmon Salmo salar in Scotland: field and experimental observations. Diseases of Aquatic Organisms, 1998. 34(3): p. 161-166, order *Alteromonadales,* which has been shown to experimentally recreate the pathology observed in field cases (Løvoll, M., et al., Atlantic salmon bath challenged with Moritella viscosa - Pathogen invasion and host response. Fish & Shellfish Immunology, 2009. 26(6): p. 877-884). *M. viscosa* has been found to be genetically heterogeneous, divided into two genotypes based on gyrB sequence analysis (Grove, S., et al., Previously unrecognised division within Moritella viscosa isolated from fish farmed in the North Atlantic. Dis Aquat Organ, 2010. 93(1): p. 51-61). The type strain (NCIMB 13584) is assumed to have specificity for Atlantic salmon, while a variant strain shows specificity for Rainbow trout (Grove et al, *supra).*

An inactivated vaccine targeting *M. viscosa* has been developed and is included in multivalent vaccines used in Norway (Greger, E. and T. Goodrich, Vaccine development for winter ulcer disease, Vibrio viscosus, in Atlantic salmon, Salmo salar L. Journal of Fish Diseases, 1999. 22(3): p. 193-199). However, outbreaks of winter ulcer are still seen in vaccinated salmonids (Gudmunsdóttir, B. K. and Bjørnsdóttir, B, (2006), Vaccine, 25:30, p. 5512 - 5523). It is thus reason to question the role of *M.viscosa* as causative agent in all winter ulcer disease cases.

Based on the number of winter ulcer outbreaks recorded in Norway the last 10 years, there is still a need for an improved vaccine to combat other unknown pathogens involved in the development in ulcers in farmed fish, in particular salmonids. Interestingly, several types of bacteria are typically isolated from ulcers of farmed fish, including *Aliivibrio wodanis* (Lunder et al. (1995), *supra,* Lunder, T., et al., Phenotypic and genotypic characterization of Vibrio viscosus sp. nov. and Vibrio wodanis sp. nov. isolated from Atlantic salmon (Salmo salar) with 'winter ulcer'. International Journal of Systematic and Evolutionary Microbiology, 2000. 50(2): p. 427-50, Benediktsdóttir, Helgason, and Sigurjónsdóttir, Vibrio spp. isolated from salmonids with shallow skin lesions and reared at low temperature. Journal of Fish Diseases, 1998. 21(1): p. 19-28) and *Tenacibaculum sp.* (Salte et al. (1994), *supra,* Olsen, A.B., et al., Tenacibaculum sp. associated with winter ulcers in sea-reared Atlantic salmon Salmo salar. Dis Aquat Organ, 2011. 94(3): p. 189-99). *A. wodanis* has however been shown to be non-pathogenic in challenge experiments (Lunder et al. (1995), *supra,* Benediktsdóttir et al. (1998), *supra),* but may play a role in suppressing the healing process of skin ulcers infected by *M.viscosa* (Olsen, A.B., et al., (2011), *supra). A.wodanis* has however, recently been shown to induce *in vitro* cytopathogenic effect in fish cell lines, in addition to invasion of Atlantic salmon in challenge studies (Toranzo, A.E., B. Magariños, and J.L. Romalde, A review of the main bacterial fish diseases in mariculture systems. Aquaculture, 2005. 246(1-4): p. 37-61. The use of *A. wodanis* in a vaccine for prevention of wodanosis and/or winter ulcer has also been suggested in a vaccine, cf. WO2013/171236.

Although *M. viscosa* has been shown to be an important factor in the pathogenesis of winter ulcers, routine histopathological examination at the Norwegian National Veterinary Institute of Bergen (NVIB) has shown the presence of long, slender bacteria in skin ulcers in farmed Atlantic salmon, appearing either as a solitary or mixed infection with *M. viscosa* (Olsen et al. (2011), *supra).* In Olsen et al. (2011), a bath challenge study with *Tenacibaculum* sp. is reported using two isolates of *Tenacibaculum* sp.. In addition, a *Moritella* viscosa-isolate from Atlantic salmon suffering from winter ulcers was included. The bath challenge was performed in 3 different experiments with smoltified Atlantic salmon of an average weight of 250 to 300 grams. The water temperatures used in this challenge study were 9, 5 and 12 °C, respectively. Importantly, because the fish used in the study was inflicted with severe skin abrasions (i.e. scraping with a scalpel leaf), the potential pathogenicity of the isolates remain uncertain. The causative agent of the ulcers developed in this study remains unclear. It is also unknown whether the isolates disclosed in Olsen et al. are involved in development of tenacibaculosis. Whether or not *Tenacibaculum* sp. isolates are able to induce ulcers in the absence of pre-existing wounds and the isolates applicability in the development of a vaccine, is therefore unknown.

Thus, it is still a need for therapeutic agents useful in prevention of tenacibaculosis salmonids as well as diagnostic tools useful in determine the presence of pathogens involved in the development of ulcers in salmonids.

As discussed above, the fact that the development of ulcer are seen in salmonids vaccinated against *M. viscosa* infections in order to prevent the development of winter ulcer, show that there is also a need for diagnostic tools enabling the detection of the presence of the pathogens involved in development of ulcers in farmed fish not caused by *M. viscosa,* such as the ulcers seen in tenacibaculosis affected fish.

### Summary of invention

It is an object of the invention to provide a novel bacterium isolate that may be used in vaccines in order to solve, or at least mitigate the problem with tenacibaculosis in farmed fish as well as providing diagnostic tools useful in determining the presence of bacteria causing tenacibaculosis and ulcers being a result of *Tenacibaculum* infections in farmed fish.

The present invention provides for the first time a *Tenacibaculum* isolate that when used in a challenge study enables to reproduce mortality and tenacibaculosis both in cohabitant fish as well as in fish infected with the isolate of the invention by bath. Importantly, the *Tenacibaculum* isolate according to the present invention induce mortality in a challenging model in fish that that are not inflicted with damage prior to challenge, and not infected with other known fish pathogenic bacteria causing ulcers.

Furthermore, the present invention includes a *Tenacibaculum* isolate belonging to a novel *Tenacibaculum* species characterized for the first time herein, and which are shown to be involved in development of tenacibaculosis and ulcers in salmonids.

In particular, the invention provides a *Tenacibaculum* isolate involved in the development of tenacibaculosis in farmed fish, wherein said isolate is deposited under the Budapest Treaty with the Leibniz Institute DSMZ-German Collection of Microorganisms and Cell Cultures (DSMZ) on 4^{th} March 2014 under deposit number DSM 28541.

The *Tenacibaculum* isolate according to the present invention have been shown to be involved in the development of tenacibaculosis in farmed fish, in particular salmonids, such as *Salmo salar.* It has also been shown that the isolate of the present invention may be involved in the development of winter ulcer in farmed fish. Due to the fact that the *Tenacibaculum* isolate of the present invention causes mortality, tenacibaculosis and development of ulcers in a challenge model, the present isolate is strong candidate for the development of a fish vaccine useful in providing protection against said disease in farmed fish. Accordingly, the invention includes a vaccine comprising a *Tenacibaculum* isolate according to the present invention, such as e.g. an attenuated, killed or inactivated form of said isolate. The isolate of the present invention may also be included in a combination vaccine together with antigen material originating from other fish pathogenic organisms, such as e.g. microorganism, fungus, amoebae, parasitic or virus being pathogenic to farmed fish.

Disclosed herein is also nucleotide sequences and oligonucleotide sequences. In particular, polynucleotides of the present invention comprise a sequence selected from the group consisting of
(a) SEQ ID No. 1 encoding a 16sRNA gene;
(b) SEQ ID No. 2, encoding an atpD gene; SEQ ID No. 4, encoding a pgk gene; SEQ ID No. 5, encoding a TUF gene, and SEQ ID No. 6, encoding a fusA gene.

The sequences disclosed herein are inter alia useful in methods for determination of tenacibaculosis infections in farmed fish. The present invention thus disclose for the first time oligonucleotide sequences enabling a definite diagnosis of tenacibaculosis in farmed fish. The providing of a method useful in determining the presence of the *Tenacibaculum* in fish affected with ulcers is an important tool in order to distinguish between winter ulcer caused by *M. viscosa* infection and ulcers caused by *Tenacibaculum* infection. Being able to distinguish between a winter ulcer caused by *M.viscosa* infection and ulcers being the results of tenacibaculosis is furthermore important in order to initiate correct and appropriate treatment of the infected fish, e.g. in respect of selecting the most proper antibiotic treatment. Selecting the most appropriate antibiotic treatment adapted to the bacteria causing the disease of question in farmed fish is both of economic as well as environmental value. Being able to distinguish between these two pathogens is also important in respect of the selection of vaccine for fish prior to stocking of fish in a cage that includes or has included fish infected with either bacterium.

The present invention furthermore includes a method for identifying in a sample, a *Tenacibaculum* bacterium involved in development of tenacibaculosis, wherein said *Tenacibaculum* belong to the same species as the *Tenacibaculum* isolate deposited under the Budapest Treaty with the Leibniz Institute DSMZ-German Collection of Microorganisms and Cell Cultures (DSMZ) on 4^{th} March 2014 under deposit number DSM 28541, wherein said method comprises hybridization under stringent conditions of an oligonucleotide to single stranded nucleic acid sequences originating from a biological sample isolated from a fish suspected of having tenacibaculosis, wherein said oligonucleotide comprising a sequence of at least 10 nucleotides, wherein said oligonucleotide sequence hybridize to a nucleic acid sequence originating from *Tenacibaculum.*

A method is also provided disclosed comprising the steps of extracting nucleic acid sequences from said sample, combining the extracted nucleic acid sequences with one or more oligonucleotide sequence according to the present invention, and wherein hybridization of the oligonucleotide sequence selectively to a portion of the extracted nucleic acid sequences or a complementary sequence thereof, respectively, indicates the presence of *Tenacibaculum* in the sample.

Another method is also disclosed, comprising detecting the presence of one or more Tenacibaculum nucleic acid sequences by
a) obtaining a biological sample from a fish suspected of having tenacibaculosis wherein said biological sample comprises nucleic acid sequences;
b) extracting nucleic acid sequences from said biological sample and contacting said nucleic acid sequences with a reverse transcriptase to produce double-stranded nucleic acid sequence comprising said nucleic acid sequence and its complementary strand;
c) a cycle comprising the following steps:
   c1) denaturing of the obtained double-stranded nucleic acid sequence, forming single-stranded nucleic acids;
   c2) hybridizing of each of said single-stranded nucleic acids with at least one oligonucleotide sequence according to any one of claims 17-20, by placing said single-stranded nucleotide sequence in contact with said at least one oligonucleotide sequence under stringent hybridization conditions;
   c3) amplifying said single-stranded nucleic acids by elongation of the oligonucleotide sequence along the strands to which they are hybridized in the presence of a polymerase, dATP, dGTP, dCTP and dTTP, said cycle being repeated about 10 to 60 times; and
d) detecting the presence of amplified nucleic acid sequence of *Tenacibaculum.*

According to one embodiment of the present method, said method comprises the steps of extracting nucleic acid sequences from said sample, combining the extracted nucleic acid sequences with one or more of said oligonucleotide sequence, and wherein hybridization of the oligonucleotide sequence selectively to a portion of the extracted nucleic acid sequences or a complementary sequence thereof, respectively, indicates the presence of *Tenacibaculum* in the sample. According to another embodiment of the present method, said oligonucleotide hybridize to SEQ ID No. 1. According to yet another embodiment of the present method, said oligonucleotide sequence selected from the group consisting of SEQ ID No. 7 - SEQ ID No. 28 and variants of any of the sequences SEQ ID No. 7 - SEQ ID No. 28, respectively, having at least 97% sequence identity with the said sequences SEQ ID No. 7-28, respectively, based on the entire sequence length of said sequences.

### Figures

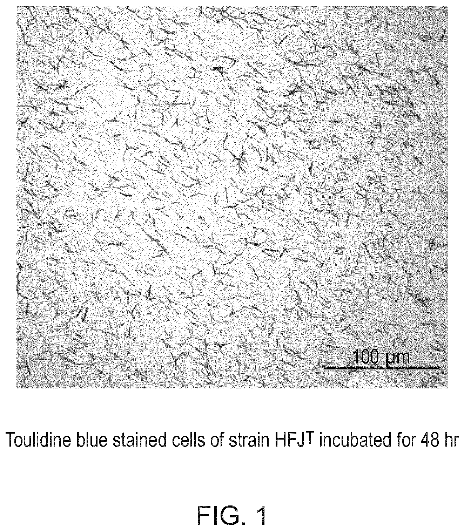

**Figure 1** show Toulidine blue stained cells of strain HFJ^{T} (DSM 28541) incubated for 48 hours.

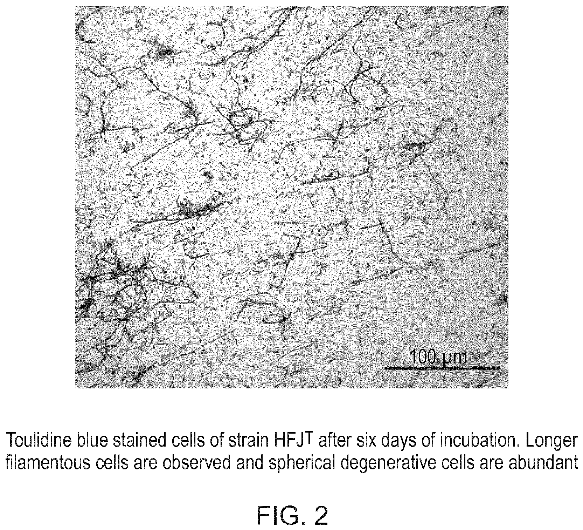

**Figure 2** show Toulidine blue stained cells of strain HFJ^{T} (DSM 28541) after six days of incubation. Longer filamentous cells are observed, and spherical degenerative cells are abundant.

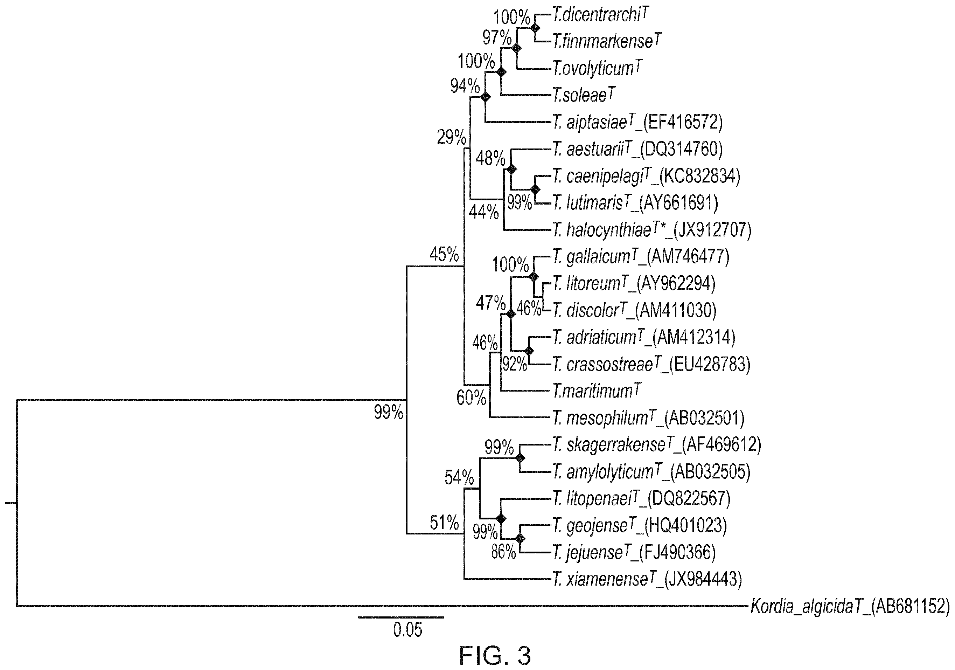

**Figure 3** show the evolutionary history from the 16s rDNA gene sequeces of the novel species *T. finnmarkense^{T},* the 21 type strains in genus *Tenacibaculum* (* = not validly published), when using the outgroup *Kordia algicida*^{T}. The phylogenetic analysis was inferred using the Bayeshian method with the best fitted evolutionary model (GTR+G+I). The posterior probability is presented next to each node in percentage. There were a total of 1341 positions in the dataset. Evolutionary analyses were conducted using the Beasts package. Share nodes identified in corresponding maximum likelihood analysis are identified by filled squares.

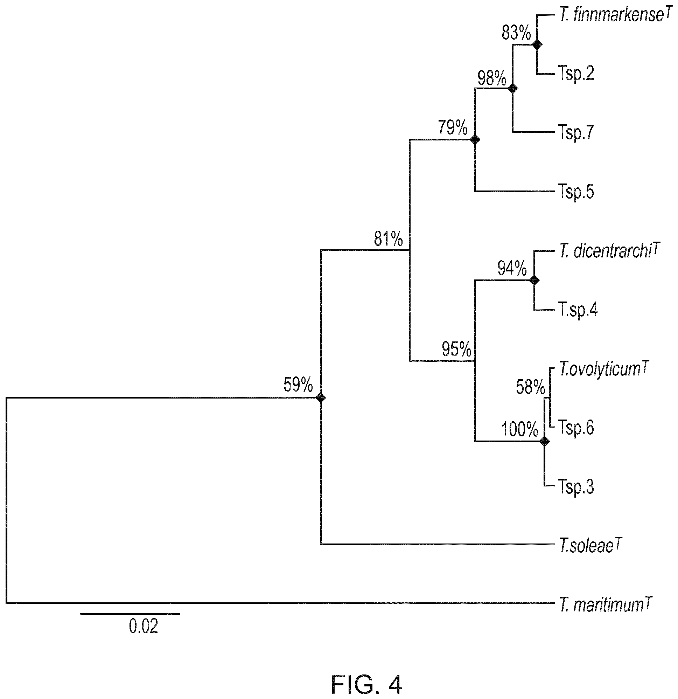

**Figure 4** show the evolutionary history of the 16s rDNA gene sequences from the novel species *T. finnmarkense^{T},* isolates Tsp.2-7 and their three closest related type strains using *T. maritimum*^{T} as outgroup. The phylogenetic analysis was inferred using the Bayeshian method with the best fitted evolutionary model (HKY+G+I). The posterior probability is presented next to each node in percentage. There were a total of 1349 positions in the dataset. Evolutionary analyses were conducted using the Beasts package. Share nodes identified in corresponding maximum likelihood analysis are identified by filled squares

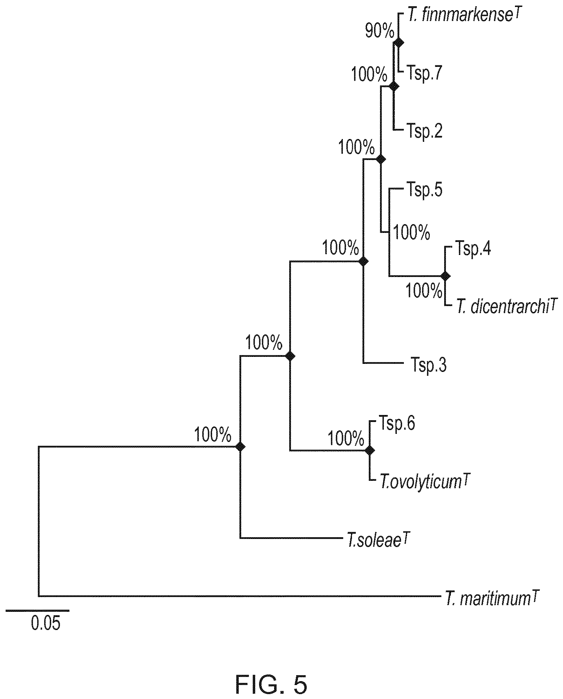

**Figure 5** shows the evolutionary history of five housekeeping genes (HK) in a concatenated sequence (Tuf, rpoB, Pgk, fusA and atpD) from the novel species T. *finnmarkense*^{T}, isolates Tsp. 2-7 and their three closest related type using *T. maritimum^{T}* as outgroup. The phylogenetic analysis was inferred using the Bayeshian method with the best fitted evolutionary model for each HK. The posterior probability is presented next to each node in percentage. There was a total of 6954 positions in the dataset. Evolutionary analyses were conducted using the KAKUSAN4 and MrBayes V3.2 (Ronquist et al., 2012) Share nodes identified in corresponding maximum likelihood analysis are identified by filled squares.

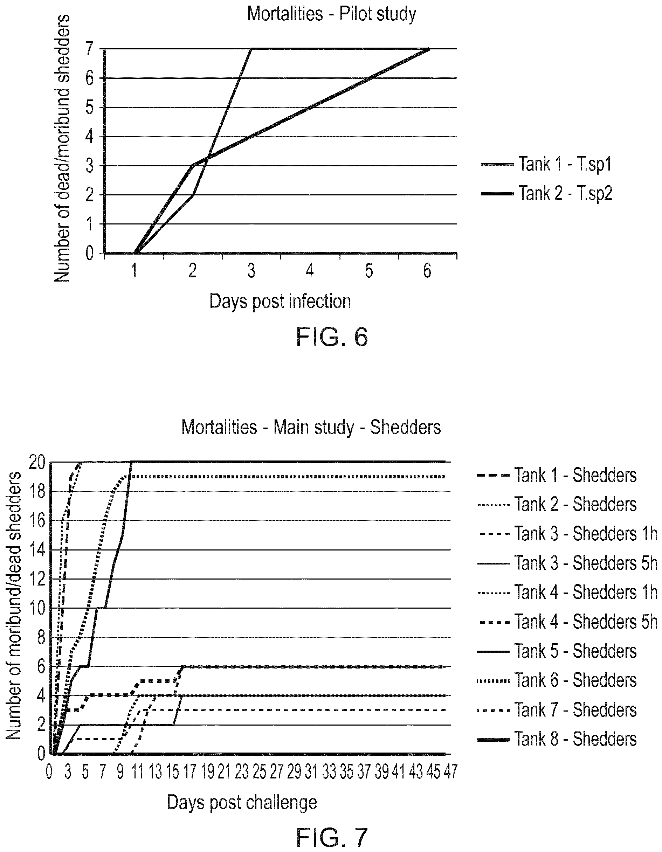

**Figure 6****:** The number of shedders that had to be removed during the pilot study as a result of extensive symptoms or lethargic behavior, plotted against days post infection. N=7 total shedders in both tanks. No cohabitants were included in the pilot study.
**Figure 7****:** show the number of shedders that had to be removed and sampled during the challenge experiment because of lethargic behavior or extensive symptoms. Shedders in tanks 3 and 4 are divided into shedders challenged for one (N=10) and five (N=10) hours. N=20 shedders for all other tanks, except for tank 6 where N=19 because of a counting error during challenge. The number of shedders that had to be removed and sampled during the challenge experiment because of lethargic behavior or extensive symptoms. Shedders in tanks 3 and 4 are divided into shedders challenged for one (N=10) and five (N=10) hours. N=20 shedders for all other tanks, except for tank 6 where N=19 because of a counting error during challenge.

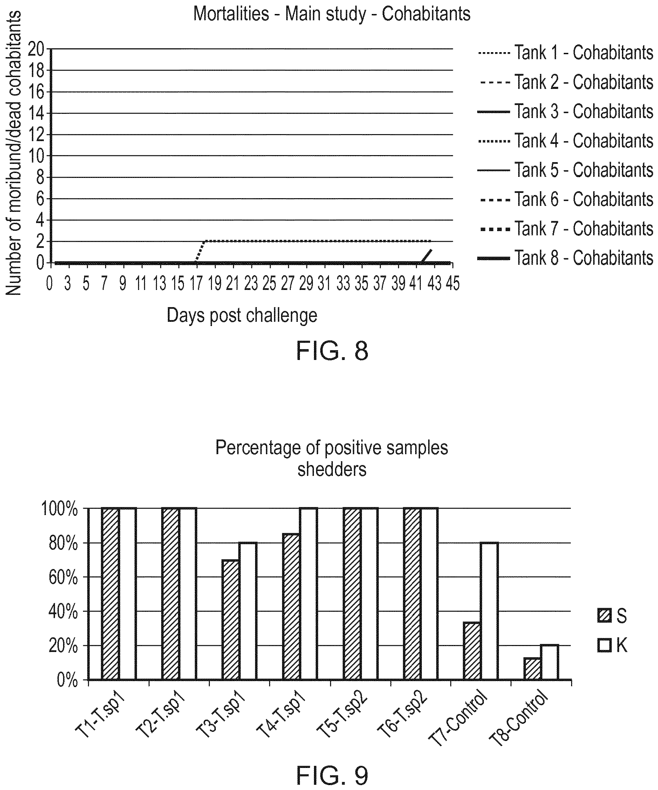

**Figure 8** show the number of symptomatic cohabitants from all tanks in the main challenge experiment. Moribund cohabitants were only observed in tank 4 (two symptomatic cohabitants) and tank 3 (one symptomatic cohabitant). 15 asymptomatic cohabitants were sampled in each tank during termination of the experiment. N=42 cohabitants in each tank at the start of the challenge experiment.
**Figure 9****:** show the percentage of positive skin- and kidney samples from shedders and analyzed for Tenacibaculum sp. N for skin samples = 20 from tank 1,2,3,4,5 and 6. N= 6 for skin samples from tank 7. N= 8 for skin samples from tank 8. N for kidney samples = 5 from each tank. In tank 1, 2, 5 and 6, the skin- and kidney samples were taken from shedders that became moribund during the challenge study. In tank 3, seven moribund fish were sampled continuously, while an additional 13 shedders were sampled at the end of the challenge study. In tank 4, 10 moribund fish were sampled continuously as they became infected, while an additional 10 apparently healthy shedders were sampled during termination of the challenge study. S: Skin sample. K: Kidney sample.

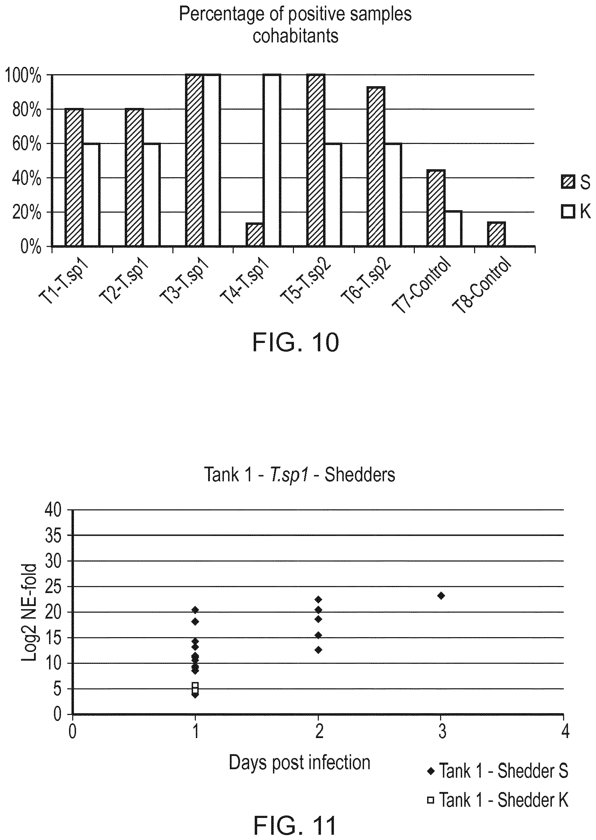

**Figure 10****:** show the percentage of positive skin- and kidney samples collected from the cohabitants and analyzed for Tenacibaculum sp. During the entire study, symptomatic cohabitants were only found in tanks 3 and 4, which had one and two symptomatic cohabitants, respectively. Skin samples, N=15 in tank 1, 2, 3, 4, 5 and 6. N=9 in tank 7 and N=7 in tank 8. Kidney samples, N= 5 from each tank. S: Skin sample. K: Kidney sample.
**Figure 11****:** show log2 NE (Normalized Expression) of the Real Time RT-PCR results of shedders in tank 1 analyzed for Tenacibaculum with Tb_rpoB and EF1A as a reference gene. This tank was challenged for 10 hours with the T.sp1-isolate. All shedders (N=20) were sampled after 3 days as a result of acute mortality. Skin samples (blue rhombus) (N=20) and kidney samples (red square) (N=5) are included in the figure. Skin samples were normalized for the EF1A-value of skin samples, while kidney samples were normalized for the EF1A-value of kidney samples. S: Skin, K: Kidney

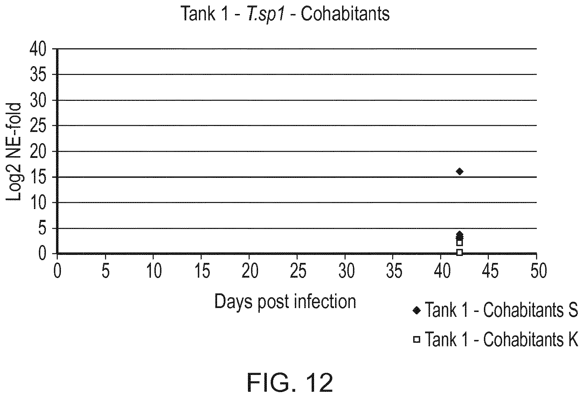

**Figure 12****:** shows Log2 NE (Normalized Expression) of the Real Time RT-PCR results of cohabitants in tank 1 analyzed for Tenacibaculum with Tb_rpoB and EF1A as a reference gene. This tank was challenged for 10 hours with the T.sp1-isolate. 15 cohabitants were sampled at the termination of this tank (42 days post challenge). No cohabitants showed any clinical signs of tenacibaculosis. Skin samples (blue rhombus) (N=15) and kidney samples (red square) (N=5) are included in the figure. Skin samples were normalized for the EF1A-value of skin samples, while kidney samples were normalized for the EF1A-value of kidney samples. S: Skin, K: Kidney.

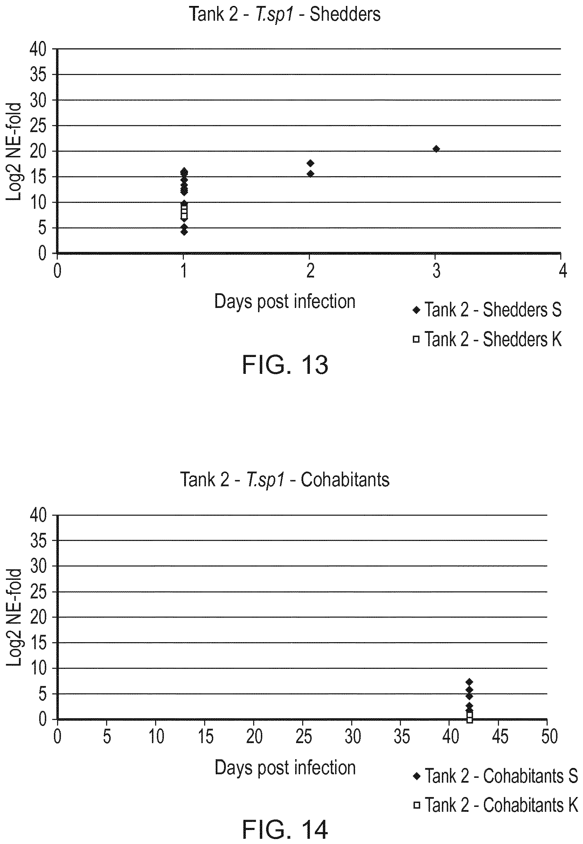

**Figure 13****:** Log2 NE (Normalized Expression) of the Real Time RT-PCR results of shedders in tank 2 analyzed for *Tenacibaculum* with Tb_rpoB and EF1A as a reference gene. This tank was challenged for 10 hours with the *T.sp1*-isolate. All shedders (N=20) were sampled after 3 days as a result of acute mortality. Skin samples (blue rhombus) (N=20) and kidney samples (red square) (N=5) are included in the figure. Skin samples were normalized for the EFlA-value of skin samples, while kidney samples were normalized for the EF1A-value of kidney samples. S: Skin, K: Kidney.
**Figure 14****:** shows Log2 NE (Normalized Expression) of the Real Time RT-PCR results of cohabitants in tank 2 analyzed for *Tenacibaculum* with Tb_rpoB and EF1A as a reference gene. This tank was challenged for 10 hours with the *T.sp1*-isolate. 15 cohabitants were sampled at the termination of this tank (42 days post challenge). No cohabitants showed any clinical signs of tenacibaculosis. Skin samples (blue rhombus) (N=15) and kidney samples (red square) (N=5) are included in the figure. Skin samples were normalized for the EF1A-value of skin samples, while kidney samples were normalized for the EF1A-value of kidney samples. S: Skin, K: Kidney.

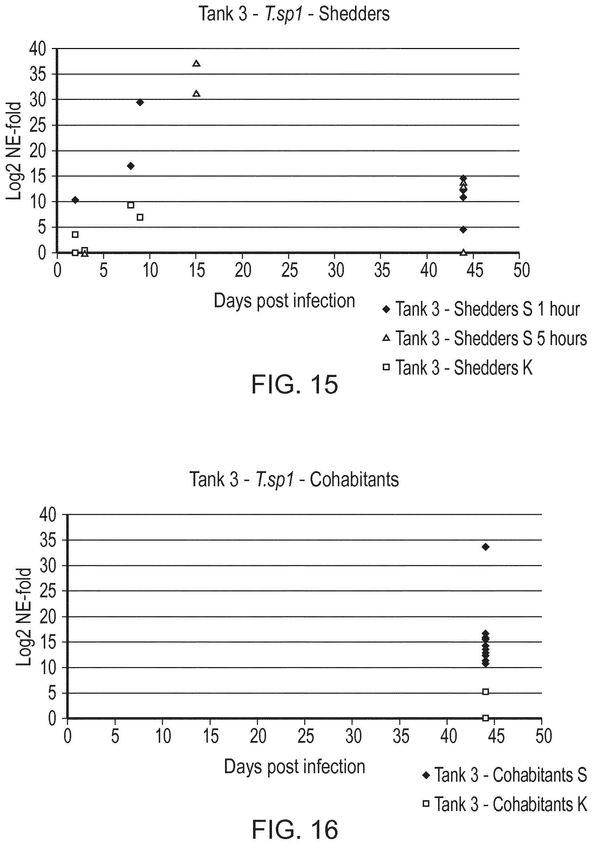

**Figure 15****:** shows Log2 NE (Normalized Expression) of the Real Time RT-PCR results of shedders in tank 3 analyzed for Tenacibaculum with Tb_rpoB and EF1A as a reference gene. This tank was challenged for 1 and 5 hours with the T.sp1-isolate. The skin samples for the shedders are divided into shedders challenged for 1 hour (blue rhombus)(N=10) and 5 hours(green triangle) (N=10). Kidney samples (red square)(N=5) are not divided into 1 and 5 hours. Skin samples were normalized for the EF1A-value of skin samples, while kidney samples were normalized for the EF1A-value of kidney samples. S: Skin, K: Kidney.
**Figure 16****:** shows Log2 NE (Normalized Expression) of the Real Time RT-PCR results of cohabitants in tank 3 analyzed for Tenacibaculum with Tb_rpoB and EF1A as a reference gene. This tank was challenged for 1 and 5 hours with the T.sp1-isolate. 15 cohabitants were sampled at the termination of this tank (44 days post challenge). Only one cohabitant showed clinical signs consistent with tenacibaculosis and it is clearly separated from asymptomatic cohabitants in the graph. Skin samples (blue rhombus) (N=15) and kidney samples (red square) (N=5) are included in the figure. Skin samples were normalized for the EF1A-value of skin samples, while kidney samples were normalized for the EF1A-value of kidney samples. S: Skin, K: Kidney.

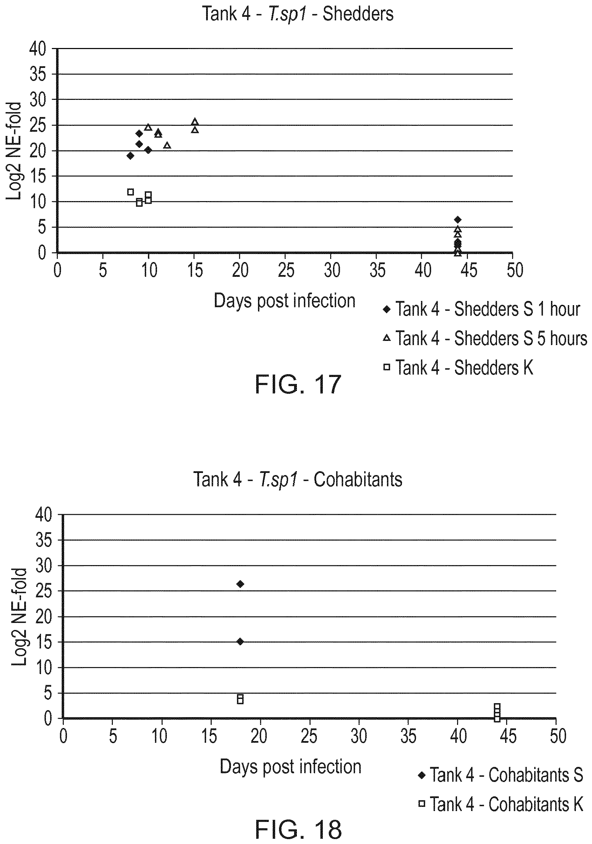

**Figure 17****:** Log2 NE (Normalized Expression) of the Real Time RT-PCR results of shedders in tank 4 analyzed for Tenacibaculum with Tb_rpoB and EF1A as a reference gene. This tank was challenged for 1 and 5 hours with the T.sp1-isolate. The skin samples for the shedders are divided into shedders challenged for 1 hour (blue rhombus)(N=10) and 5 hours(green triangle) (N=10). Kidney samples (red square)(N=5) are not divided into 1 and 5 hours. Skin samples were normalized for the EF1A-value of skin samples, while kidney samples were normalized for the EF1A-value of kidney samples. S: Skin, K: Kidney.
**Figure 18****:** shows Log2 NE (Normalized Expression) of the Real Time RT-PCR results of cohabitants in tank 4 analyzed for Tenacibaculum with Tb_rpoB and EF1A as a reference gene. This tank was challenged for 1 and 5 hours with the T.sp1-isolate. 13 cohabitants were sampled at the termination of this tank (44 days post challenge). Two cohabitants displayed symptoms consistent with tenacibaculosis after 18 days, and are clearly separated in the graph. Skin samples (blue rhombus) (N=15) and kidney samples (red square) (N=5) are included in the figure. Skin samples were normalized for the EF1A-value of skin samples, while kidney samples were normalized for the EF1A-value of kidney samples. S: Skin, K: Kidney.

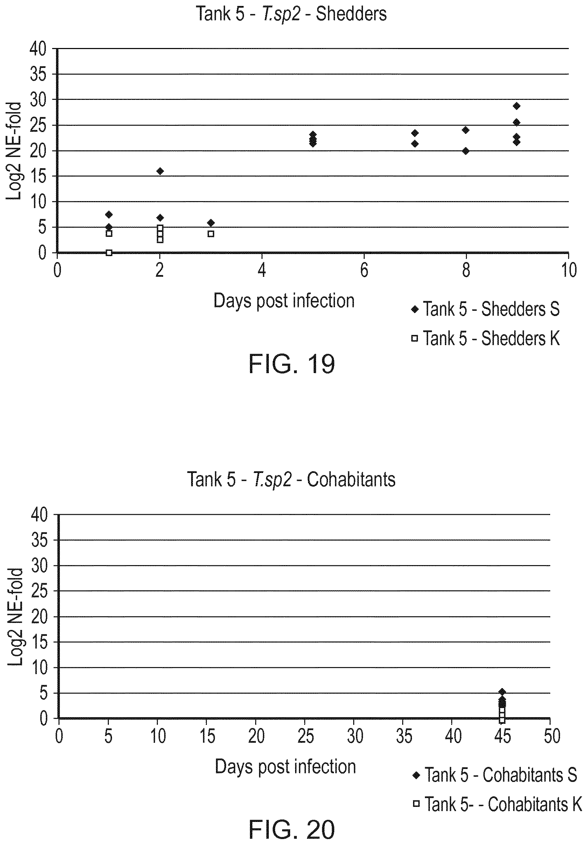

**Figure 19****:** shows Log2 NE (Normalized Expression) of the Real Time RT-PCR results of shedders in tank 5 analyzed for Tenacibaculum with Tb_rpoB and EF1A as a reference gene. This tank was challenged for 10 hours with the T.sp2-isolate. All shedders (N=20) were sampled after nine days, displaying a more chronic mortality. The later the shedders were sampled, the stronger the positive result was. Skin samples (blue rhombus) (N=20) and kidney samples (red square) (N=5) are included in the figure. Skin samples were normalized for the EF1A-value of skin samples, while kidney samples were normalized for the EF1A-value of kidney samples. S: Skin, K: Kidney.
**Figure 20****:** shows Log2 NE (Normalized Expression) of the Real Time RT-PCR results of cohabitants in tank 5 analyzed for Tenacibaculum with Tb_rpoB and EF1A as a reference gene. This tank was challenged for 10 hours with the T.sp2-isolate. 15 cohabitants were sampled at the termination of this tank (45 days post challenge). No cohabitants showed any clinical signs of tenacibaculosis. Skin samples (blue rhombus) (N=15) and kidney samples (red square) (N=5) are included in the figure. Skin samples were normalized for the EFlA-value of skin samples, while kidney samples were normalized for the EF1A-value of kidney samples. S: Skin, K: Kidney.

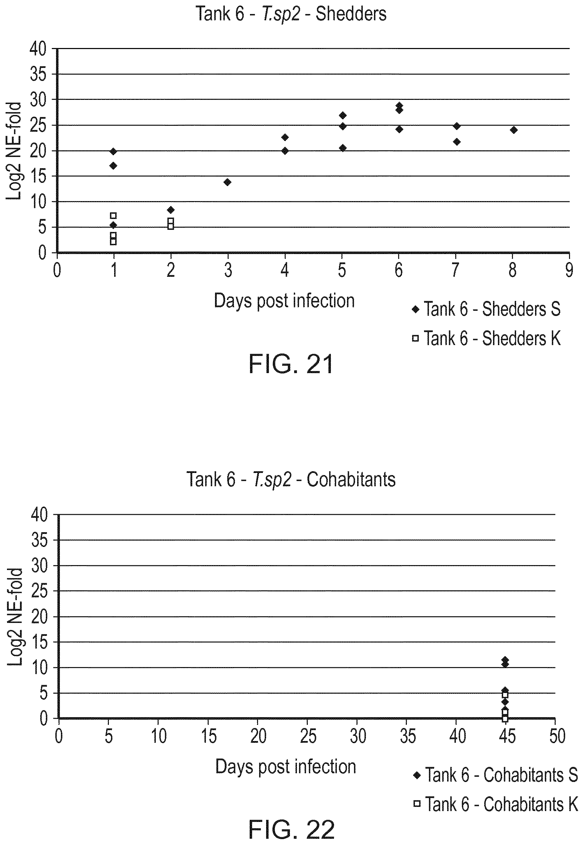

**Figure 21****:** shows Log2 NE (Normalized Expression) of the Real Time RT-PCR results of shedders in tank 6 analyzed for Tenacibaculum with Tb_rpoB and EF1A as a reference gene. This tank was challenged for 10 hours with the T.sp2-isolate. All shedders (N=20) were sampled after eight days, displaying a more chronic mortality. The later the shedders were sampled, the stronger the positive result was. Skin samples (blue rhombus) (N=20) and kidney samples (red square) (N=5) are included in the figure. Skin samples were normalized for the EF1A-value of skin samples, while kidney samples were normalized for the EF1A-value of kidney samples. S: Skin, K: Kidney.
**Figure 22****:** shows Log2 NE (Normalized Expression) of the Real Time RT-PCR results of cohabitants in tank 6 analyzed for Tenacibaculum with Tb_rpoB and EF1A as a reference gene. This tank was challenged for 10 hours with the T.sp2-isolate. 15 cohabitants were sampled at the termination of this tank (45 days post challenge). No cohabitants showed any clinical signs of tenacibaculosis. Skin samples (blue rhombus) (N=15) and kidney samples (red square) (N=5) are included in the figure. Skin samples were normalized for the EF1A-value of skin samples, while kidney samples were normalized for the EF1A-value of kidney samples. S: Skin, K: Kidney.

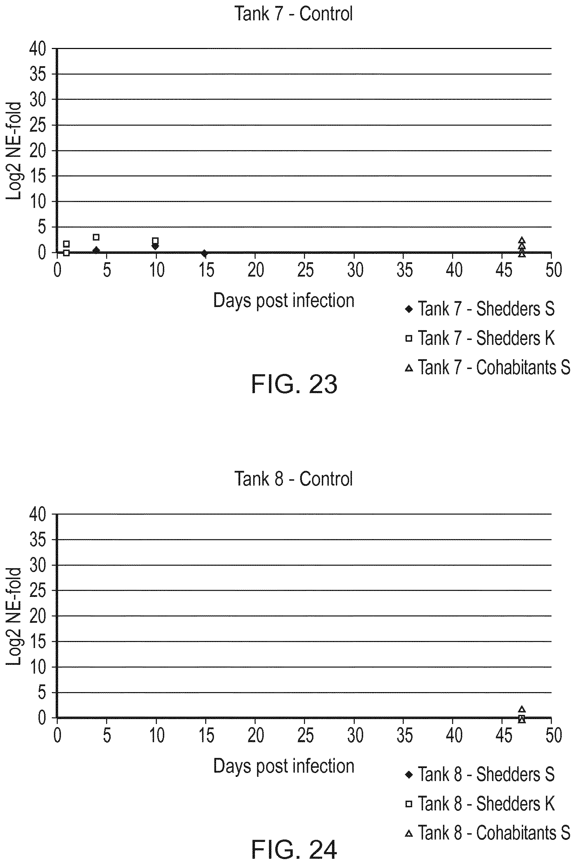

**Figure 23****:** Log2 NE (Normalized Expression) of the Real Time RT-PCR results of shedders and cohabitants in tank 7 analyzed for Tenacibaculum with Tb_rpoB and EF1A as a reference gene. This tank was challenged with marine broth for 10 hours and is part of the control group. Skin samples from shedders (blue rhombus) (N=7) and kidney samples from shedders (red square) (N=5) are included in the graph, in addition to skin samples from cohabitants (green triangle) (N=9). The shedders that became moribund were all sampled within 15 days post challenge. The cohabitants were sampled during the termination of the tank (47 days post challenge). The figure clearly demonstrates that despite having some positive samples in the control tanks, the obtained Log2 NE-value is significantly lower than the challenges tanks. S: Skin, K:Kidney.
**Figure 24****:** Log2 NE (Normalized Expression) of the Real Time RT-PCR results of shedders and cohabitants in tank 8 analyzed for Tenacibaculum with Tb_rpoB and EF1A as a reference gene. This tank was challenged with marine broth for 10 hours and is part of the control group. Skin samples from shedders (blue rhombus) (N=8) and kidney samples from shedders (red square) (N=5) are included, in addition to skin samples from cohabitants (green triangle) (N=7)

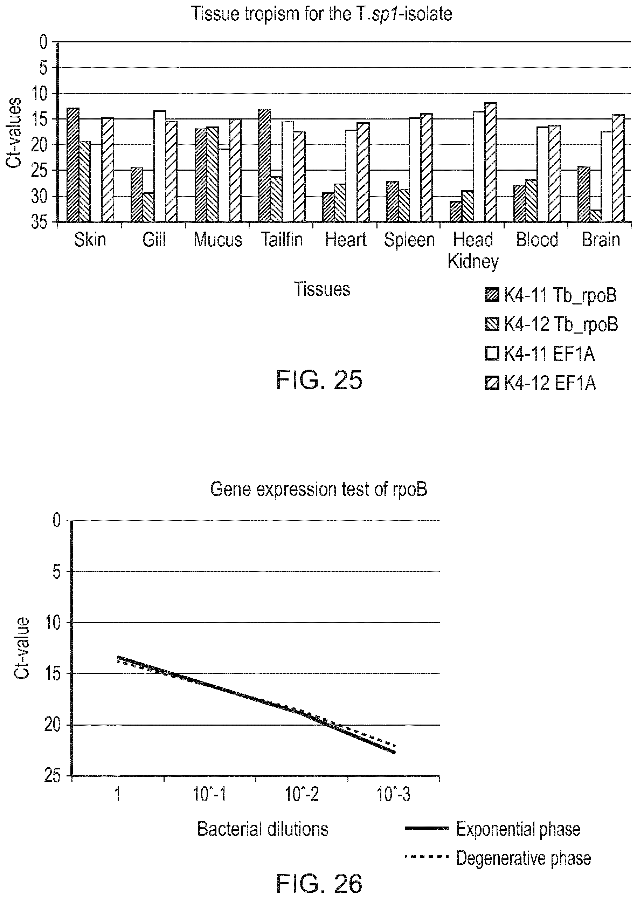

**Figure 25****:** shows the tissue tropism test conducted for the two symptomatic cohabitants in tank 4 challenged with the T.spl-isolate (K4-151113-11 and K4-151113-12). EF1A is included as an endogenous control. The Ct-values are not normalized.
**Figure 26****:** shows the standard curve for the Ct-values obtained from the expression test of rpoB, during exponential and degenerative phases, cf. example 2. Despite having a significantly lower amounts of starting cells in the degenerative phase, the Ct-values are practically identical.

### Detailed description

The present invention provides a novel *Tenacibaculum* isolate involved in the development of tenacibaculosis in salmonids. Isolates of *Tenacibaculum* according to the present invention was isolated in April 2013 from skin lesions in the mouth region of Atlantic salmon (*Salmo Salar L*.) obtained in Finnmark, Norway showing clinical signs consistent with winter ulcer. One of the isolates were provisionally named *T.spl,* and deposited under the Budapest Treaty with the Leibniz Institute DSMZ-German Collection of Microorganisms and Cell Cultures (DSMZ) on 4^{th} March 2014under deposit number DSM 28541.

Characterization of the isolates of the present invention have shown that the isolates represents a novel species in the genus *Tenacibaculum,* for which inventors have suggested to be named *Tenacibaculum finnmarkense sp. nov.,* and where the type strain have been suggested named HFJ^{T} (NCIMB 42389^{T}).

It is to be understood that T.spl(DSM28541) and HFJ^{T} refers to the same isolate, and that said terms may be used interchangeable herein. The isolate according to the present invention have been re-isolated and the identity confirmed by sequencing of the 16S rRNA gene, thus fulfilling Koch's criteria.

The term "tenacibaculosis" is to be understood to mean a disease caused by *Tenacibaculum* infections in fish, such as salmonids, such as in Atlantic salmon (*Salmon salar*). Fish being infected with *Tenacibaculum* infections typically develop ulcers in cold water, with clinical and pathological signs described for winter ulcers seen in fish assumed to be infected with *Moritella viscosa.* The providing of the present novel *Tenacibaculum* isolate and the nucleic acid sequences and oligonucleotide sequences of the present invention allows for the first time a definite diagnosis of a pathogenic agent of tenacibaculosis and thus means for distinguishing between *M. viscosa* and *Tenacibaculum* infections in farmed fish, which enables for the first time that a proper diagnosis can be made and thus, the selection of the most appropriate treatment of the affected fish.

In particular, as will be shown below, the *Tenacibaculum* isolate of the present invention is a novel Gram-negative, aerobic, non-flagellated, rod-shaped gliding bacterial strain. Colonies of the isolates of the present invention is yellow pigmented with entire margins and not adhering to agar. 16S rDNA sequence shows that it belonged to the genus *Tenacibaculum* (family *Flavobacteriaceae,* phylum 'Bacteroidetes'). The strain HFJT exhibited the highest 16S rDNA sequence similarity values to *Tenacibaculum dicentrarchi* NCIMB 14598T (97.2%). It grew at 2-20°C, and only in the presence of sea salts. In the experiments reported herein, the respiratory quinone is menaquinone 6 (100 %), and the major fatty acids is shown to be iso-C15 : 0, anteiso-C15 : 0, Iso-C15 : 1 and iso-C15 : 0 3-OH. The DNA G+C content is shown to be 34.1 mol%. DNA-DNA hybridization and comparative phenotypic and genotypic tests have been performed with the closest phylogenetically related type strains; *T. dicentrarchi* and *T. ovolyticum* NCIMB 14598^{T}. These data, as well as phylogenetic analyses, presented herein support that the strain HFJ^{T} should be classified as a representative of a novel species.

The genus *Tenacibaculum* was described by Suzuki et al. (2001), International Journal of Systematic and Evolutionary Microbiology, 51, 1639-52. To date the genus comprises 21 species derived from a variety of marine environments (http://www.bacterio.net/tenacibaculum.html); including *Tenacibaculum maritimum, Tenacibaculum dicentrarchi, Tenacibaculum discolor* and *Tenacibaculum soleae* from diseased fish (Wakabayashi et al. (1986), International Journal of Systematic Bacteriology, 36, 396-398, Piñeiro-Vidal et al., 2008, Tenacibaculum soleae sp. nov., isolated from diseased sole (Solea senegalensis Kaup). International Journal of Systematic and Evolutionary Microbiology, 58, 881-885, Piñeiro-Vidal et al, 2008, International Journal of Systematic and Evolutionary Microbiology, 62, 425-429 Piñeiro-Vidal et al. (2012), International Journal of Systematic and Evolutionary Microbiology, 58, 21-25.

Phylogenetic and genotypic analysis of genomic DNA of the isolate of the present invention has been performed and compared with Tenacibaculum strains known in the prior art. All the Tenacibaculum field isolates and strains included in the genotypic and phylogenetic characterization are shown in table 1.

**Table 1 Isolate/strain included in phylogenetic and genotypic characterization.**

| **Name** | **Isolate/Strain** | **Type of isolate** | **Origin** | **Host** | **Tissue** | **Year** |
|---|---|---|---|---|---|---|
| Tps1 (HFJ^{T}) | *Tenacibaculum* sp. | Field isolate of the present invention | Norway | Atlantic salmon | Skin | 2013 |
| Tsp.2 | *Tenacibaculum* sp. | Field isolate of the present invention | Norway | Atlantic salmon | Skin | 2013 |
| Tsp.3 | *Tenacibaculum* sp. | Field isolate of the present invention | Norway | Atlantic salmon | Gill | 2014 |
| Tsp.4 | *Tenacibaculum* sp. | Field isolate of the present invention | Norway | Atlantic salmon | Skin | 2013 |
| Tsp.5 | *Tenacibaculum* sp. | Field isolate of the present invention | Norway | Atlantic salmon | Skin | 2014 |
| Tsp.6 | *Tenacibaculum* sp. | Field isolate of the present invention | Norway | Atlantic salmon | Skin | unknown |
| Tsp.7 | *Tenacibaculum* sp. | Field isolate of the present invention | Norway | Farmed Atlantic cod | Skin | 2009 |
| | *Tenacibaculum maritimum* | Type (NCIMB 2154^{T}) | Japan | Red sea bream fingerling | Kidney/skin | 1977 |
| | *Tenacibaculum soleae* | Type (NCIMB 14368^{T}) | Spain | Senegal sole | Unknown | 2007 |
| | *Tenacibaculum ovolyticum* | Type (NCIMB 13127^{T}) | Norway | Atlantic halibut eggs | Eggs | 1989 |
| | *Tenacibaculum dicentrarchi* | Type (NCIMB 14598^{T}) | Spain | European sea bass | Skin | 2009 |

The genotypic, phylogenetic and chemotaxonomic characterization of the isolate of the present invention show that it is significantly different from the prior art *Tenacibaculum* strains, and that it may be may therefore be classified as a novel species in the genus *Tenacibaculum.*

The phylogenetic analysis based on the 16S rDNA sequences and the concatenated HK gene sequences showed that strain HFJ^{T} clustered together with other type strains of genus *Tenacibaculum,* but formed a distinct branch separated from the closest related type strains. This separation was evident in all phylogenetic trees using both bayeshian and maximum likelihood method. All phylogenetic trees showed *T.dicentrarchi^{T}* as the closest related type strain, from which strain HFJT formed a distinct branch with robust support, clearly separated from *T. dicentrarchi^{T}* (see figure 3, 4 and 5).

According to the present invention, a Tenacibaculum isolate is provided that is deposited under the Budapest Treaty with the Leibniz Institute DSMZ-German Collection of Microorganisms and Cell Cultures (DSMZ) on 4^{th} March 2014 under deposit number DSM 28541.

The new *Tenacibaculum* according to the present invention differs from other isolates previously disclosed in the prior art as seen from the experimental data presented herein. It is noted that the new isolate provides improved characteristics compared with earlier known isolates of importance e.g. when being utilized in vaccine production. For example, the new isolate of the present invention has inter alia the following characteristics:
- The present isolate has growth characteristics that differs compared with previous described *Tenacibaculum isolates.* This is e.g. evident from the fact that *T. dicentrarchi* grows on blood agar comprising 1.5 % NaCl, showing β-hemolytic characteristics, contrary to the isolate of the present invention which do not grow on blood agar.
- The present isolate and *T. dicentrarchi* show 97% identity of 1354 bp of the 16s rRNA. In order to be seen as the same species, two isolates should have more than 98.6 % identity of the 16s RNA.
- Analysis of 16s and the housekeeping gene rpoB shows that the present isolate belongs to a distinct group of isolates separated from *T. dicentrarchi,* see the figures 3-5.
- The present isolates cause mortality and clinical sign of disease similar to what is observed in the field and the bacterium was re-isolated from the affected fish, see challenge studies presented herein.

Although the present inventors have isolated and characterized a particular strain disclosed herein which may be used in the preparation of a fish vaccine (DSM28541), the skilled person will acknowledge that other strains being closely related to DSM 28541, i.e. having substantially the same genotypic and phenotypic characteristics, may be used.
The skilled person will acknowledge that various substitutions, deletions or additions to the genomic sequence may be introduced by commonly available gene technology methods or randomly during culturing of a bacterium strain, and that such alteration may be introduced with purpose or randomly/accidentally without substantially changing the characteristics of the bacterial strain. It is to be understood that *Tenacibaculum* strains differing from the strains of the present invention merely by the purposely or randomly introduction of such alteration in the genome that do not substantially affects the genotypic characteristics of the strain compared with the deposited isolate DSM 28541 are covered by the present invention. The expression "related strains having substantially the same genotypic characteristics" is thus to be understood to mean strains where the relevant parts of the genome of the strains to be compared has a high degree of sequence identity compared with a *Tenacibaculum* strain of the same species.

For example, a *Tenacibaculum* isolate comprising a 16sRNA gene sequence as depicted in SEQ ID No. 1 and variants thereof having at least 97% sequence identity with said sequence SEQ ID No. 1 based on the entire sequence length of said sequence. Disclosed herein is also a *Tenacibaculum* isolate comprising a 16sRNA gene sequence as depicted in SEQ ID No. 1 and variants thereof having more than 97.2 % sequence identity with said sequence SEQ ID No. 1 based on the entire sequence length of said sequence.

Disclosed herein is furthermore a *Tenacibaculum* isolate wherein said isolate comprises a 16sRNA gene sequence as depicted in SEQ ID No. 1 or variants thereof having 97.2 % or less sequence identity compared with the 16sRNA gene sequence of *T. dicentrarchi^{T}* as depicted in SEQ ID No. 52, and 96.4% or less sequence identity with the 16sRNA gene sequence of *T.soleae^{T}* as depicted in SEQ ID No. 53 and 96.0% or less sequence identity with the 16sRNA gene sequence of *T. ovolyticum^{T}* as depicted in SEQ ID No. 54.

Disclosed herein is furthermore a *Tenacibaculum* isolate, wherein said isolate comprises a 16sRNA gene sequence as depicted in SEQ ID No. 1 or variants thereof having at least 98.6 % identity of SEQ ID No. 1.

Disclosed herein is furthermore a *Tenacibaculum* isolate, wherein said isolate comprises a 16sRNA gene sequence as depicted in SEQ ID No. 1 or variants thereof having at least 98.65 % identity of SEQ ID No. 1.

Disclosed herein is furthermore a *Tenacibaculum* isolate, wherein said isolate comprises a 16sRNA gene sequence as depicted in SEQ ID No. 1 or variants thereof having at least 99 % identity of SEQ ID No. 1.

The skilled person will acknowledge that the relationship of two or more bacterial isolates may be determined by various techniques comparing the alignment of more than one gene sequence, i.e. by analyzing the % average nucleotide identity (ANI) of concatenated sequences of a selection of genes, in particular house-keeping genes. The skilled person will furthermore acknowledge that a *Tenacibaculum* strain, which upon analysis of the concatenated sequence of the house keeping genes atpD, pgk, TUF and fusA show 95% or more average nucleotide identity with the concatenated sequence of said house - keeping genes as depicted in SEQ ID No. 2, SEQ ID No. 4, SEQ ID No. 5, and SEQ ID No. 6 belong to the same species as the deposited strain DSM 28541, and that such related isolates are within the scope of the present invention.

In particular, a *Tenacibaculum* isolate is disclosed comprising the housekeeping (HK) genes atpD, pgk, TUF and fusA, which when aligned concatenated, such as e.g. in SEQ ID No. 55, with the housekeeping genes of *T.dicentrarchi* have an ANI value of 94.4%.

Disclosed herein is also a *Tenacibaculum* isolate comprising the housekeeping genes as depicted in SEQ ID No. 2 (the atpD gene sequence), SEQ ID No. 4 (the pgk gene sequence), SEQ ID No. 5 (the TUF gene sequence), and SEQ ID No. 6 (the fusA gene sequence); as well as variants thereof comprising variants of the said housekeeping genes have an average nucleotide identity of at least 95% when aligned concatenated with the housekeeping gene of SEQ ID No. 2, SEQ ID No. 4, SEQ ID No. 5 and SEQ ID No. 6.

The expression "related strains having substantially the same phenotypic characteristics" as used herein is to be understood to refer to one or more observable properties of a *Tenacibaculum* isolate that are the result of the interaction of the inherent genotype of the bacteria and/or the non-hereditary environmental variations. The term "substantially the same phenotypic characteristics" is to be understood to mean the characteristics of having substantially the same phenotypic characteristics as DSM28541, such as having similar growth characteristics (e.g. in respect of culturing condition (temperature, growth medium, etc.), the ability to reproduce mortality and tenacibaculosis in cohabitant fish in a challenging model as described herein, and as outlined in table 3. Reference is made to example 2 presenting the phenotypic characteristics of the *Tenacibaculum* isolate according to the present invention. It is to be understood that the *Tenacibaculum* isolates covered by the present invention have the same phenotypic characteristics as the novel *Tenacibaculum* species with the suggested name *Tenacibaculum finnmarkense sp. nov* described for the first time herein. *Tenacibaculum* strains may exert more or less equivalent genotypic and phenotypic characteristics as the isolate DSM 28541. It is to be understood that *Tenacibaculum* isolate exerting more or less equivalent genotypic and phenotypic characteristics as the isolate DSM 28541 is a *Tenacibaculum* isolate belonging to the same *Tenacibaculum* species as DSM 28541. The present invention thus disclose *Tenacibaculum* isolates having substantially the same ability to induce tenacibaculosis in farmed fish when used in a challenging model as disclosed herein as DSM 28541.

Furthermore, in respect of phenotypic characteristics, it is noted that the cells of the present novel species are strictly aerobic, Gram-stain-negative, straight rods, 0.5 µm in diameter and 5-25 µm in length (filamentous cells >100 µm long may occur in older cultures) and motile by gliding. Degenerative spherical cells are observed in ageing cultures. Colonies on MA are circular, convex, yellow pigmented with translucent edges, have entire or undulating margins and a smooth texture with a shiny and sometimes nacreous appearance. The colonies are slightly viscous and do not stick to agar. Congo red absorption is negative. Growth occurs in media containing 50-100% strength seawater, but not in media supplemented with NaCl only. No growth occurs on BAS. Growth occurs at 2, 4, 8, 16 and 20°C, but not at 25, 30 and 37°C. Growth occurs at pH 4.0-9.0 (optimum pH 6-8). Catalase and cytochrome oxidase activities are present. Gelatin and casein are hydrolyzed, but Tween 80 and starch are not. The Voges-Proskauer and flexirubin test are negative. No anaerobic growth was observed. H₂S and indole are not produced. L-Proline, and L-glutamate are utilized, but D(+)-sucrose, D(2)-ribose, D(+)-galactose, D(+)-glucose and L-tyrosine are not. In the API ZYM system, alkaline phosphatase, esterase (C4), esterase lipase (C8), leucine arylamidase, valine arylamidase, cystein arylamidase, acid phosphatase and naphthol-AS-BI-phosphohydrolase are present, but lipase (C14), trypsin, a-chymotrypsin and all enzymes related to the metabolism of carbohydrates are absent. The present *Tenacibaculum* isolate is susceptible to Trimethoprim-sulfamethoxazole, gentamicin, ceftazidime, ciprofloxacin, pipemidic acid, cefuroxime, penicillin G, ampicillin, tetracycline, erythromycin, florfenicol, oxytetracycline, oxolinic acid, but resistant to kanamycin and streptomycin. The major fatty acids (>5% of the total fatty acids) are iso-C_{15 : 0} , anteiso-C_{15 : 0}, ISO-C_{15 : 1} and iso-C_{15 : 0} 3-OH. The respiratory quinone is menaquinone 6 (100 %).The type strain is HFJ^{T} (=DSM 28541^{T} = NCIMB 42386^{T}), isolated from diseased Atlantic salmon (*Salmo salar* L.) in Norway. The DNA G+C content of the type strain is 34.1 mol %. Reference is made to figure 1 and figure 2 showing Toulidine blue stained cells of the deposited strain DSM 28541 after 48 hr and 6 days incubation, respectively.

Based on the detailed description of the genotypic and phenotypic characteristics of the deposited strain DSM 28541 herein and common general knowledge, the skilled person will be able to determine whether a *Tenacibaculum* isolate belong to the same species as said deposited strain. Methods useful in determining the specie of a bacterial isolate includes inter alia the determination of the %Average Nucleotide identity (ANI) of concatenated housekeeping gene sequences of the isolate of question upon alignment with the concatenated housekeeping gene sequences of a reference strain, or determination of %identity of the 16SRNA gene of the isolate of question with the 16SRNA gene of a reference strain.

The *Tenacibaculum* isolate of the present invention may be used in pharmaceutical compositions, such as vaccines, which upon administration to fish, result in that said fish gain protection against *Tenacibaculum* infections and/or the development of tenacibaculosis and ulcers associated with this disease. Thus, the invention relates to the use of a *Tenacibaculum* isolate according to the present invention in the preparation of a vaccine for combatting *Tenacibaculum* infection in fish. Furthermore, pharmaceutical composition according to the present invention may be used to prevent the development of tenacibaculosis. The pharmaceutical composition may furthermore be useful in preventing winter ulcers in farmed fish.

The isolate according to the present invention may be cultured in marine brought or marine agar, such as Difco™ Marine Brotht or Difco™ Marine Agar 2216 well known to the skilled person and commercially available e.g. from BD (http://www.bd.com/ds/productCenter/212185.asp). The isolate according to the present invention may e.g. be cultured on Difco™ Marine Agar 2216 with an incubation temperature of 16 °C. The isolate according to the present invention may also be cultured in Difco™ Marine Broth 2216, incubated at 16 °C, and stirring (140 rpm).

For the manufacture of a pharmaceutical composition according to the invention, both live, attenuated, inactivated/killed *Tenacibaculum* isolates of the invention may be used. Also a fragmented *Tenacibaculum* isolate (e.g. when being pressed through a French press), provided that the fragmented isolate enables the development of cross specific immunity in the fish being administered a vaccine comprising the fragmented material.

The term "live attenuated bacterium" or "live attenuated *Tenacibaculum* isolate" as used herein is to be understood to mean a bacterium that is less pathogenic than its wild-type counterpart, and which nevertheless is able to induce a cross specific immunity, and thus an efficacious immune response in a fish administered with a vaccine comprising said live attenuated bacterium. The skilled person is well known with the various methods used to obtain attenuated strains. E.g. an attenuated strain can be obtained exposing a culture of the bacterium for mutagenizing conditions, e.g. by chemical mutagenesis or UV-radiation. Attenuated strain may also be obtained using site-directed mutagenesis.

The term "inactivated bacterium" or "inactivated *Tenacibaculum* isolate" or "killed bacterium" or "killed *Tenacibaculum* isolate" as used herein is to be understood to mean bacterial that after culturing and prior to the formulation of a pharmaceutical composition is treated in order to obtain a bacterial material that cannot replicate, but which still is able to induce a cross specific immunity, and thus provide an efficacious immune response in a fish administered with a vaccine comprising said inactivated/killed *Tenacibaculum* isolate. The skilled person is well aware of the various methods available in order to inactivate bacteria useful in e.g. vaccine. For example, the *Tenacibaculum* isolate of the present invention may be inactivated by heat-treatment or chemical treatment, e.g. by exposing a culture of the *Tenacibaculum* isolate according to the present invention to formalin, binary ethylene imine, or thimerosal.

The providing of the present *Tenacibaculum* isolate furthermore enables the development of subunit vaccines, e.g. where the antigenic material included in the vaccine comprises parts of the isolate or e.g. a recombinant protein encoding an epitope applicable in a vaccine in order to provide treatment or prevention of tenacibaculosis. Also, DNA vaccines may be developed based on the providing of the novel *Tenacibaculum* isolate of the present invention.

A vaccine according to the present invention may also comprise adjuvants well known to the skilled person, e.g. commonly used in order to further enhance the immune response in a non-specific manner. Adjuvants that may be used in accordance with the present invention is e.g. described by Jan Raa in Reviews in Fisheries Science 4(3): 229-288 (1996), which is incorporated by reference in its entirety.

A vaccine according to the present invention may also comprise other vehicles, carriers or excipient well known to the skilled person, and be prepared according to methods well known in the art. Reference is e.g. made to Anderson, D.P. (1997), Developments in Biological Standardization 90, p. 257-265, Vinitnantharat, S. et al (1999), Advances in veterinary medicine 41, p. 539-550 (1999), Buchmann, K. et al. (2001), J. Acta Parasitologica 46, p. 71-81 and Sommerset, L. et al. (2005), Expert Review of Vaccines, 4, p. 89-101, said publications being incorporated by reference in its entirety.

For example, the present invention may comprise excipients added as stabilizers, such as e.g. dextran, mannitol, sorbitol, starch, or sucrose. Emulgators may also be comprised in the vaccine composition according to the present invention, such as e.g. polysorbate 85, polysorbate 80, PEG-6 sorbitan oleate, and sorbitan oleate, etc.

In some instances, it may be desirable to combine the vaccine of the present invention with another antigenic material in a combination vaccine, or in a kit comprising both components for separate, sequential or simultaneous administration, for treatment or prevention of tenacibaculosis as well as a multitude of diseases to which the fish are susceptible. The other diseases are to be understood to be caused by other pathogenic organisms of marine origin, such as various bacterial fish diseases, viral fish diseases or parasitic fish diseases. For example, the vaccine according to the present invention when formulated as a combination vaccine includes at least one other antigen material originating from a virus selected from the group consisting of infectious salmon anemia virus (ISAV), infectious pancreatic necrosis virus (IPNV), piscine reovirus (PRV), piscine myocarditis virus (PMCV), salmonid alpha virus (SAV), viral hemorrhagic septicemia virus (VHSV); and/or from a microorganism selected from the group consisting of *Bifidobacterius sp., Brevibacterium sp., Cytophaga sp., Flavobacterium sp., Edwardsiella sp., Francisella sp., Listonella sp., Moritella viscose, Mycobacterium sp.,Nocardia sp., Photobacterium damsel, Pedicoccus sp., Piscirickettsia sp. Aeromonas sp., Pseudomonas sp., Renibacterium sp., Streptococcus sp., Lactococcus sp., Leuconostoc sp., Vibrio sp., Yersinia sp.* and/or a parasite selected from the group consisting of *Caligus sp., Ichthyopthirius Sp. or Lepeophtheirus sp..*

The vaccine according to the present invention may also comprise antigenic material originating from a fish parasite, such as e.g. *Paramoeba spp, Cryptobia salmonistica, Nucleospora salmonis,* parasite belonging to the group Myxosporeans, *Eubothrium spp,* or belonging to the group crustaceans, such as e.g. *Lepoptheirus salmonis* or *Caligus spp..*

In respect of the additional antigenic material to be included in a combination vaccine, it may be in the form of e.g. attenuated, killed or inactivated bacteria of virus, or in the form of subunit or part thereof. An example of subunit to be used as antigen material in a vaccine is e.g. recombinant proteins provided by e.g. heterologous expression of a viral or bacterial DNA sequence encoding a useful epitope in an appropriate host cell.

The vaccine according to the present invention may be administered by any route well known to the skilled person, such as e.g. by bath, immersion, spray, intraperitoneal injection, intramuscular injection, or oral administration. The vaccine may be administered to young fish, for example salmon in the fresh water stage, e.g. prior to stocking in cages.

A pilot and a main challenge experiment has been conducted with the purpose of proving Kock's postulates, prove cohabitant transmission and to find the optimum dose for cohabitant challenge and vaccine testing. The fish has been bath challenged with various doses and exposure times, but only with Tenacibaculum and without any disruption of the skin. The disease has been reproduced and the bacterium has been reisolated and sequenced from the diseased fish, thus fulfilling Kock's postulates. Mortality, macroscopic and histological clinical signs are coherent to field observations of tenacibaculosis. Tissue smears and real time RT-PCR results show high presence of the bacterium in the skin, but also in the internal organs. Two cohabitants in the large-scale trial showed symptoms of tenacibaculosis and the bacteria was reisolated and the fish was positive for Tenacibaculum by real time RT-PCR

### Determination of the presence of the novel Tenacibaculum species.

Not only does the identification of a novel *Tenacibaculum* species as described herein allow for the development of pharmaceutical preparation and vaccines as described above, but also allow for the development of diagnostic tools useful in monitoring the development and determining presence of infectious and pathogenic *Tenacibaculum* strains in farmed fish. The present invention thus provides a method of diagnosis of tenacibaculosis. For example, the *Tenacibaculum* isolate according to the present invention may be used to provide an antibody capable of binding selectively to a Tenacibaculum strain belonging to the same species as the isolate of the present invention. Such monoclonal or polyclonal antibodies may be used in well-known immunoassay commonly used as diagnostic tools, such as e.g. enzyme-linked immunosorbent assay (ELISA), immunofluorescence tests (IFT) and Western blot analysis.

The Tenacibaculum isolate of the present invention, and in particular the gene sequences or fragments thereof disclosed herein (SEQ ID No. 1, SEQ ID No. 3, SEQ ID No. 4, SEQ ID No. 5, and SEQ ID No. 6), may be used to develop diagnostic tools useful in methods utilizing hybridization/PCR technology and the like. For example, the DNA sequences or fragments thereof, or probes or primers derived therefrom, may be used to detect the presence of Tenacibaculum strain(s) belonging to the same species as the present Tenacibaculum isolate of the invention in infected fish. The skilled person will acknowledge that a sample taken e.g. from an ulcer in a farmed fish may be used to derive nucleic acid sequences that, in case they derive from a Tenacibaculum strain belonging to the same species as the isolate of the present invention, will hybridize (under stringent conditions) with DNA sequences according to the present invention.
A nucleotide sequence is disclosed, wherein said sequence is selected from the group consisting of SEQ ID No. 1 encoding a 16sRNA gene and variants or fragments thereof having at least 97 % sequence identity with said sequence SEQ ID No. 1 based on the entire sequence length of said sequence.

The term "% identity" is to be understood to refer to the percentage of nucleotides that two or more sequences or fragments thereof contains, that are the same. A specified percentage of nucleotides can be referred to as e.g. 70% identity, 80% identity, 85% identity, 90% identity, 95% identity, 99% identity or more (or any number in between) over a specified region when compared and aligned for maximum correspondence. The skilled person will acknowledge that various means for comparing sequences are available. For example, one non-limiting example of a useful computer homology or identity program useful for determining the percent homology between sequences includes the Basic Local Alignment Search Tool (BLAST) (Altschul et al., 1990, J. of Molec. Biol., 215:403-410, "The BLAST Algorithm; Altschul et al,, 1997, Nuc. Acids Res. 25:3389-3402, Karlin and Altschul 1990, Proc. Nat. Acad. Sci. USA, 87:2264-68; 1993, Proc. Natl Acad. Sci. USA 90:5873-77).

The present invention furthermore provides nucleic acid sequences comprising the housekeeping genes as depicted in SEQ ID No. 2 (the atpD gene sequence), , SEQ ID No. 4 (the pgk gene sequence), SEQ ID No. 5 (the TUF gene sequence), and SEQ ID No. 6 (the fusA gene sequence); or variants thereof having an average nucleotide identity of less than 95% when aligning concatenated the housekeeping genes atpD, pgk, TUF and fusA of said variant with the housekeeping gene selected from the group consisting of:
(i) SEQ ID No. 37, SEQ ID No. 40, SEQ ID No. 43, SEQ ID No. 46, and SEQ ID No. 49 from *T.dicentrachi;*
(ii) SEQ ID No. SEQ ID No. 38, SEQ ID No. 41, SEQ ID No. 44, SEQ ID No. 47 and SEQ ID No. 50 from *T. ovolyticum*; and
(iii) SEQ ID No. 39, SEQ ID No. 42, SEQ ID No. 45, SEQ ID No. 48 and SEQ ID No. 51 from *T. soleae.*

The term "% average nucleotide identity" refers to the average percentage of identical nucleotides found when two or more concatenated sequences consisting of five or more housekeeping genes linked together from different isolates are aligned. The term "concatenated sequences" is to be understood to refer to two or more gene sequences wherein said two or more gene sequences or fragments thereof is linked together consecutively. The concatenated sequences may be aligned for comparison with the corresponding concatenated two or more gene sequences or fragments thereof from another strain or isolate. The alignment of concatenated sequences is typically performed in order to determine whether an isolate belong to a known species. For example, the house keeping genes atpD, pgk, TUF and fusA originating from the present isolate may be concatenated, aligned and compared with a sequence comprising concatenated sequences of the same house keeping genes from another *Tenacibaculum* species, such as e.g. *T. dicentrarchi, T.soleae^{T}, or T. ovolyticum^{T}.*

The oligonucleotides disclosed herein may be used as oligonucleotide probes and oligonucleotide primers in method for detection of the presence of *Tenacibaculum* belonging to the same species as the isolate of the present invention, such as DSM 2854, in a sample taken from a fish to be tested. The detection of nucleic acids present in a biological sample is widely applied in both human and veterinary diagnosis, wherein nucleic acids from e.g. pathogens present in biological samples are isolated and hybridized to one or more hybridizing probes or primers used in order to amplify a target sequence.

The skilled person will acknowledge that an oligonucleotide probe or primer according to the present invention may be a fragment of DNA or RNA of variable length used herein in order to detect the presence of a *Tenacibaculum* strain belonging to the same species as the isolate of the present invention, upon hybridization of the oligonucleotide probe to complementary sequence(s) of the said target sequence to be analyzed. The oligonucleotide probe according to the present invention may furthermore be labeled with a molecular marker in order to easily visualize that hybridization, and thus detection of the SNPs disclosed herein, have been achieved.

Molecular markers commonly known to the skilled person may be used, e.g. a radiolabel, and more preferably, a luminescent molecule or a fluorescent molecule enabling the visualization of the binding of the probe(s) to a target sequence.

An oligonucleotide probe as the ones disclosed herein is able to hybridize to another nucleic acid molecule, such as the single strand of DNA or RNA originating from e.g. an ulcer of a fish to be analyzed, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognize as the "stringency" of the hybridization. The suitable stringency for hybridization of a probe to target nucleic acids depends on inter alia the length of the probe and the degree of complementation, variables well known to the skilled person. An oligonucleotide probe or primer according to the present invention typically comprises the appropriate number of nucleotides allowing that said probe or primer align with the target sequence to be analyzed A oligonucleotide probe or primer according to the present invention typically comprises a nucleotide sequence which under stringent conditions hybridize to at least 8, 10, 12, 1 6, 20, 22, 25, 30, 40, 50 (or any other number in-between) or more consecutive nucleotides in a target nucleic acid molecule, e.g. single-stranded DNA or RNA isolated from an ulcer of a fish to be analyzed according to the present invention. For example, oligonucleotide probes or primers are provided comprising about 18 - 25 consecutive nucleotides, more preferably about 20 nucleotides.
The skilled person will be well aware of the various methods available for preparing the oligonucleotide probes or primers of the present invention. Furthermore, the skilled person will be well aware of the various assays available for using the oligonucleotide primers and probes for detection of the presence of a *Tenacibaculum* strain belonging to the same species as DSM 28541in farmed fish, such as polymerase chain reaction (PCR), including RT-PCR.

According to one embodiment, an oligonucleotide probe or oligonucleotide primer is provided comprising a oligonucleotide sequence that hybridize to a DNA sequence selected from the group consisting of SEQ ID No. 1, SEQ ID No. 2, SEQ ID No. 4, SEQ ID No. 5 and SEQ ID No. 6.

Also an oligonucleotide sequence is disclosed herein that under stringent conditions hybridize to the sequence depicted in SEQ ID No. 1 or variants thereof having 97.2 % or less sequence identity compared with the 16sRNA gene sequence of *T. dicentrarchi^{T} (SEQ ID No.* 52), and 96.4% or less sequence identity with the 16sRNA gene sequence of *T.soleae^{T} (SEQ ID No.* 54)and 96.0% or less sequence identity with the 16sRNA gene sequence *of T. ovolyticum^{T}(SEQ ID No.* 53).

Also, an oligonucleotide sequence is disclosed herein that under stringent conditions hybridize to the sequence depicted in SEQ ID No. 1 or variants thereof having at least 98.6 % identity of SEQ ID No. 1.

Furthermore, an oligonucleotide sequence selected from the group consisting of SEQ ID No. 7 - SEQ ID No. 28 is disclosed herein.

Also variants of the sequences SEQ ID No. 7 - SEQ ID 28 is disclosed having at least 75% sequence identity with each sequences SEQ ID No. 7 - SEQ ID 28, respectively, such as at least 80 % identity, such as at least 85% identity, such as at least 90% identity, such as at least 95% identity, such as at least 96%, 97%, 98% or 99% identity with said sequences SEQ ID No. 7 - SEQ ID 28, respectively.

The disclosed oligonucleotide sequences may be used in any method commonly known to the skilled person where probes or primers are used for diagnostic purposes and determination of tenacibaculosis in fish.

The present invention is further supported by the following non-limiting examples, which are provided by way of illustration and not intended to be limiting to the present invention.

### Examples

### Example 1: Isolation of the novel Tenacibaculum strains

Two isolates of *Tenacibaculum sp.* were obtained from farmed Atlantic salmon at a marine site and a smolt production site in northern Norway. The isolates were derived from fish showing clinical signs consistent with *Tenacibaculum sp.* infections/winter ulcer. The isolates were provisionally named *T.sp1* and *T.sp2.*

*T.sp1* was isolated in April 2013 from the skin of salmon kept at low temperatures (4-5 °C) at a seawater site in Finnmark County in northern Norway. The salmon, with an average weight of approximately 2.5 kg, developed large ulcers 14 months after sea transfer. The ulcers appeared after periods of handling and coincided with increased mortality at the site. *T.sp2* was obtained in February 2013 from salmon smolt kept at low temperatures in tanks with full strength seawater in Nordland County. The smolts had developed skin ulcers and the bacterium was isolated on marine agar from these ulcers. The fish had not been subjected to any specific handling before the outbreak and the seawater intake at the production sites was treated with UV-light before it was introduced to the fish tanks.
The two isolates, both grown on marine agar, were incubated at 16 °C. Both isolates grouped within the genus *Tenacibaculum* based on analysis of the 16S rRNA gene sequences.

T.sp1 was deposited at the Budapest Treaty with the Leibniz Institute DSMZ-German Collection of Microorganisms and Cell Cultures (DSMZ) on the 4^{th} March 2014 and provided the deposit number DSM 28541.

The further characterization of *T.spl,* also named HFJ^{T} herein, showed that it belongs to a novel Tenacibaculum species, for which the name *Tenacibaculum finnmarkense* sp nov is proposed.

### Example 2: Characterization of the novel Tenacibaculum isolates

Genomic DNA for phylogenetic and genotypic analysis was extracted using the E.Z.N.A. tissue DNA kit™ (Omega Bio-Tek) following the cultured cells protocol. PCR was performed using the 16S rDNA primers 27F and 1518R (Giovannoni et al., 1996) and specific primers for five housekeeping genes (rpoB, tuf, atpD, fusA and Pgk) shown in table 2 below. Amplification was based on a standard reaction mixture containing 2.5 µl Extra buffer, 1.25 mM DNTP (Deoxyribonucleotide Triphosphate), 0.75 units (0.15 µL) *Taq* DNA polymerase (BioLabs, New England), 5 µM (1 µL) of forward and reverse primer and DNase-RNase free water was added to a final solution of 25 µL (16.85 µL H₂O). Amplification was performed in a GeneAmp PCR system 2700 (Applied Biosystems) at 95°C for 5 minutes; 35 cycles of 95°C for 30 seconds, 58°C for 30 seconds, 72°C for 60-90 seconds, followed by 72°C for 10 minutes. The PCR product was confirmed by gel electrophoresis and enzymatically purified using ExoStar 1 -Step ® (GE Healthcare Bio-Sciences Corp) in an Artik Thermal Cycler (Thermo Scientific) at 37°C for 15 minutes and 80°C for 15 minutes. The sequence reaction was performed using a BigDye® version 3.1 reaction in an Arktik Thermal Cycler, at 96°C for 5 minutes; 30 cycles of 96 °C for 10 seconds, 58°C for 5 seconds and 60°C for 4 minutes. The reaction was composed of a mixture of 5.5 µL deionized water, 1 µL BigDye® Terminator 3.1 version sequencing buffer, 1 µL BigDye Terminator 3.1 version Ready Reaction Premix (2.5X) (Invitrogen), 3.2 pmol (1µL) forward and reverse primer and 1.5 µL purified PCR product. Sequencing was carried out by the Sequencing Facility at Hayteknologisenteret i Bergen, http://www.uib.no/seqlab). Samples were cleaned with Agencourt CleannSeq (Beckman Coulter, Inc.) before being sequenced in a 96-capillary 3730x1 DNA Analyzer (Applied Biosystems). The software Vector NTI® v.9.0 (Invitrogen) was used to assemble and align received sequences.

**Table 2: primers for the five housekeeping genes rpoB, tuf, atpD, fusA and Pgk**

| **Target gene** | **Name** | **Sequence (5' - 3')** | **SEQ ID No./source** |
|---|---|---|---|
| 16S rRNA | B27F | AGAGTTTGATCMTGGCTCAG | SEQ ID No 34/Giovannoni et al 1996* |
| 16S rRNA | A1518R | AAGGAGGTGAT CCANCCRCA | SEQ ID No. 35/Giovannoni et al. 1996* |
| 16S rRNA | Tsp F1 | GAATCTGCCTTGTACAGGAG | SEQ ID No 7/T.sp1 |
| 16S rRNA | Tsp R1 | AGGTCGCTCCTYRCGGTAACG | SEQ ID No 8/T spl |
| tuf | Tb_tuf_F1 | ACCTCCTTCACGGATAGC | SEQ ID No 9/T spl |
| tuf | Tb_tuf R1 | TTACGATCGTTCGAAGCCCC | SEQ ID No. 10/T.sp1 |
| rpoB | Tb_tuf F1 | ATYTCTCCAAAACGCTGACC | SEQ ID No 11/T.sp1 |
| rpoB | Tb_rpoB R1 | AAAACGAATCAAGGWACGAAYA | SEQ ID No. 12/T.sp1 |
| rpoB | Tb_rpoB F2 | ACCCTTTCCAAGGCATAAAGG | SEQ ID No 13/T.sp1 |
| rpoB | Tb_rpoB R2 | GAGCCATYGGTTTTGAAAGAGA | SEQ ID No 14/T.sp1 |
| rpoB | Tb_rpoB F3 | CTCTTGCTGTCTCCTCATCTG | SEQ ID No.15/T. spl |
| rpoB | Tb_rpoB R3 | ATCCACCAAGATATAGCATCCA | SEQ ID No 16/T.sp1 |
| Pgk | Tb_PGK F1 | GCTCCWCCACCWGTAGAAAC | SEQ ID No. 17/T.sp1 |
| Pgk | Tb_PGK R1 | TYCGTGTAGATTTTAATGTGCCT | SEQ ID No. 18/T.sp1 |
| atpD | Tb_atpD F1 | TGGYCCAGTWATCGATGTTGA | SEQ ID No 19/T.sp1 |
| atpD | Tb_atpD R1 | AATACGYTCTTGCATTGCTC | SEQ ID No 20/T spl |
| fusA | Tb_ atpD F2 | ATGGTAACTCACCCATTCCAGA | SEQ ID No 21/T.sp1 |
| fusA | Tb_ b_atpD R2 | TGGCATGAIGCAACACAAGG | SEQ ID No. 22/T.sp1 |

| | | | |
|---|---|---|---|
| * Giovannoni, S.J., et al., 16S rRNA genes reveal stratified open ocean bacterioplankton populations related to the Green Non-Sulfur bacteria. Proc Natl Acad Sci U S A, 1996. 93(15): p. 7979-84 | | | |

All *Tenacibaculum* field isolates and strains included in the genotypic and phylogenetic characterization are listed in table 1 above. For the phylogenetic analysis a total of three alignments were constructed; a 16S rDNA sequence alignment of 1349 positions containing sequences from strain HFJ^{T} = *T. finnmarkense*^{T}, the isolates T. sp2-7 and their three genetically closest type strains *T. dicentrachi*^{T}, *T. ovolyticum*^{T} and *T. soleae*^{T}. *T. maritimum^{T}* was used as an outgroup. The second 16S rDNA sequence alignment consisted of 1341 positions and includes sequences of HFJ^{T} = *T. finnmarkense*^{T}, and the 21 published type strains in the genus *Tenacibaculum. Kordia algicida*^{T} was used as an outgroup. In the latter alignment, all sequences were obtained from GeneBank with the exception of *T. flnnmarkense^{T}, T. dicentrachi^{T}, T. ovolyticum^{T}, T. soleae^{T} and T. maritimum^{T}.* The third alignment, of 6954 positions, was constructed by using a concatenated sequence of the five Housekeeping genes (HK); atpD at position 1-807, fusA at position 808-1779, Pgk at position 1780-2715, rpoB at position 2716-5982 and tuf at position 5983-6954. Alignments were constructed in AlignX in the Vector NTI® v.9.0 (Invitrogen) software package before sequences were manually adjusted to equal length and correct reading frames in GeneDoc (Nicholas et al. (1997), EMBNEW.NEWS, 4:14). Concatenation of HK alignments was performed using KAKUSAN4 (Tanabe Tanabe (2011), Molecular Ecology Resources, 11, p. 914-921.
The best fitted evolutionary model for each alignment was calculated using Mega 6 (Tamura et al., 2013, MEGA6: Molecular Evolutionary Genetics Analysis version 6.0. Molecular Biology and Evolution). For the bayesian analysis of the concatenated HK alignment, KAKUSAN4 (Tanabe, 2011, *supra)* was used for calculation of substitution rate and the best fit model for the individual loci and codon positions and exported into a Mr. Bayes-block (V. 3.2.2 x86). A phylogenetic analysis of all three alignments were conducted using the Maximum Likelihood (ML) method with the best fitted evolutionary model, 1000 bootstrap replications and default settings in Mega 6 (Tamura et al., 2013, *supra).* The Beast package v1.8 (Drummond et al. (2007), BMC Evolutionary Biology, 7, p. 214) was used for bayesian analysis of the two 16s rDNA datasets using the best fitted model, relaxed lognormal molecular clock and a mcmc of 100 000 000 generations. The Bayesian phylogenetic analysis of the HK dataset was conducted in MrBayes V. 3.2.2 (Ronquist et al. (2012), Systematic Biology, 61, p. 539-542) using the data block with Proportional Codon, Proportional model from KAKUSAN4 (Tanabe, 2011) and a mcmc of 100 000 000 generations. The phylograms for the ML analysis were constructed in Mega 6 (Tamura et al., 2013, *supra).* The Effective sample size values (ESS) in the Bayesian analysis were inspected using Tracer ver. 1.4. ESS values were higher than recommended (200) for all parameters. A maximum clade credibility tree was obtained using a 10% burn-inn in Tree-Annotator and viewed using FigTre (Drummond et al. (2012), Molecular Biology and Evolution, 29, p. 1969-1973). For 16S rDNA sequence similarity analysis, Percent Nucleotide identity (PNI) were calculated using the distance matrix option in BioEdit (Hall (2011), Int J Syst Bacteriol, 42, p. 451-8). For calculating Average Nucleotide identity (ANI), the sequences of *T. finnmarkense^{T}, T.dicentrarchi^{T}, T.ovolyticum^{T}, T.soleae^{T}* from the concatenated HK alignment was uploaded and analyzed using the Average Nucleotide Identify option in EzGenome (Kim et al. (2012), International Journal of Systematic and Evolutionary Microbiology, 62, 716-721).

### Phenotypic characteristics

Morphological, physiological and biochemical tests were performed as described by Bernardet et al. (2002). All tests were performed on cultures incubated for 48h at 16°C unless otherwise stated. The type strains of the closest phylogenetic neighbors (*T.dicentrarchi* NCIMB 14598^{T}, *T.ovolyticum* NCIMB 13127^{T}) were obtained from freeze-dried cultures from the National Collection of Industrial bacteria (NCIMB) and grown under the same conditions as the isolate and used as reference strains in the phenotypic tests. Colony shape, margin, elevation, size, texture, appearance, pigmentation and optical property were examined as described by Smibert et al. (1994), Phenotypic characterization. In: GERHARDT, P., MURRAY, R. G. E., WOOD, A. W. & KRIEG, R. N. (eds.) Methods for General and Molecular Bacteriology. Washington D.C.: American Society for Microbiology.

The ability to stick to agar and viscosity of the colonies was also investigated. Cell morphology was investigated using scanning electron microscopy (SEM), transmission electron microscopy (TEM) and light microscopy. Gliding motility was determined by phase contrast microscopic examination of a Marine broth (MB) (Difco 2216) culture by the hanging drop technique as recommended by Bernardet et al. (2002) International Journal of Systematic and Evolutionary Microbiology, 52, p. 1049-70.

Presence of flexirubin type pigments was determined by the bathochromic shift test using a 20% (w/v) KOH solution (Fautz et al., 1980, FEMS Microbiology Letters, 8, p. 87-91). Congo red absorption was tested as described by Bernardet et al. (2002), *supra.* The Gram reaction were performed with the Fluka 77730 Gram Staining Kit (Fluka® analytical) following the manufacturer's protocol and the non-staining KOH method (Buck, 1982, Applied and Environmental Microbiology, 44, p. 992-993). The Voges-Proskauer reaction was performed as described (Pineiro-Vidal et al., 2012, *supra).* Oxidase activity and ability to split indole from tryptophan was tested using BBL™ DrySlide Oxidase and BBL™ DrySlide Indole (BD BBL™, U.S.A), following the manufacturer's protocol. Catalase activity was examined using the slide (drop) method following the protocol by Reiner (2010), Catalse Test Protocol [Online]. ASMMicrobeLibrary: American Society For Microbiology. Available: http://www.microbelibrary.org/library/laboratory-test/3226-catalase-test-protocol 2014]. Growth under anaerobic conditions was tested on MA using the GasPak anaerobic system (BBL). Production of H₂S was detected by taping a lead acetate impregnated paper strip (Sigma) to the inside of the lid of MA plates, using Parafilm to seal lid and plate. Growth on blood agar was tested using blood agar containing 2% NaCl (BAS) (Microbial laboratory, Haukeland University Hospital, Bergen). Evaluation of H₂Sproduction and growth on BAS was evaluated after 7 days of incubation. Degradation of starch (1% w/v), casein (1% w/v), and tween 80 (1% v/v) was investigated on MA. MB supplemented with gelatin (1% w/v) was used to investigate degradation of gelatin. Utilization of carbon sources was tested on basal agar medium [0.2 g NaNO₃, 0.2 g NH₄Cl, 0.05 g yeast extract, 15 g agar and 36 g red sea salt] per liter distilled water containing 0.4% carbon source [D(+)-sucrose, D(-)-ribose, D(+)galactose, D-glucose, L-proline, L-glutamate, L-tyrosine] as described by (Suzuki et al., 2001, *supra).* Absent of growth after one month of incubation was recorded as negative. Other enzymatic reactions were evaluated in the API ZYM system (bioMerieux) following the manufacturer's instructions, except that sterile seawater was used as suspension medium. Growth at pH 4-10 (at unit intervals) was assessed in MB; pH was adjusted using 1 M NaOH and 1 M HCl. The temperature range for growth was determined on MA plates incubated at 2, 4, 8, 16, 20, 25, 30 and 37°C for 7 days. Salinity requirement was determined with saltless MA [per liter distilled water: 5.0 g peptone, 1 g yeast extract and 0.1 g ferric citrate] containing 10, 20, 30, 50, 70 and 100% strength seawater (100% seawater = 38.2 g/l red sea salt) or 0.8, 1.0, 3.0, 5.0, 7.0 and 10.0% (w/v) NaCl (Sigma). Sensitivity to antimicrobials was evaluated by the disc diffusion method following the procedures of The Clinical and Laboratory Standards Institute (CLSI, 2005, Methods for antimicrobial disc suseptibility testing of bacteria isolated from aquatic animals; proposed guidelines M42-P Vol.25 No.21 Clinical and Laboratory standards institute).The tests were performed on MA plates using commercial discs (Neo-sensitabs™ and Sensi-disc™) containing kanamycin (500 µg), streptomycin (10 µg), gentamicin (30 µg), trimethoprim + sulfamethoxazole (1,25 + 23,75µg), ceftazidime (30 µg), ciprofloxacin (5 µg), pipemidic acid (30 µg), cefuroxime (30 µg), penicillin G (1U), ampicillin (2 µg), tetracycline (30 µg), erythromycin (15 µg), florfenicol (30 µg), oxolinic acid (10 µg) and oxytetracycline (30 µg), at 16°C for 10 days.

### Chemotaxonomic and genotypic characterization

Chemotaxonomic and genotypic analyses (DNA G+C content, DNA-DNA hybridization, menaquinones, fatty acid methyl esters) were carried out by the Identification Service of the DSMZ (Braunschweig, Germany). For DNA-DNA hybridization, cells were disrupted using a Constant Systems TS 0.75 KW (IUL Instruments, Germany) and the DNA in the crude lysate purified by chromatography on hydroxyapatite as described by Cashion et al. (1977), Analytical Biochemistry, 81, p. 461-466. DNA-DNA hybridization was carried out as described by Ley et al. (1970), European Journal of Biochemistry, 12, p. 133-142 under consideration of the modifications described by Huss et al. (1983), Systematic and Applied Microbiology, 4, p. 184-192 using a model Cary 100 Bio UV/VIS-spectrophotometer equipped with a Peltier-thermostatted 6x6 multicell changer and a temperature controller with *in-situ* temperature probe (Varian). Extraction of fatty acid methyl esters, washing of extracts and GC analysis were performed by using the Sherlock MIS (MIDI Inc, Newark, USA) system using the MIDI Sherlock version 6.1 and TSBA40 database.

### Results of the phenotypic and genotypic characterization of the novel Tenacibaculum isolate

Cells of strain Tsp.1 (HFJ^{T}) were rod-shaped, 0.5 µm wide and 5-25 µm in length and gram negative. Considerably longer flexible filamentous cells and spherical degenerative cells were frequently observed in older cultures. A rapid decrease in viability occurred with prolonged incubation (>96 h). Differential phenotypic characteristics between strain HFJ^{T}, *T.dicentrarchi^{T}* and *T.ovolyticum^{T}* are summarized in table 3.

**Table 3. Differential characteristics of strain Tsp.1 (HFJ^{T}), T.dicentrarchi^{T} and T.ovolyticum^{T}**

| **Characteristic** | **HFJ^{T}** | ***T. dicentrarchi^{T}*** | ***T.ovolyticum^{T}*** |
|---|---|---|---|
| Cell size | 5-25 x 0.5 µm | 2-40 µm | 2-10 µm |
| Colony morphology | | | |
| Color | Yellow | Brownish yellow | Pale yellow |
| Growth temp °C | 2-20 | 2-25 | 8- 25 |
| Salinity range | | | |
| NaCl | NG | NG | NG |
| Seawater%* | 50-100 | 50-100 | 70-100 |
| pH range | 4-9 | 5-10 | NT |

| **Growth on:** | | | |
|---|---|---|---|
| L-tyrosin | - | - | + |
| D-glucose | - | - | + |
| Blood agar (2% NaCl) | - | + § | - |

| **Hydrolization of:** | | | |
|---|---|---|---|
| Tween 80 | - | + | + |
| H₂S | - | + | + |

| **API-ZYM:** | | | |
|---|---|---|---|
| Esterase (C4) | + | + | - |
| Cystein arylamidase | + | + | - |
| Trypsin | - | - | + |
| N-acetyl-β-glucosaminidase | - | - | + |
| G+C (mol %) | 34.1 | 31.3 | 30.3 |

| | | | |
|---|---|---|---|
| All data is from this study, except the DNA G+C contents of the two reference strains taken from Piñeiro-Vidal et al. (2012) and Suzuki et al. (2001). +, Positive; -, negative; NT, Not tested; NG, no growth. *Percent calculated using a relation of 100% seawater = 38.2 g red sea salt 1⁻¹. § Induces beta- hemolysis or causing hemedigestion on blood agar containing 2% NaCl. Hemedigestion: a non-specific destruction of red blood cells (CDC, 2013) | | | |

The G+C content of strain *Tsp.1* (HFJ^{T}) was 34.1 mol% and is within the range reported for other type strains in the genus *Tenacibaculum* (29.8-35.2 mol%). The major fatty acids (>5% of the total fatty acids) were iso-C_{15 : 0}, anteiso-C_{15 : 0}, ISO-C_{15 : 1} and iso-C_{15 : 0} 3-OH. Fatty acids that made up >1% of the total mass for strain HFJ^{T} and *T.dicentrarch^{T}* are listed in table 4 below. The respiratory quinone was menaquinone 6 (100 %) and flexirubin-type pigment is absent. This is in accordance with the chemotaxonomic characteristics of the genus *Tenacibaculum* (Suzuki et al., 2001).

**Table 4. Cellular fatty acid composition (%) of strain HFJ^{T} and T.dicentrarchi NCIMB 14598^{T}.**

| **Fatty acid** | 1 | 2 |
|---|---|---|
| Straight chain | | |
| C_{14 : 0} | 1.3 | 1.0 |
| C_{15 : 0} | 1.8 | 3.8 |

| Branched chain | | |
|---|---|---|
| iso-C_{13 : 0} | 1.3 | 1.3 |
| iso-C_{14 : 0} | 1.6 | 2.5 |
| iso-C_{15 : 0} | 17.1 | 15.2 |
| anteiso-C_{15 : 1} | 17.7 | 13.3 |
| Iso-C_{15 : 1} | 9.5 | 9.0 |
| anteiso-C_{15 : 1} | 1.9 | 1.9 |
| iso-C_{16 : 0} | Tr | 1.0 |
| iso-C_{16 : 1} | Tr | 2.8 |

| Unsaturated | | |
|---|---|---|
| C_{15 : 1}ω6C | 3.3 | 3.1 |
| C_{16 : 1}ω5c | 1.8 | 1.6 |
| C_{17 : 1}ω6c | Tr | 2.0 |

| Hydroxylated | | |
|---|---|---|
| iso-C_{15 : 0} 3-OH | 12.4 | 11.6 |
| C_{15 : 0} 2-OH | 1.0 | 1.3 |
| C_{15 : 0} 3-OH | 1.5 | 2.2 |
| iso-C_{16 : 0} 3-OH | 2.9 | 5.3 |
| C_{16 : 0} 3-OH | 4.0 | 3.8 |
| iso-C_{17 : 0} 3-OH | 2.4 | 2.7 |
| Summed feature 3* | 9.5 | 10.3 |

| | | |
|---|---|---|
| Strains: 1, HFJ^{T}; 2, *T.dicentrarchi* NCIMB 14598^{T}. All data are from this study. Fatty acids amounting to <1% of the total fatty acids in all strains are not shown. Tr, Trace (<1 %). *Summed features are groups or two or three fatty acids that cannot be separated by GLC using the MIDI system. Summed feature 3 comprises C_{16 : 1} ω7c and/or iso-C_{15 : 0} 2-OH. | | |

The 16S rDNA gene sequence analysis (PNI) showed a similarity between strain HFJ^{T} and *T.dicentrarchi*^{T}, *T.soleae*^{T} and *T.ovolyticum*^{T} of 97.2%, 96.4% and 96.0%, respectively. DNA-DNA hybridization was necessary as strain HFJ^{T} showed more than 97% 16S rDNA sequence similarity to *T.dicentrarchi*^{T} (Stackebrandt et al. (1994), International Journal of Systematic Bacteriology, 44, p. 846-849, Tindall et al. (2010), International Journal of Systematic and Evolutionary Microbiology, 60, p. 249-266). The DNA-DNA hybridization tests revealed that the DNA relatedness of strain HFJ^{T} was 76.1 (70.3)% to *T.dicentrarchi*^{T} and 36.6 (39.7)% to *T.ovolyticum*^{T}*.* Results from replicate tests are shown in parentheses. The recommendation of a threshold value of 70% DNA-DNA relatedness for delineation of bacterial species by the *ad hoc* committee (Wayne et al., 1987, Int J Syst Bacteriol, 37, p. 463-464), means that strain HFJ^{T} is not a member of *T.ovolyticum*^{T}*.* Furthermore, in order to strengthen the conclusion that the isolated strain belong to a novel Tenacibaculum species, a polyphasic approach that integrates the phenotypic data with the genotypic and phylogenetic data was performed. This approach is recommended by several authors for bacterial taxonomic investigations (Bernardet et al., 2002, *supra,* Tindall et al., 2010, *supra).*

An ANI value of 95-96% has been found to correspond to the DDH threshold value of 70% (Richter et al. (2009), Proceedings of the National Academy of Sciences, 106, 19126-19131, Kim et al., 2014, *supra).* Comparing the ANI value derived from the concatenated HK gene sequence for strain HFJ^{T} and *T.dicentrarchi* revealed an ANI value of 94.4%. This supports that the DDH similarity might actually be less than 70% for strain HFJ^{T} and *T.dicentrarchi*^{T}*.* This is further supported by a study by Kim et al. (2014), *supra,* that revealed that a 16S rDNA sequence similarity between two strains of 98.65% would correspond to an ANI of 95-96 %. Accordingly, the 97.2% 16S rDNA sequence similarity between strain HFJ^{T} and *T.dicentrarchi*^{T} would correspond to an ANI less than 95-96 %.

A DDH similarity below 70% for strain HFJ^{T} is also supported by that 16S rDNA sequence similarities between six type strains in genus *Tenacibaculum* have sequence similarities between 99.4-98.2%, yet have DDH values below 70%. (Piñeiro-Vidal et al. (2008), International Journal of Systematic and Evolutionary Microbiology, 58, p. 21-25, Park et al. (2013), Antonie van Leeuwenhoek, 104, p. 225-231).

The phylogenetic analysis based on the 16S rDNA sequences and the concatenated HK gene sequences showed that strain HFJ^{T} clustered together with other type strains of genus *Tenacibaculum,* but formed a distinct branch separated from the closest related type strains. This separation was evident in all phylogenetic trees using both bayeshian and maximum likelihood method. All phylogenetic trees showed *T.dicentrarchi*^{T} as the closest related type strain, from which strain HFJ^{T} formed a distinct branch with robust support, clearly separated from *T.dicentrarchi*^{T}*.*

Results from the phenotypic tests shows that strain HFJ^{T} differs from *T.dicentrarchi*^{T} in several tests (Table 3), and strongly supports strain HFJ^{T} as a novel species.

In conclusion, the differential genotypic, phylogenetic, phenotypic and chemotaxonomic data presented indicates that strain Tsp.1 (HFJ^{T}) should be classified as a novel species in genus *Tenacibaculum.* The figures 3, 4 and 4 shows the evolutionary history in respect of the 16S RNA genes and various housekeeping genes, respectively, in comparison with prior art *Tenacibaculum* strains.

### Example 4: Challenging trial

### Pilot study

A pilot study, using Atlantic salmon smolts, was conducted to determine the most effective way of inducing tenacibaculosis. The fish were divided into four separate fiberglass tanks of 150 L, with an estimated water flow of 375 L/hour. The light regime consisted of a 12:12 hour schedule. Each tank held seven fish, and the water temperature was held at 4 °C. Low water temperatures were chosen since field outbreaks of tenacibaculosis in Norway typically occur during temperatures below 8 °C. The tanks were inspected twice a day, and moribund or dead fish were removed and sampled. The water temperature, salinity and oxygen levels were measured daily. The salinity was kept at approximately 34.0 ‰ throughout the experiment. Seawater was supplied from an intake at 105 meters depth, first filtered through at drum filter at 20 µm and subsequently treated with a UV -filter.

Inoculation cultures were incubated for two days prior to the pilot challenge at 16 °C. The inoculation cultures consisted of 2 x 1.5 liter Erlenmeyer flasks with 1 L marine broth per isolate. The bacterial isolates were added to the inoculation cultures by suspending agar cultures in marine broth, and subsequently added to each flask. On the day of challenge, 500 µL of bacterial culture was sampled from one flask per isolate and diluted to 10⁻⁶. The dilution series were then counted in a Bûrker counting chamber, to estimate the challenge dosages for both isolates. The formula used to calculate the number of cells in 10 µL was provided by the producer (see appendix). 100 µL of each dilution was also plated on marine agar to determine the CFU (Colony Forming Units).

The shedders in tank 1 and 2 were bath-challenged with *T.sp1* and *T.sp2,* respectively, for 17 hours. The prolonged exposure time was chosen, because previous challenge studies with *Tenacibaculum maritimum* have demonstrated a necessity for an 18 hour challenge time to induce tenacibaculosis (Avendaño-Herrera et al.,2(006), Diseases of Aquatic Organisms, 2004. 58(1): p. 1-8). Prior to bath-challenge, the fish were crowded for 5 minutes with a hand net to induce a stress reaction. The fish were thereupon transferred to a 100 L container, containing 60 L of seawater at 4 °C. The temperature was held by submerging the challenge-container into a second container, in which water at 4 °C flowed continuously.

Oxygen and fish behavior was monitored every 30 minutes during the challenge. Oxygen was supplied by two oxygen-diffusors in each challenge container. The shedders in tank 1 and 2 were then challenged by adding bacteria incubated in 3 L of marine broth each. The fish were subsequently transferred back to the main 150 L tanks after the challenge period. However, some fish had to be transferred back to the main tanks earlier, because of signs of distress. The fish in the two remaining tanks, tank 3 and 4, were challenged with a rostral sub-dermal injection of 0, 5 mL of MB (Marine Broth) containing suspended bacteria from the same inoculation cultures used in the bath challenge. Prior to injection, the fish was anesthetized with Finquel™ (Tricaine).

### Large scale challenging trial

A larger main study was thereafter conducted, based on the results from the pilot study. In this main experiment, eight tanks were included, with 62 smolts in each tank, divided into four separate groups. The challenge study was run in duplicates. The T.spl-isolate was included into two groups, group 1 and 2, as it was believed to be the most virulent based on the pilot study. The *T.sp2*-isolate was only included in group 3. The injection challenge was not applied in the main study because no injected shedders developed disease in the pilot study. The remaining setup was as described for the pilot study.

**Table 5: Showing the different groups and tanks with corresponding challenge isolates and challenge times. w: with ; MB: Marine Broth; L : Liter; mL: Milliliter. The challenge dosages were calculated by cell counting. Revised challenge dosages as calculated by CFU are listed in below.**

| **Group** | **Tank** | **Isolate** | **Challenge time** | **Inoculum** | **Numberof bacteria** |
|---|---|---|---|---|---|
| **1** | 1, 2 | *T.sp1* | 10 hours | 2 L MB w *T.sp1* | 2,04 x 10⁸ |
| **2** | 3, 4 | *T.sp1* | 1/5 hours | 100 mL MB w *T.sp1* | 1,94 x 10⁷ |
| **3** | 5, 6 | *T.sp2* | 10 hours | 2 L MB w *T.sp2* | 2,70 x 10⁸ |
| **4** | 7, 8 | Control | 10 hours | 2 L MB | - |

Prior to challenge, the inoculum was prepared using MB, incubated at 16 °C at 140 rpm. For group 1 and 3, the *T.sp1* and *T.sp2*-isolates was grown on marine agar, before it was added to two liters of marine broth. In group 2 however, the inoculum consisted of only 100 mL of MB. In group 4, no bacteria were added to the broth. The bacterial isolates were added to the MB two days before challenge to ensure that the bacteria were in exponential growth curve during challenge. As for the pilot study, a dilution series from 1 to 10⁻⁸, was created to estimate the challenge dosages, using a Bûrker counting chamber and CFU (Colony Forming Units).

In each tank, 20 fish were selected to be shedders (except for tank 6, where a counting error resulted in only 19 shedders). For every separate challenge, 40 shedders were challenged at the same time, 20 for every tank. The shedders were crowded five and five individuals for 5 minutes, before they were transferred to a bucket containing 10 liters of seawater and Finquel (Tricaine™). When the fish exhibited a loss of equilibrium, the adipose fin was cut to mark the individual as a shedder. The fish were then conveyed into the 60 liter challenge container. Before the bacterial culture was added, the fish were allowed to acclimatize to the challenge container for 20 minutes. Because acute mortality was observed in the pilot study after the 17 hour exposure time, a reduced challenge time of 10 hours was used in group 1,3 and 4. In group 2, 20 fish from each tank were challenged only one hour, while the remaining 20 were challenged for five hours. Upon retransfer to tanks 3 and 4, the fish in group 2, challenged for one hour was marked with a fluorescent dye, while the ones challenged for 5 hours, were cut in the adipose fin.

### Sampling

Each tank was examined twice a day, and moribund or dead fish was sampled continuously. The experiment was terminated 42, 44, 45 and 47 days post infection for group 1, 2, 3 and 4, respectively. The remaining fish were then killed and sampled. All fish were killed by a blow to the head immediately before sampling, to avoid degradation of tissue. Sampled fish were transported to the dissection laboratory in separate plastic bags, and immediately put on ice or in a cold locker at 4 °C, whilst sampling was carried out. Prior to sampling, the length and weight of each individual was recorded. Any clinical and macroscopic pathology were photographed and noted.

Tissue samples for Real-Time RT-PCR of approximately 3 mm³ were taken from 35 individuals per tank. The Real Time RT-PCR-samples consisted of skin, gill, ventricle and head kidney. Skin tissue was collected from affected areas, margins of ulcers if present, or from the top of the operculum. Gill samples were taken from the second gill arch. In addition to the regular tissue samples, tissues from spleen, mucus, tailfin and blood were also collected from two cohabitants from group 2 that displayed an apparent tenacibaculosis. The tissue samples were stored at -32 °C.

Histology samples were taken from the margins of ulcers, affected areas or normal tissue for comparison. Kidney-, heart-, eyes-, brain-, spleen-, liver- and pylorus samples were only collected from two cohabitants, from tank 4, which displayed an apparent Tenacibaculosis. The samples were placed in a modified Karnovsky fixative solution. The modification made to the Karnovsky fixative, is a replacement of water by Ringer's solution, containing 4 % sucrose. The sample glasses were then stored at 4 °C. The samples used for histology were prepared with a Technovit ™ 7100 kit (Kulzer).

Bacteriological sampling was performed with an inoculation loop. Samples were taken from margins of ulcers, gills, damaged areas or simply from the ventral side of the host. Samples from skin, gills and kidney were streaked on blood agar (BA) containing 2 % NaCl (Microbial laboratory, Haukeland hospital), and on marine agar (MA) (Difco™). Furthermore, streaks from head kidneys and margins of ulcers were always smeared on both marine agar and blood agar. Plates from separate individuals were kept in separate plastic bags and put directly into an incubator at 16 °C and monitored daily for growth. If growth was detected, each colony type was examined under a microscope. Colonies that were suspected of being *T.sp1* or *T.sp2* were transferred to a new plate, before DNA was extracted. A representable number of bacterial colonies, not resembling *Tenacibaculum,* were also transferred to new plates, and DNA was extracted to be used for sequencing.

In addition, mucus from skin and gills were smeared on microscope slides, and thereafter colored using Colorrapid™ (Lucerna Chem). The slides were then examined under microscope, to uncover the presence of bacteria resembling *Tenacibaculum* sp.

### RNA and DNA extraction

RNA was extracted from fish tissue and bacterial cultures, using a protocol developed at the Fish Disease Group at UiB. The tissues and cultures were lyzated with Isol-RNA Lysis Reagent™ (5-prime) (see Appendix). Samples were eluted in 50 µL RNase-free water, heated to 70 °C. For every session of RNA extraction, two negative controls (NTs) were included to test for possible contaminations. All RNA samples were stored at -32 °C.

DNA was extracted from a loop of bacteria from a pure culture, using an E.Z.N.A. tissue DNA kit™ (Omega Bio-Tek) (see Appendix). DNA extraction was carried out with a modification of the kit protocol. Instead of washing bacteria with a PBS-solution, the bacteria were added directly into a 1.5 mL Eppendorf tube containing 220 µL BL buffer and 25 µL OB protease. The following extraction was performed in accordance to the producer's recommendations. DNA samples were stored at -32°C.

### Polymerase Chain Reaction (PCR)

PCR was performed using unspecific 16S primers and specific primers for 3 genes (16S rRNA, tufA and rpoB) in this study (see table 3). Amplification was based on a standard reaction mixture containing 2.5 µL 10x Extra buffer (VWR®), 1.25 mM DNTP (Deoxyribonucleotide Triphosphate), 0.75 units (0.15 µL) *Taq* DNA polymerase (VWR®) 5 µM (1 µL) of forward and reverse primer and DNase, RNase free water to a final solution of 25 µL (16.85µL H₂O). Amplification was performed in a GeneAmp PCR system 2700 (Applied Biosystems) at 94°C for 5 minutes; 35 cycles of 94 °C for 30 seconds, 58 °C for 30 seconds, 72 °C for 90 seconds, followed by 72 °C for 8 minutes. PCR product was confirmed by gel electrophoresis, and subsequently purified using ExoStar 1-Step ® (illustra™) in an Artik Thermal Cycler (Thermo Scientific) at 37 °C for 15 minutes and 80 °C for 15 minutes. The reaction was performed with a mixture of 2 µL ExoStar and 5 µL PCR-product. The PCR primers used in this experiment is shown in table 2 above.'

Gel electrophoresis was performed using a gel containing 1 % agarose dissolved in 1 X Tris-acetate-EDTA (TAE) buffer. The fluorescent dye GelRed ™ (Biotium, Inc., USA) was added to the gel to stain the nucleic acids. Afterwards, the gel was allowed to harden for 10-15 minutes and subsequently immersed in 1 X TAE-buffer. Before PCR product was added to the wells in the gel, a volume of 1 µL loading dye 6x was mixed with 5 µL of PCR product. A volume of 5 µL Smartladder (Eurogentec) was added in the first well, to be used as a molecular weight marker. Gels were run for 1 hour and 10 minutes at 80 Volts, before examination under UV-light in a Gel Logic 212 Pro (Carestream, U.S.A.) machine.

Sequencing of the PCR products using two unspecific 16S-rRNA-primers (Eug B27F and Eug A1518R) was performed to verify the identity of *Tenacibaculum*-like colonies isolated from the fish in the challenge experiment. The reaction was accomplished using a BigDye® version 3.1-reaction in an Arktik Thermal Cycler, at 96 °C for 5 minutes; 30 cycles of 96 °C for 10 seconds, 58 °C for 5 seconds and 60 °C for 4 minutes. The reaction was composed of a mixture of 6 µL deionized water, 1 µL BigDye® Terminator 3.1 version sequencing buffer, 1 µL BigDye Terminator 3.1 version Ready Reaction Premix (2.5X) (Invitrogen), 3.2 pmol (1µL) forward and reverse primer and 1 µL purified PCR product. After completion of the reaction, a volume of 10 µL deionized water was added before the sample was delivered to the sequencing facility (Høyteknologisenteret In Bergen, http://www.uib.no/seqlab). Here the samples were purified with an Agencourt CleannSeq (Beckman Coulter, Inc.) before being sequenced in a 96-capillary 3730xl DNA Analyzer (Applied Biosystems®). The software Vector NTI® v.9.0 (Invitrogen) was used to analyze the received sequences.

In order to detect the presence of *T.sp1* and *T.sp2* in the water- and tissue samples, Real Time RT-(Reverse transcriptase) PCR was performed. AgPath-ID™ One-Step RT-PCR was used in order to set up a Real Time RT-PCR reaction mixture for all assays. The reaction mixture consisted of 6.25 µL 2X RT PCR Buffer, 400 nm forward primer, 600 nm reverse primer, 170 nm probe, 2.60 µL of deionized water, 0.5 µL of enzyme mix and 2 µL RNA-template for a final volume of 12.5 µL per well. Analysis was performed in 7500 Fast Real Time PCR system and 7500 Real Time PCR System (Applied Biosystems). The amplification curves were given a fixed threshold line at 0.1. The temperature regime consisted of a standard AgPath run protocol of 45 °C for 10 minutes (reverse transcriptase); 95 °C for 10 minutes (Polymerase activation); 45 cycles of 95 °C for 15 seconds (DNA-dissociation) and 60 °C for 45 seconds (annealing and elongation).

Skin samples (S) were selected as the most relevant tissue for Real Time PCR analysis, and as such, all skin samples collected were analyzed for *Tenacibaculum* sp., *M.viscosa* and salmon elongation factor alpha 1 A. In addition, kidney samples from 10 shedders and 10 cohabitants from each group were analyzed. Real Time RT-PCR analysis was also performed on skin-, ventricle-, kidney-, brain-, spleen-, gill-, blood, mucus and tailfin tissue of two cohabitants in group two that exhibited an apparent *Tenacibaculum* infection. This was done to examine possible tissue tropism for the *Tenacibaculum* sp. isolates. Non-template-Controls (NTCs) were included in each Real-Time RT-PCR analysis. Negative RNA-extraction samples (NTs) were also included in each plate, to demonstrate that RNA samples were not contaminated during extraction.

Since no Real Time RT-PCR-assay has been made to detect *Tenacibaculum* sp., an assay had to be developed. The different assays developed in this study, are listed in table 4. Two new assays targeting *Tenacibaculum* sp. was designed by Øyvind Brevik (Cermaq) for this study (Tb_TUF and Tb_rpoB), all using Taqman probes. The amplicon for Tb_TUF is a 56 bp long fragment of the elongation factor Tu (EF-Tu), a predicted highly expressed gene (PHX) and a processing factor for protein synthesis (Karlin, S., et al.(2001), Journal of Bacteriology, 183(17): p. 5025-5040). The amplicon for Tb_rpoB on the other hand, is a 64 bp fragment of the gene for the DNA directed RNA polymerase β which is a processing factor for protein synthesis and predicted to be highly expressed (PHX) (Karlin, S., et al.(2001), *supra.* To exclude the possibility of the ulcers being induced by *M.viscosa,* a third assay was developed, targeting *M. viscosa.* The amplicon for this assay is a 70 bp fragment of the outer membrane protein ompA gene. To act as an endogenous control, an assay targeting EF1A (Salmon Elongation Factor 1 Alpha A) was included in this study (Olsvik, P.A. et al. (2005), BMC Molecular Biology, 2005. 6(1): p. 1-9), incorporated in its entirety herein by reference. This assay was used with a concentration of primers and probe of 400/400 nM and 140 nM, respectively.

**Table 6: The Real Time RT-PCR assays used in this study, their corresponding target and sequence, as well as their origin. P: Probe. F:Forward primer. R: Reverse primer**

| **Target** | **Primer/Probe** | **Direction** | **Sequence** | **SEQ ID No./Origin** |
|---|---|---|---|---|
| ***Tenacibaculum sp*** | TB_TUF F | Fwd | AGTGTGACGTCCACCTT | SEQ ID No. 23/T.spl |
| | Tb_TUF R | Rev | CTGTAAGCCAGGTTCTGT | SEQ ID No. 24/T.spl |
| | TB_TUF P | - | TTTCAATACATACACCTCAGC | SEQ ID No. 25/T.spl |
| ***Tenacibaculum sp*** | Tb_rpoB F | Fwd | GGAGCAAACATTGACCAAATT | SEQ ID No. 26/T.spl |
| | Tb_rpoB R | Rev | GGTATGTCCGTAACGTGGAA | SEQ ID No. 27/T.spl |
| | Tb_rpoB P | - | TCCTGCTTGATCAGTTAAAGCGT | SEQ ID No. 28/T.spl |
| ***M. viscosa*** | Mv_ompA F2 | Fwd | GATGATAACGCAACAGCAG | SEQ ID No. 29 |
| | Mv_ompA R2 | Rev | CGGAAACTTACACCAGATAATG | SEQ ID No. 30 |
| | Mv_ompA P2 | - | | SEQ ID No. 31 |
| **Salmon elongation factor 1 Alpha A** | EF1A F | Fwd | CCCCTCCAGGACGTTTACAAA | SEQ ID No. 32/Olsvik et al.* |
| | EF1A R | Rev | CACACGGCCCACAGGTACA | SEQ ID No. 33/Olsvik et al.* |
| | EF1AP | - | ATCGGTGGTATTGGAAC | SEQ ID No. 34/Olsvik et al.* |

| | | | | |
|---|---|---|---|---|
| *Olsvik, P.A., et al. (2005), *supra.* | | | | |

The two assays targeting *Tenacibaculum* sp. and the assay targeting *M.viscosa* were first tested against 27 bacterial isolates. This was done to ensure the specificity of the assays and to make sure that only target *Tenacibaculum-* and *Moritella-* RNA was amplified. The different bacterial isolates used to test the three assays are listed in table 7.

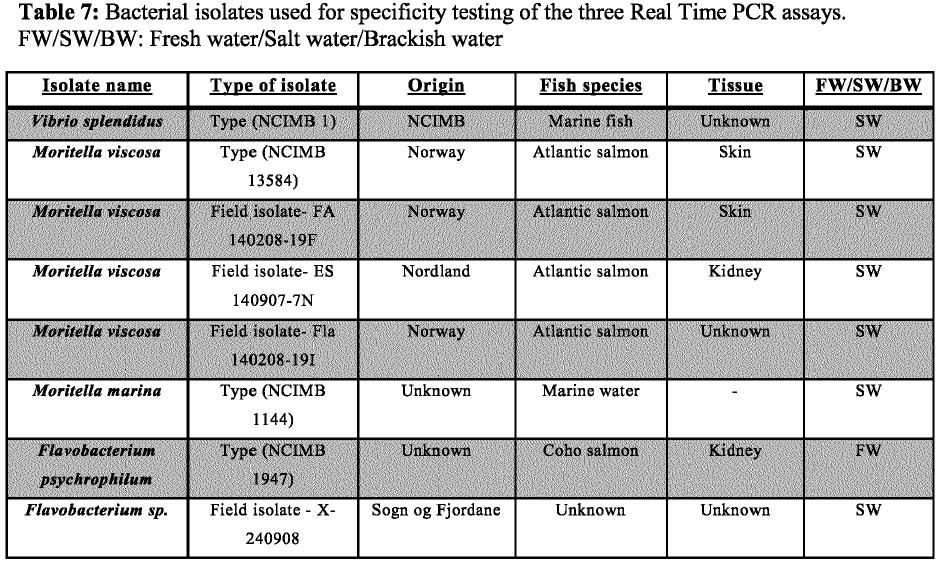

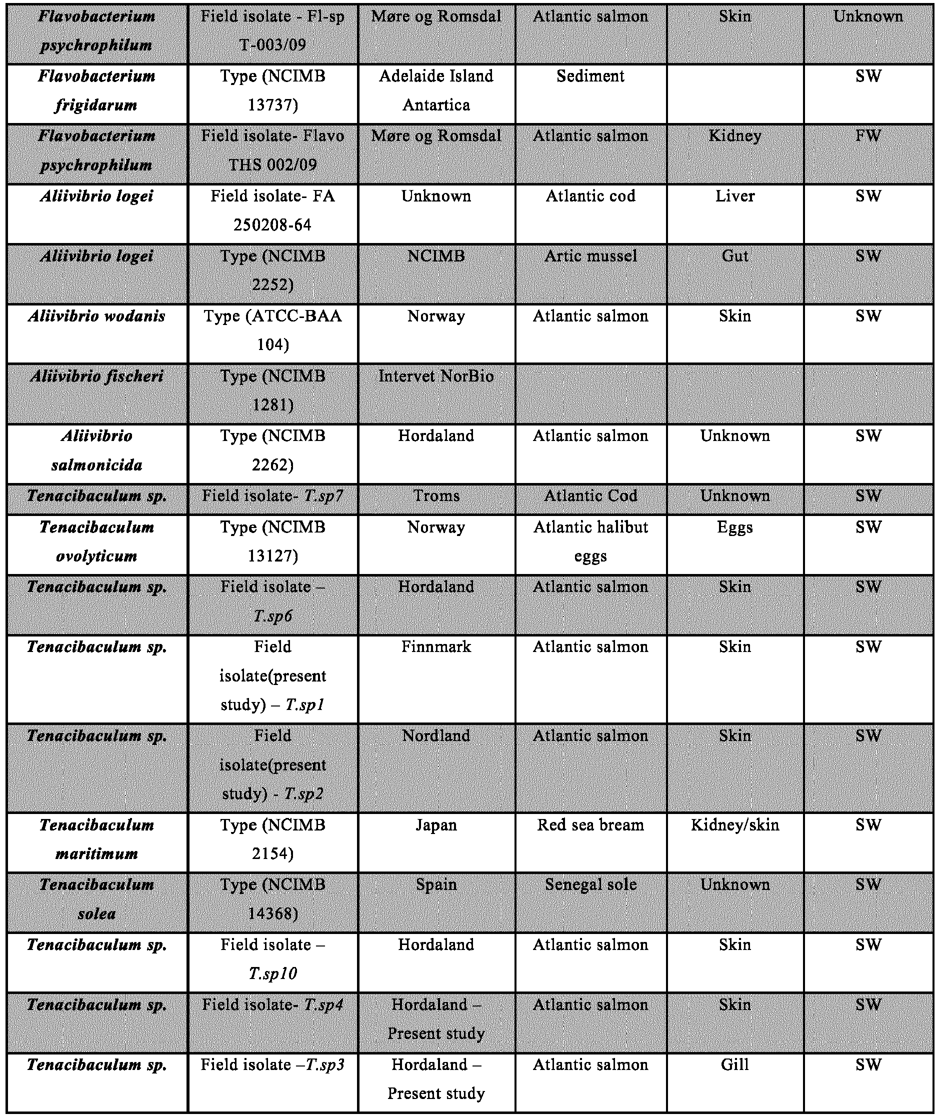

To optimize the assays, RNA from a pure culture of *T.sp1* and the type strain of *M.viscosa* (NCIMB 13584) was used. First, the assays were tested using 16 different concentrations of forward and reverse primer, using a recommended concentration of probe of 120 nM (Forward/Reverse in nm; 200-200, 200-400, 200-600, 200-800, 400-200, 400-400, 400-600, 400-800, 600-200, 600-400, 600-600, 600-800, 800-200, 800-400, 800-600, 800-800). Secondly, the assays were tested using six different concentrations of probe (in nM; 70, 95, 120, 145, 170, 195) and an optimized concentration of primer.

After the primer-probe optimization, an efficacy test was conducted for all three assays to test the assay's efficacy to detect target template. Target RNA from Tsp.1 (for the assays targeting *Tenacibaculum*) and from *M.viscosa* (NCIMB 13584) was diluted from 1 to 10⁻⁷. Each dilution was tested in triplicates. The average Ct-values was then entered in Microsoft Excel to create a standard curve. Excel was then used to calculate the regression number and slope of the graph. The two assays targeting *Tenacibaculum* sp. were at this stage compared to each other, to examine which had the highest sensitivity and lowest Ct-value.

To determine whether or not the rpoB-gene was expressed at the same rate during the exponential growth phase as during the degenerative phase, an expression-test was conducted. The *T.sp1*-isolate was grown in MB, and a 200 µl sample was collected from the culture after two and six days post inoculation. Thereafter, a ten-fold dilution series, from 1 to 10⁻³, was created using RNase free water. The number of bacteria in the 10⁻¹-dilution was thereupon estimated by counting a sample of 20 µl in a Malassez counting chamber (MARIENFELD™). Subsequently, each dilution was pelleted by centrifuging the tubes containing bacteria at 10 G for five minutes, before the water was removed from each tube. RNA was thereafter extracted from each sample and analyzed using Real Time RT-PCR. Each sample was run in triplicates.

The final stage of the development of the Real Time assays was a sensitivity-test of Tb_rpoB and Mv_ompA. Tb_TUF was not included in this test, as it's specificity was deemed too low for this challenge study .This was done by creating a tenfold dilution series of both Tsp.1 and M.viscosa (NCIMB 13584) from a stock of 200 µL of bacteria and marine broth, and then estimating the number of cells in the 10⁻¹-dilution of both bacteria in a Malassez counting chamber (MARIENFELD™).The average number of bacterial cells in the 10⁻¹-dilution was then calculated in accordance to the producer's recommendations. RNA was later extracted using an E.Z.N.A Total RNA Kit I ™ (Omega Bio-tek). Real Time RT-PCR was then performed as described earlier.

### RESULTS

### Real Time RT-PCR Assay testing

### Assay specificity

Three different assays were developed during this study and first compared to each other by performing a specificity test, using 27 different isolates of bacteria. The assays were tested against RNA obtained from pure cultures. The assay targeting *M.viscosa* (Mv_ompA) only tested positive for the four different *M.viscosa*-isolates, and did not test positive for any *Tenacibaculum-, Flavobacterium-* or *Aliivibrio*-isolates. The first assay targeting *Tenacibaculum* sp. (Tb_TUF) tested positive for ten different *Tenacibaculum-*isolates, but none of the *Moritella-, Aliivibrio-,* and *Flavobacterium-isolates.* The second assay targeting *Tenacibaculum* sp. (Tb_rpoB) tested positive for six different isolates of *Tenacibaculum,* but none of the *Moritella-, Aliivibrio-,* and *Flavobacterium*-isolates. The assays and the isolates are presented in table 8.

**Table 8: The three Real Time RT-PCR assays developed in this study and the bacterial isolates they tested positive for. Mv_ompA targeting M.viscosa, Tb_TUF and Tb_rpoB targeting Tenacibaculum sp. Tb_rpoB was chosen to analyze tissue samples from the challenge experiment because Tb_rpoB was shown to be slightly more specific towards the T.sp1- and T.sp2-isolates. All the bacterial isolates that were tested are listed in table 5.**

| **Mv_ompA** | **Tb_TUF** | **Tb_rpoB** |
|---|---|---|
| **NCIMB 13584 - *M.viscosa*** | NCIMB 13127 - *T.ovolyticum* | NCIMB 14368 - *T.solea* |
| **Fa 140208-19F *- M.viscosa*** | NCIMB 14368 - *T.solea* | *T.sp1* - *Tenacibaculum* sp. |
| **ES140907-7N - *M.viscosa*** | T.sp1 - *Tenacibaculum* sp. | *T.sp2* - *Tenacibaculum* sp. |
| **Fla 140208-191 - *M.viscosa*** | T.sp2 - *Tenacibaculum* sp. | *T.sp4* - *Tenacibaculum* sp. |
| | *T.sp3- Tenacibaculum* sp. | *T.sp7* - *Tenacibaculum* sp. |
| | *T.sp4- Tenacibaculum* sp. | *T.sp10* - *Tenacibaculum* sp. |
| | *T.sp6- Tenacibaculum* sp. | |
| | *T.sp7- Tenacibaculum* sp. | |
| | *T.sp10 -Tenacibaculum* sp. | |

### Assay optimization

The three assays were optimized for primer and probe concentration by using different combinations of primer and probe molarity. The optimized primer and probe concentrations are listed in table 9.

**Table 9: The different assays and their optimized combination of forward- and reverse primer and probe, used to analyze fish samples. The concentrations are given in nanomolar (nM).**

| **Assay** | **Forward primer (nM)** | **Reverse primer (nM)** | **Probe (nM)** |
|---|---|---|---|
| **Mv_ompA** | 400 | 600 | 170 |
| **Tb_rpoB** | 400 | 600 | 170 |
| **Tb_TUF** | 600 | 600 | 170 |

### Gene expression test

The gene expression test was conducted as explained above. The results from the test are listed in table 10 and figure 26. Spherical degenerative cells were observed when estimating bacterial numbers in the sample taken six days post inoculation. As such, it can be assumed that the culture used to create the dilution series was in the degenerative phase. The number of bacterial cells was much higher in the dilution series made for the exponential phase. Nonetheless, the Ct-values from the Real Time RT-PCR analysis are remarkably similar. The Ct-values for the last dilution of bacterial cells from the degenerative phase are actually lower than the Ct-values for the exponential phase.

**Table 10: The dilutions with corresponding numbers of bacteria per µL and total bacteria in suspension used to extract RNA, for subsequent Real Time RT-PCR analysis. A total volume of 200 µL of marine broth or DNase-free water was used to suspend the bacteria. Bacteria were sampled after two (exponential phase) and six days (degenerative phase). The Ct-values listed are the average Ct-value obtained from the triplicates.**

| **Phase** | **Dilution** | **Bacteria per µL** | **Bacteria total (200 µL)** | **CT-value obtained from Real Time RT-PCR** |
|---|---|---|---|---|
| **Exponential phase** | 1 | 3234.4 | 646 874 | 13.44 |
| | 10⁻¹ | 323.4 | 646 87.4 | 16.35 |
| | 10⁻² | 32.3 | 646 8.7 | 18.96 |
| | 10⁻³ | 3.2 | 646.8 | 22.75 |
| **Degenerative phase** | 1 | 1984.4 | 396 875 | 13.82 |
| | 10⁻¹ | 198.4 | 396 87.5 | 16.43 |
| | 10⁻² | 19.8 | 396 8.7 | 18.80 |
| | 10⁻³ | 1.9 | 396.8 | 22.15 |

### Sensitivity test

The sensitivity test was carried out as explained above with the assays used for detecting *Tenacibaculum* spp. (Tb_rpoB) and *M.viscosa* (Mv_ompA). The three first dilutions were tenfold, while the remaining nine dilutions were twofold. The sensitivity test of the two assays demonstrate the assays ability to amplify target RNA when diluted to as little as 0.35 cells (Tb_rpoB) and 0,07 cells (Mv_ompA). The total starting amount of bacterial cells was significantly higher in the *Tenacibaculum* dilutions (9140 cells per µL) than the *Moritella* dilutions (3640 cells per µL).

### Estimation of challenge dosage - pilot study

The challenge study was divided into four tanks with seven shedders in each tank. Tank 1 and 2 were bath-challenged for 17 hours with the *T.sp1-* and *T.sp2*-isolate, respectively. Tank 3 and 4 were challenged with a rostral injection of 0.5 mL of bacteria cultured in marine broth. However, as no infection resulted from the latter challenge method (data not shown). The challenge dosage for the pilot study bath challenge was only calculated on the basis of cell counting in a cell counting chamber (5.2 x 10⁹ cells ml⁻¹).

### Screening of fish prior to challenge experiment

Of the ten gill samples collected from the fish to be used in this study and screened by Real Time RT-PCR prior to the challenge experiment, none were positive for IPNV (Infectious Pancreas Necrosis Virus), ISAV (Infectious Salmon Anemia Virus), PRV (Piscine Orthoreo Virus), *Piscirickettsia salmonis* and *Yersinia ruckeri.* However, one fish tested weakly positive for *Flavobacterium psychrophilum* with at Ct-value of 36.5. In addition, one fish tested weakly positive for *Ichthyobodo salmonis* with a Ct-value of 33.4. The samples were collected before the fish was transferred to seawater. The fish was not screened for *Tenacibaculum* spp. or *M.viscosa* because the fish was held in freshwater.

### Mortalities - Pilot study

In tank 1, where the fish was challenged with the T.spl-isolate for 17 hours, acute mortality started after 2 days (figure 6). After three days, all shedders had been sampled. In tank 2, were the seven shedders were challenged with the *T.sp2*-isolate, mortalities started immediately 1 day post challenge. Nonetheless, the mortalities in tank 2 were more chronic than in tank 1, and the mortalities increased gradually. All shedders in tank 2 were sampled after six days. The macroscopic pathological observations in the pilot study are identical with the ones described for tank 1 and 2 in the main study (see below).

### Study design - large study

The challenge study was divided into 4 groups, with two duplicate tanks per group. Each tank had 20 shedders and 42 cohabitants. Group 1 (tank 1 and 2) were challenged with the *T.sp1*-isolate for 10 hours. Group 2 (tank 3 and 4) were also challenged with the *T.sp1-*isolate, but with a much lower dosage. 10 shedders from each of the two tanks were challenged for 5 hours, and an additional 10 shedders from each tank were challenged for 1 hour. Group 3 (tank 5 and 6) had 20 shedders challenged for 10 hours with the *T.sp2-*isolate. Group 4 (tank 7 and 8) were the control group and the shedders in this group was only exposed to marine broth.

### Estimation of challenge dosage - main study

Because the mortalities in the pilot study were too acute, the challenge medium was reduced from bacteria grown in 3 liters MB, to 2 liters MB in group 1 (tank 1 and 2) and 3 (tank 5 and 6), and 100 ml MB in group 2 (tank 3 and 4). The number of cells was estimated on the basis of counting bacterial cells from a 10⁻³ dilution of each challenge inoculum and from colony forming units (CFU). The result of the CFU-test is listed in table 11. The estimation of challenge dosages are listed in table 12.

**Table 11: The inoculum for each group used in the challenge experiment was diluted tenfold, using MB and PBS, and the dilutions were plated on marine agar plates. All dilutions of less than 10-4 demonstrated heavy growth, covering the entire plate or no growth and are as such not included in this table. No growth was observed for group 2, except in the 10-1 dilution, where the entire plate was covered. The 10-4 dilution was then used to calculate the challenge dosages in group 1 and 3.**

| **Group** | **Tanks** | **Isolate** | **Dilution of bacterial suspension plated on marine agar** | **Number of colonies formed** |
|---|---|---|---|---|
| **1** | T1 & T2 | *T.sp1* | 10⁻⁴ | 35 |
| | | | 10⁻⁵ | 3 |
| | | | 10⁻⁶ | 0 |
| | | | 10⁻⁷ | 0 |
| **2** | T3 & T4 | *T.sp1* | 10⁻⁴ | 0 |
| | | | 10⁻⁵ | 0 |
| | | | 10⁻⁶ | 0 |
| | | | 10⁻⁷ | 0 |
| **3** | T5 & T6 | *T.sp2* | 10⁻⁴ | 44 |
| | | | 10⁻⁵ | 0 |
| | | | 10⁻⁶ | 0 |
| | | | 10⁻⁷ | 0 |

**Table 12: The estimated number of cells per ml in the challenge medium was calculated for all groups on the basis of cell counting and CFU. No re-estimation of cells in challenge medium is listed for group 2 in the CFU-column because an error was made while creating the dilution series from the challenge medium. No re-estimation of cells in challenge medium is listed in group 4, as it was only challenged with marine broth without any bacteria.**

| **Group** | **Tanks** | **Isolate** | **Estimation of bacterial cells by counting chamber, cells ml-1** | **Re-estimation of cells in challenge medium by CFU, cells ml-1** |
|---|---|---|---|---|
| **1** | T1 & T2 | *T.sp1* | 2,04 x 10⁸ | 2,35 x 10⁵ |
| **2** | T3 & T4 | *T.sp1* | 1,94 x 10⁷ | - |
| **3** | T5 & T6 | *T.sp2* | 2,70 x 10⁸ | 2,93 x 10⁵ |
| | T7 & T8 | Control | - | - |

### Average weight during termination - main study

The average weight for all fish at the start of the challenge experiment was measured by ILAB's staff and was calculated to be 55 grams. The weight was also measured during termination of the experiment. In all tanks, the average weight varied from 60 to 67 grams. However, the differences in weight varied significantly within tanks, from 84 to 49 grams.

**Table 13: Average weight of all fish at the start of the experiment, and average weight of fish sampled during termination of the experiment. Average weight before experiment was measured by ILAB staff. N: Number of individuals included into the calculation of average weight.**

| | | |
|---|---|---|
| | | |
| *T.sp1* | 15 | 65g |
| *T.sp1* | 15 | 60g |
| *T.sp1* | 28 | 62g |
| *T.sp1* | 23 | 65g |
| *T.sp2* | 15 | 63g |
| *T.sp2* | 15 | 65g |
| Control | 15 | 67g |
| Control | 15 | 63g |

### Mortalities - Main study

In tanks 1 and 2, where the fish were challenged with the *T.sp1*-isolate for 10 hours, acute mortalities of shedders started as early as one dpc (days post challenge) (figure 7). During the first dpc, 13 shedders were sampled in tank 1 because of moribund/lethargic behavior. In tank 2, 16 shedders had to be removed and sampled one dpc. The following two days, all remaining shedders in tanks 1 and 2 had to be sampled because of moribund behavior. After all the shedders were removed and sampled, no mortality or symptomatic cohabitants were recorded in tanks 1 and 2 (figure 8).

In tanks 5 and 6, where the fish was challenged with the *T.sp2*-isolate for 10 hours, mortalities of shedders also started during the first dpc, but to a more chronic degree than in tanks 1 and 2 (figure 7). Mortalities were more or less constant over the following nine days, with one to five shedders removed daily because of lethargic behavior or extensive symptoms. After nine days all shedders had been removed and sampled. No cohabitants in tanks 5 or 6 showed any symptoms consistent with tenacibaculosis (figure 8).

In tanks 3 and 4, 10 fish (in each tank) were challenged for one hour, and 10 fish (in each tank) were challenged for five hours with a lower dosage of the T.spl-isolate than in group 1. In tank 3, the mortalities of shedders started two days post challenge, but then stopped again for a period of four days (figure 7). In the following time, mortality of shedders increased gradually. A total of seven shedders had to be sampled, three challenged for one hour and four challenged for five hours. Out of the first five shedders that had to be sampled in tank 3, three of them had only been challenged for one hour. In tank 4, mortality did not start until eight days post challenge (figure 7). A total of 10 moribund shedders were sampled in tank 4, four challenged for one hour and six challenged for five hours. Four of the first five shedders to show lethargic behavior or symptoms in tank 4, were fish challenged for only one hour. Tanks 3 and 4 were the only tanks where the cohabitants demonstrated signs of tenacibaculosis. In tank 4, two cohabitants became moribund 17 days post challenge (figure 8). In tank 3, only one cohabitant showed clinical signs consistent with tenacibaculosis. This cohabitant did not show any clinical signs until the last day of the challenge experiment, 44 dpc (figure 8).

In tank 7 (control group), a total of six moribund shedders were sampled. Three shedders demonstrated a moribund behavior immediately following challenge with marine broth, while three others had to be sampled over the next 14 days (figure 7). There were no moribund or lethargic fish in tank 8.

### Macroscopic pathological symptoms - Main study

Ulcers of varying degree of severity, distributed on all parts of the body were the main pathological observation during the challenge experiment and were observed on symptomatic shedders in all challenge groups. Identical pathology was observed for three symptomatic cohabitants in group 2. Ulcers typically started after one to two days post challenge as brownish uneven circles of approximately 1-3 cm on the dorsal side, or yellowish circles on the ventral side of shedders. As days post challenge increased, so did the severity and extensiveness of the ulcers. After approximately three to five days post challenge, the center of the ulcers appeared greyish, with yellow or brown margins. The grey center always appeared to be without any scales. Larger well-developed ulcers on shedders where present in most tanks after roughly one week post challenge.

Since no cohabitants in group 1 displayed any clinical changes consistent with tenacibaculosis, the following description of pathology pertains only to the shedders. In group 1, starting rostral ulcers and jaw lesions were commonly observed on the shedders (see table 14). Color changes around snout to a light brownish or off-yellow color of the shedders became evident almost immediately after removal from the challenge tank. Starting ulcers appearing as brown circles on the dorsal side around the dorsal-, adipose- and caudal fin were also recurrent on shedders. Ulcers located around the ventral side of shedders however, were far less common in group 1 than in the other groups (see table 14). Ulcers located along the sides of the body were only observed on two of the shedders in tank 2 (see table 14). None of the ulcers developed enough to expose muscular tissue in group 1 and all shedders became moribund within three days. Tank 1 was the only tank in the challenge experiment where a swollen spleen was beheld at any regular basis (60% of all fish sampled, both cohabitants and shedders) (table 14). Yellow liver, petechia on the viscera and eye hemorrhages were only observed sporadically on all fish.

The shedders in group 2 (tanks 3 and 4) had a lower prevalence of ulcers located around the dorsal fin, but a much higher degree of ulcers located around the ventral fins than group 1 (table 14). Ulcers were also frequently observed around the adipose-, and caudal fin of shedders. Several of these lesions developed into large ulcers with brownish margins and exposed muscular tissue. Rostral ulcers and jaw lesions were also frequently found on shedders, howbeit in a slightly less frequency than in group 1. In some cases, jaw lesions were so extensive that the entire skeleton of the jaws were exposed. Ulcers located along the flanks of the shedders and fin rot were found in highest prevalence in tank 4 during the challenge study. Fin rot typically affected the tail-, ventral-, pectoral and anal fins, but not the dorsal fin (figure 23 and 24). Group 2 also had the highest occurrence of yellow liver in the entire challenge experiment, while petechia on the viscera, swollen spleen and eye hemorrhages were only observed sporadically (Table 16). Group 2 was also the only group to have symptomatic cohabitants, with identical pathology as the shedders in group 2. All three cohabitants displayed well developed ventral ulcers with exposed muscular tissue, located around the ventral fins (see figure 25). Extensive fin rot was also present on the caudal-, pectoral- and ventral fins, with exposed fin rays clearly visible. No rostral changes or jaw lesions were present, as was characteristic for the shedders in all groups.

**Table 14: Occurrence of different macroscopic pathological observations of all moribund or symptomatic fish made during necropsy. Only fish that showed symptoms, both shedders and cohabitants are included in this table. N= is listed in table X. Since all shedders, but none of the cohabitants in group 1 and 3 displayed pathology, N=20 for both groups In tank 3, N= 8 because there were seven symptomatic shedders and one symptomatic cohabitant. In tank 4, N=12, because there were 10 symptomatic shedders and two symptomatic cohabitants. Because no fish were symptomatic in tank 8, observations made during sampling at the end of the termination of the challenge experiment are instead included. The shedders that became moribund in tank 7 is included (N=6) in addition to 9 fish sampled at the end of the challenge experiment (N=15). T: Tank**

| **Tanks** | **Isolate** | **Rostral ulcers/Jaw lesions** | **Ulcers located around the dorsal fin** | **Ulcers located around the ventral fins** | **Ulcers located around the adipose- and caudal fin** | **Ulcers located along the flanks** |
|---|---|---|---|---|---|---|
| **T1** | *T.sp1* | 50 % | 35 % | 5 % | 20 % | 0 % |
| **T2** | *T.sp1* | 70 % | 35 % | 5 % | 20 % | 10 % |
| **T3** | *T.sp1* | 37,5 % | 12,5 % | 37,5 % | 62,5 % | 12,5 % |
| **T4** | *T.sp1* | 66,6 % | 8,1 % | 66,6 % | 50 % | 33,3 % |
| **T5** | *T.sp2* | 55 % | 10 % | 55 % | 85 % | 10 % |
| **T6** | *T.sp2* | 80 % | 25 % | 55 % | 55 % | 5 % |
| **T7** | Control | 0 % | 0 % | 13,3 % | 0 % | 0 % |
| **T8** | Control | 0 % | 0 % | 0 % | 0 % | 0 % |

Group 3, challenged with the *T.sp2*-isolate, displayed the highest prevalence of lesions affecting the snout and jaw of shedders. To that point, two shedders in tanks 5 and 6 displayed split jaw bones, where all connective and muscular tissue was eroded (figure 18 and 19). Ulcers located around the ventral fins and adipose-, and caudal fin of shedders were also frequently observed (figure 22). Fin rot affecting shedders was observed with high incidence. Ulcers around the dorsal fin were however observed with less frequency than in group 1. Despite this, the shedders in group 3, in addition to group 2, had the most severe ulcers. Swollen spleen, yellow liver and eye hemorrhages were only observed sporadically. No cohabitants became symptomatic, and they are therefore not included in table 14 and 15.

**Table 15: Occurrence of different macroscopic pathological observations of all moribund or symptomatic fish, made during necropsy. Only fish that showed symptoms, both shedders and cohabitants, are included in this table. Since all shedders, but none of the cohabitants in group 1 and 3 displayed pathology, N=20 for both groups. In tank 3, N= 8 because there were seven symptomatic shedders and one symptomatic cohabitant. In tank 4, N=12, because there were 10 symptomatic shedders and two symptomatic cohabitants. Because no fish were symptomatic in tank 8, observations made during sampling at the end of the termination of the challenge experiment are instead included. The shedders that became moribund in tank 7 is included (N=6) in addition to 9 fish sampled at the end of the challenge experiment (N=15). T: Tank.**

| **Tanks** | **Isolate** | **Fin rot** | **Swollen pleen** | **Yellow liver** | **Petechia viscera** | **Eye hemorrhage** | **N=** |
|---|---|---|---|---|---|---|---|
| **T1** | *T.sp1* | 5 % | 60 % | 10 % | 10 % | 5 % | 20 |
| **T2** | *T.sp1* | 20 % | 15 % | 20 % | 25 % | 15 % | 20 |
| **T3** | *T.sp1* | 37,5 % | 25 % | 50 % | 12,5 % | 0 % | 8 |
| **T4** | *T.sp1* | 91,6 % | 25 % | 33,3 % | 0 % | 8,3 % | 12 |
| **T5** | *T.sp2* | 45 % | 20 % | 25 % | 0 % | 0 % | 20 |
| **T6** | *T.sp2* | 45 % | 15 % | 30 % | 0 % | 20 % | 20 |
| **T7** | Control | 13,3 % | 6,6 % | 6,6 % | 0 % | 0 % | 15 |
| **T8** | Control | 0 % | 0 % | 0 % | 0 % | 0 % | 15 |

Two shedders in tank 7 in the control group displayed ulcers around the ventral fin. Furthermore, one shedder also displayed swollen spleen and yellow liver. In tank 8, no fish displayed any signs of an infection.

### Colored smears from skin, ulcers and gills

*Tenacibaculum-like* bacteria were found, almost without exception, on colored smears from skin and ulcers). Typically, larger ulcers displayed more bacteria on smears. Sometimes a yellowish coating could be seen on the belly of the shedders, and when smears from this yellow coating was examined under a microscope, large amounts of long, slender and threadlike bacteria were found. Bacteria resembling *Tenacibaculum* were only found sporadically on colored smears from gills, and in much less density and number, compared to ulcers.

### Re-isolation of T.sp1 and T.sp2 from fish

The *T.sp1*-isolate was successfully re-isolated from ulcers, skin and gills of shedders in the tanks 1, 2, 3 and 4. The bacterium was isolated from the margins ulcers located around the adipose-, ventral-, dorsal- and anal fins. The *T.sp1*-isolate was also re-isolated from jaw lesions affecting shedders in tanks 3 and 4. The position of the ulcers did not seem to matter, as the bacterium was isolated from ulcers located throughout the body surface. The *T.sp1*-isolate was only sporadically re-isolated from the gills. *Tenacibaculum-like* bacteria were only isolated on marine agar, despite efforts to isolate the bacteria on blood agar with 2 % NaCl. All 16S rRNA sequences of *Tenacibaculum-like* bacteria isolated from fish in tanks 1, 2, 3 and 4 matched perfectly with the *T.sp1*-isolate. The *Tenacibaculum-like* bacteria that were isolated on marine agar, from the ventral ulcers of the three moribund cohabitants in group 2 were all shown to give a full match to the *T.sp1*-isolate.

The *T.sp2*-isolate was also successfully re-isolated from ulcers affecting shedders in tanks 5 and 6, using marine agar. All 16S rRNA sequences from *Tenacibaculum-like* bacteria isolated from this group, matched completely with the *T.sp2*-isolate.

A third *Tenacibaculum* sp.-isolate, called *T.sp3,* was isolated from the gills of one fish in the control tanks. When sequenced using unspecific 16S rRNA primers, this isolate demonstrated 97 % sequence similarity to *T.sp1* and *T.sp2,* respectively. In the pilot study, an additional *Tenacibaculum* sp.-isolate was isolated from the skin of a symptomatic shedder in the tank challenged with the *T.sp2*-isolate. This isolate was called *T.sp4* and when sequenced using the unspecific 16S rRNA primers, it displayed a 99 % sequence similarity to *T.sp1* and *T.sp2.*

Several other types of bacteria were isolated and sequenced from the fish used in the challenge study. *V.splendidus* and *Vibrio* sp. were frequently isolated from the skin and gills of both moribund and healthy fish. In addition, both *Pseudomonas* sp. and *Pseudoalteromonas* sp. were sporadically isolated from ulcers and skin samples. A single isolation of *M.viscosa*/*M.marina* was also obtained from a blood agar plate, sampled from the skin of one shedder in tank 2. However, the bacteria were never isolated again.

**Table 16: The PNI (Percentage Nucleotide Identity) of type strains and the Tenacibaculum spp.-isolates used in this study, based on 847 nucleotides in the 16S rRNA gene. Note that the bacteria isolated from the cohabitants (K4-11, K4-12 and K3-16) display a 100 % match to the T.sp1-isolate. Selections of the Tenacibaculum sp. isolated from the shedders (K2-20, K5-8 and K6-12) are also included, and they display 100 % matches to the T.sp1- and T.sp2-isolates.**

| **Isolates** | ***T.sp1*** | ***T.sp2*** | ***T.sp3*** | ***T.sp4*** | ***T.solea*** | ***T.maritimum*** | ***T.ovolyticum*** | **K4-11** | **K4-12** | **K3-16** | **K2-20 MA** | **K5-8 MA** | **K6-12 MA** |
|---|---|---|---|---|---|---|---|---|---|---|---|---|---|
| ***T.sp1*** | 100 % | 99 % | 97 % | 99 % | 95 % | 92 % | 96 % | 100 % | 100 % | 100 % | 100 % | 99 % | 99 % |
| ***T.sp2*** | 99 % | 100 % | 97 % | 99 % | 96 % | 92 % | 96 % | 99 % | 99 % | 99 % | 99 % | 100 % | 100 % |
| ***T.sp3*** | 97% | 97 % | 100 % | 97 % | 95 % | 92 % | 96 % | 97 % | 97% | 97 % | 97 % | 97 % | 97 % |
| ***T.sp4*** | 99 % | 99 % | 97 % | 100 % | 96 % | 92 % | 96 % | 99 % | 99 % | 99 % | 99 % | 99 % | 99 % |
| ***T.solea*** | 95 % | 96 % | 95 % | 96 % | 100 % | 93 % | 96% | 95 % | 95 % | 95 % | 95 % | 96 % | 96 % |
| ***T.maritimum*** | 92 % | 92 % | 92 % | 92 % | 93 % | 100 % | 92 % | 92 % | 92 % | 92 % | 92 % | 92 % | 92 % |
| ***T.ovalyticum*** | 96 % | 96 % | 96 % | 96 % | 96% | 92 % | 100 % | 96 % | 96% | 96 % | 96 % | 96 % | 96 % |
| **K4-11** | 100 % | 99 % | 97 % | 99 % | 95 % | 92 % | 96 % | 100 % | 100 % | 100 % | 100 % | 99 % | 99 % |
| **K4-12** | 100 % | 99 % | 97 % | 99 % | 95 % | 92 % | 96 % | 100 % | 100 % | 100 % | 100 % | 99 % | 99 % |
| **K3-16** | 100 % | 99 % | 97 % | 99 % | 95 % | 92 % | 96 % | 100 % | 100 % | 100 % | 100 % | 99 % | 99 % |
| **K2-20 MA** | 100 % | 99 % | 97 % | 99 % | 95 % | 92 % | 96 % | 100 % | 100 % | 100 % | 100 % | 99 % | 99 % |
| **K5-8 MA** | 99 % | 100 % | 97 % | 99 % | 96 % | 92 % | 96 % | 99 % | 99 % | 99 % | 99 % | 100 % | 100 % |
| **K6-12 MA** | 99 % | 100 % | 97 % | 99 % | 96 % | 92 % | 96 % | 99 % | 99 % | 99 % | 99 % | 100 % | 100 % |

### Histology

The histology samples taken from a shedder and a symptomatic cohabitant in group 2 of the challenge experiment revealed loss of epidermis in areas, and signs of focal hyperplasia and increased number of mucus cells. The dermis layer, upper stratum spongiosum and lower stratum compactum contained large amounts of long slender rods, morphology characteristic for *Tenacibaculum* spp. (figure 32). A slight tendency for accumulation of bacteria below individual scales was observed. Bacteria could also be found in the hypodermis. The presence of *Tenacibaculum* spp. on gills was detected on the symptomatic cohabitants, although the lamella seemed to be unaffected.

### Real Time RT-PCR analysis of tissue samples

### The percentage of positive skin and kidney samples

The number of skin samples from shedders positive for *Tenacibaculum* varied significantly, from 12 % to 100 % in some tanks (see figure 9). The average percentage of positive skin- and kidney samples from cohabitants was considerably lower (see figure 10). A total of 35 skin samples (20 from shedders and 15 from cohabitants) were analyzed from each challenge tank, using Real Time RT-PCR, while in each control tank 15 skin samples were analyzed. A total of 10 kidney samples were also analyzed from each tank, five samples derived from shedders and five samples from cohabitants, respectively.

The shedders in tanks challenged with the *T.sp1*-isolate (Tanks 1, 2, 3 and 4) yielded positive results with high prevalence. Of the 20 shedders tested in tank 1 and 2, all gave positive results for both kidney and skin samples. The percentage of positive samples from shedders was however lower in tank 3 and 4, where 70 and 85 % skin samples tested positive, while 80 and 100 % of kidney samples gave positive results. The percentage of positive shedders was not reflected in the number of positive cohabitants. The cohabitants from tank 1 and 2 gave identical numbers of positive skin and kidney, where 80 % of skin samples and 60 % of kidney samples tested positive. On the other hand, all the cohabitants in tank 3 tested positive for both kidney and skin samples, while only 13.3 % of skin samples and a staggering 100 % of kidney samples were positive in tank 4.

The two tanks challenged with the *T.sp2*-isolate, tanks 5 and 6, had identical prevalence of positive skin and kidney samples from shedders as tank 1 and 2 , yielding 100 % prevalence of positive samples in tank 5 and 6. The prevalence of positive skin samples derived from cohabitants only decreased for tank 6 (93.3 %), while the percentage of positive skin samples from cohabitants in tank 5 was the same as for the shedders (100 %). Kidney samples on the other hand, were significantly less frequently positive, and only 60 % of kidney samples from cohabitants tested positive for *Tenacibaculum* sp. in both tanks.

Samples from the shedders tank 7 in the control group also demonstrated a high percentage of positive skin- (33 %) and kidney samples (80%). Cohabitants from tank 7 also displayed a high prevalence of positive skin- (44 %) and kidney samples (20 %). In steep contrast, the shedders from tank 8 in the control group only yielded 12 % and 20 % positive skin and kidney samples, while only 14 % of skin samples and 0 % of kidney samples from cohabitants tested positive.

### Normalized Real Time RT-PCR values from tissue samples

Ct-values from the Tb_rpoB assay were normalized against the corresponding EF1A Ct-value. Since no skin samples tested positive for *M.viscosa* analyzed with Mv_ompA, no normalization was performed for this assay. The logarithmic normalized expressions (log2 NE) of each sample analyzed for *Tenacibaculum* sp. are presented in figures 11-24. All tanks have two separate charts, one for shedders and one for cohabitants. , where the Log2 NE (Normalized Expression)-fold is plotted against days post infection. Kidney and skin samples are both included in each separate chart.

The shedders in all challenge tanks yielded consistently stronger positive results than cohabitants, when skin samples were analyzed. Typically, the later the shedder was sampled, the stronger the positive results were, with a few exceptions. Kidney samples yielded significantly weaker positive results than skin samples. As for the skin samples, kidney samples derived from shedders demonstrated consistently stronger positive results than the cohabitants. Typically, fish that gave a strong positive result for skin samples also yielded stronger positive results for kidney samples.

The shedders in tank 1 and 2 (*T.sp1*) all gave strong positives. A clear trend can be seen in figure 11 and 13, where the Log2-NE-fold steadily increases with days post infection. The strongest positives came from the last shedder sampled in the tanks. Except for one cohabitant in tank 1, all cohabitants in tanks 1 and 2 gave weak positive results, both for kidney and skin samples (figure 22 and 24).

In tanks 3 and 4 (*T.sp1*) several shedders tested strongly positive for *Tenacibaculum* sp. (figure 15 and 17). The symptomatic shedders that had been challenged for five hours typically tested slightly stronger than the ones challenged for one hour. However, the shedders that did not become moribund gave positive results similar to asymptomatic cohabitants. On the other hand, the skin samples for the two symptomatic cohabitants in tank 4, and the single symptomatic cohabitant in tank 3, also gave strongly positive results when analyzed for *Tenacibaculum* sp. similar to the strongest positive shedders (figure 16 and 18).

In tanks 5 and 6 (*T.sp2*)*,* the shedders that were sampled first tested only weakly positive (figure 19 and 21). However, as days post infection increased, stronger positive results were obtained. In tank 5, the shedders that were removed last yielded by far the strongest positives, while in tank 6, there was a marked drop in the strength of the positive samples from the shedders that were sampled last. The cohabitants in tank 5 yielded only weak positives, while two cohabitants in tank 6 gave moderately strong results (figure 20 and 22). As in the other groups, the kidney samples from the shedders tested stronger positive than the kidney samples from the asymptomatic cohabitants.

The control tanks, tank 7 and 8, yielded only weak positives when analyzed for *Tenacibaculum* sp. (figure 23 and 24).

### Tissue tropism for two symptomatic cohabitants

A tissue tropism analysis was conducted for the two symptomatic cohabitants in tank 4 (K4-11 and K4-12). Nine different tissues were included and tested using Real Time RT-PCR. The samples were analyzed using EF1A, Tb_rpoB and Mv_ompA. No samples tested positive for *M.viscosa.* The results of the tissue tropism test are illustrated in figure 25. Both cohabitants tested strongly positive for *Tenacibaculum* sp. for samples from skin and mucus, but weakly positive for samples from gill, heart, spleen, head kidney, and blood. However, K4-11 also tested strongly positive for *Tenacibaculum* sp. on samples derived from the tailfin. Interestingly, K4-11 also displayed a lower Ct-value for brain-tissue than K4-12, despite a stronger EF1A-value for K4-12. In addition, the K4-11 gill sample also tested stronger positive, than K4-12, despite EF1A being fairly similar.

## Claims

1. A *Tenacibaculum* isolate involved in the development of tenacibaculosis in farmed fish, wherein said isolate is deposited under the Budapest Treaty with the Leibniz Institute DSMZ-German Collection of Microorganisms and Cell Cultures (DSMZ) on 4^{th} March 2014 under deposit number DSM 28541.

2. Vaccine comprising a *Tenacibaculum* isolate according to claim 1.

3. A *Tenacibaculum* isolate according to claim 1, for use in a vaccine for prevention of tenacibaculosis in farmed fish.

4. A polynucleotide having a sequence selected from the group consisting of
(a) SEQ ID No. 1 encoding a 16sRNA gene;
(b) SEQ ID No. 2, encoding a atpD gene; SEQ ID No. 4 encoding a pgk gene; SEQ ID NO. 5 encoding a TUF gene; and SEQ ID No. 6 encoding a fusA gene.

5. A method for identifying in a sample, a *Tenacibaculum* bacterium involved in development of tenacibaculosis, wherein said *Tenacibaculum* belongs to the same species as the *Tenacibaculum* isolate deposited under the Budapest Treaty with the Leibniz Institute DSMZ-German Collection of Microorganisms and Cell Cultures (DSMZ) on 4^{th} March 2014 under deposit number DSM 28541, wherein said method comprises hybridization under stringent conditions of an oligonucleotide to single stranded nucleic acid sequences originating from a biological sample isolated from a fish suspected of having tenacibaculosis, wherein said oligonucleotide comprises a sequence of at least 10 nucleotides, wherein said oligonucleotide sequence hybridizes to a nucleic acid sequence originating from *Tenacibaculum*

6. Method according to claim 5, wherein said method comprises the steps of extracting nucleic acid sequences from said sample, combining the extracted nucleic acid sequences with one or more of said oligonucleotide sequence, and wherein hybridization of the oligonucleotide sequence selectively to a portion of the extracted nucleic acid sequences or a complementary sequence thereof, respectively, indicates the presence of *Tenacibaculum* in the sample.

7. Method according to any one of the claims 5-6, wherein said oligonucleotide hybridize to SEQ ID No. 1.

8. Method according to any one of the claims 5-6, wherein said oligonucleotide sequence is selected from the group consisting of SEQ ID No. 7 - SEQ ID No. 28 and variants of any of the sequences SEQ ID No. 7 - SEQ ID No. 28, respectively, having at least 97% sequence identity with the said sequences SEQ ID No. 7-28, respectively, based on the entire sequence length of said sequences.

## Patentansprüche

1. *Tenacibaculum-Isolat,* das bei der Entwicklung von Tenacibaculose bei Zuchtfischen involviert ist, wobei das Isolat gemäß dem Budapester Vertrag beim Leibniz-Institut DSMZ, Deutsche Sammlung von Mikroorganismen und Zellkulturen (DSMZ), am 4. März 2014 unter der Hinterlegungsnummer DSM 28541 hinterlegt wurde.

2. Impfstoff, umfassend ein *Tenacibaculum-Isolat* nach Anspruch 1.

3. *Tenacibaculum-Isolat* nach Anspruch 1 zur Verwendung in einem Impfstoff zur Prävention von Tenacibaculose bei Zuchtfischen.

4. Polynucleotid, das eine Sequenz hat, die aus der Gruppe, bestehend aus
(a) SEQ ID No.1, die ein 16sRNA-Gen codiert;
(b) SEQ ID No.2, die ein atpD-Gen codiert; SEQ ID No.4, die ein pgk-Gen codiert; SEQ ID No.5, die ein TUF-Gen codiert, und SEQ ID No.6, die ein fusA-Gen codiert, ausgewählt ist.

5. Verfahren zur Identifizierung eines *Tenacibaculum*-Bakteriums, das bei der Entwicklung von Tenacibaculose involviert ist, in einer Probe, wobei das *Tenacibaculum* zu derselben Spezies gehört wie das *Tenacibaculum*-Isolat, das gemäß dem Budapester Vertrag beim Leibniz-Institut DSMZ, Deutsche Sammlung von Mikroorganismen und Zellkulturen (DSZM), am 4. März 2014 unter der Hinterlegungsnummer DSM 28541 hinterlegt wurde, wobei das Verfahren Hybridisierung unter stringenten Bedingungen eines Oligonucleotids an einzelsträngige Nucleinsäuresequenzen, die aus einer biologischen Probe stammen, die von einem Fisch isoliert wurde, von dem angenommen wird, dass er Tenacibaculose hat, umfasst, wobei das Oligonucleotid eine Sequenz von wenigstens 10 Nucleotiden umfasst, wobei die Oligonucleotidsequenz an eine Nucleinsäuresequenz, die von Tenacibaculum stammt, hybridisiert.

6. Verfahren nach Anspruch 5, wobei das Verfahren die Schritte Extrahieren von Nucleinsäuresequenzen aus der Probe, Kombinieren der extrahierten Nucleinsäuresequenzen mit einer oder mehreren der Oligonucleotidsequenz(en) umfasst und wobei eine Hybridisierung der Oligonucleotidsequenz selektiv an einen Teil der extrahierten Nucleinsäuresequenzen bzw. eine komplementäre Sequenz davon das Vorliegen von Tenacibaculum in der Probe anzeigt.

7. Verfahren nach irgendeinem der Ansprüche 5-6, wobei das Oligonucleotid an SEQ ID No.1 hybridisiert.

8. Verfahren nach irgendeinem der Ansprüche 5-6, wobei die Oligonucleotidsequenz aus der Gruppe, bestehend aus SEQ ID No.7 - SEQ ID No.28 bzw. Varianten irgendeiner der Sequenzen SEQ ID No.7 - SEQ ID No.28, die wenigstens 97% Sequenzidentität mit den Sequenzen SEQ ID No.7 - 28, auf der Basis der gesamten Sequenzlänge der Sequenzen, haben, ausgewählt wird.

## Revendications

1. Isolat de *Tenacibaculum* impliqué dans le développement de la ténacibaculose chez des poissons d'élevage, dans lequel ledit isolat est déposé dans le cadre du Traité de Budapest auprès de l'Institut de Leibniz - Collection allemande de micro-organismes et cultures cellulaires (DSMZ) le 4 mars 2014 sous le numéro de dépôt DSM 28541.

2. Vaccin comprenant un isolat de *Tenacibaculum* selon la revendication 1.

3. Isolat de *Tenacibaculum* selon la revendication 1, pour utilisation dans un vaccin pour la prévention de la ténacibaculose chez des poissons d'élevage.

4. Polynucléotide ayant une séquence choisie dans le groupe consistant en
(a) SEQ ID NO: 1 codant un gène 16sARN ;
(b) SEQ ID NO: 2 codant un gène atpD ; SEQ ID NO: 4 codant un gène pgk ; SEQ ID NO: 5 codant un gène TUF ; et SEQ ID NO: 6 codant un gène fusA.

5. Procédé d'identification dans un échantillon, d'une bactérie *Tenacibaculum* impliquée dans le développement de la ténacibaculose, dans lequel ledit *Tenacibaculum* appartient à la même espèce que l'isolat de *Tenacibaculum* déposé dans le cadre du Traité de Budapest auprès de l'Institut de Leibniz DSMZ - Collection allemande de micro-organismes et cultures cellulaires (DSMZ) le 4 mars 2014 sous le numéro de dépôt DSM 28541, dans lequel ledit procédé comprend l'hybridation dans des conditions stringentes d'un oligonucléotide avec des séquences d'acide nucléique simple brin provenant d'un échantillon biologique isolé à partir d'un poisson suspecté d'être atteint de ténacibaculose, dans lequel ledit oligonucléotide comprend des séquences d'au moins 10 nucléotides, dans lequel ladite séquence oligonucléotidique s'hybride avec une séquence d'acide nucléique provenant de *Tenacibaculum.*

6. Procédé selon la revendication 5, dans lequel ledit procédé comprend les étapes d'extraction de séquences d'acide nucléique à partir dudit échantillon, la combinaison des séquences d'acide nucléique extraites avec une ou plusieurs de ladite séquence oligonucléotidique, et dans lequel l'hybridation de la séquence oligonucléotidique sélectivement avec une portion des séquences d'acide nucléique extraites ou une séquence complémentaire de celles-ci, respectivement, indique la présence de *Tenacibaculum* dans l'échantillon.

7. Procédé selon l'une quelconque des revendications 5 et 6, dans lequel ledit oligonucléotide s'hybride avec SEQ ID NO: 1.

8. Procédé selon l'une quelconque des revendications 5 et 6, dans lequel ladite séquence oligonucléotidique est choisie dans le groupe consistant en SEQ ID NO: 7 à SEQ ID NO: 28 et des variants de l'une quelconque des séquences SEQ ID NO: 7 à SEQ ID NO: 28, respectivement, ayant au moins 97% d'identité de séquence avec lesdites séquences SEQ ID NO: 7 à 28, respectivement, sur toute la longueur de séquence desdites séquences.
